# EUROPEAN PATENT SPECIFICATION

(11) **EP 4 076 390 B1**
(45) Date of publication and mention of the grant of the patent: **07.02.2024**
(21) Application number: 20839248.0
(22) Date of filing: 16.12.2020
(51) Int. Cl.: A61K 9/00, A61K 9/68

(54) **PREBIOTIC AND PROBIOTIC TREATMENT TO REDUCE ORAL DYSBIOSIS AND PROMOTE EUBIOSIS**
PRÄBIOTISCHE UND PROBIOTISCHE BEHANDLUNG ZUR VERRINGERUNG DER ORALEN DYSBIOSE UND ZUR FÖRDERUNG DER EUBIOSE
TRAITEMENT PRÉBIOTIQUE ET PROBIOTIQUE POUR RÉDUIRE LA DYSBIOSE BUCCALE ET FAVORISER L'EUBIOSE

(30) Priority: 17.12.2019 EP 19383131; 11.06.2020 EP 20179452
(43) Date of publication of application: 26.10.2022
(60) Divisional application: 23216530.8
(73) Proprietor: Fundación para el Fomento de la Investigación Sanitaria y Biomedica de la comunitat Valenciana, 46020 Valencia (ES)
(72) Inventor: MIRA OBRADOR, Alejandro, 46020 VALENCIA (ES); ROSIER, Bob Thaddeus, 46020 VALENCIA (ES); FERRER GARCÍA, María Desamparados, 46020 VALENCIA (ES); LÓPEZ LÓPEZ, Aránzazu, 46020 VALENCIA (ES)
(74) Representative: ZBM Patents - Zea, Barlocci & Markvardsen
(86) International application number: PCT/EP2020/086413
(87) International publication number: WO 2021/122741

(56) References cited:
- WO-A1-2017/182876
- JP-A- 2015 157 768
- KOOPMAN JESSICA E ET AL: "Nitrate and the Origin of Saliva Influence Composition and Short Chain Fatty Acid Production of Oral Microcosms", MICROBIAL ECOLOGY, SPRINGER VERLAG, NEW YORK, NY, US, vol. 72, no. 2, 7 May 2016 (2016-05-07), pages 479-492, XP035998621, ISSN: 0095-3628, DOI: 10.1007/S00248-016-0775-Z [retrieved on 2016-05-07] cited in the application
- BURLEIGH MIA ET AL: "Dietary nitrate supplementation alters the oral microbiome but does not improve the vascular responses to an acute nitrate dose", NITRIC OXIDE: BIOLOGY AND CHEMISTRY, vol. 89, 1 August 2019 (2019-08-01), pages 54-63, XP085711148, ISSN: 1089-8603, DOI: 10.1016/J.NIOX.2019.04.010 cited in the application
- EMBRIETTE R. HYDE ET AL: "Metagenomic Analysis of Nitrate-Reducing Bacteria in the Oral Cavity: Implications for Nitric Oxide Homeostasis", PLOS ONE, vol. 9, no. 3, 26 March 2014 (2014-03-26), page e88645, XP055289203, DOI: 10.1371/journal.pone.0088645

## Description

### FIELD OF THE INVENTION

The present invention relates to the fields of medicine, oral care, and microbiology, and particularly to compositions comprising nitrate and/or probiotic bacteria to reduce oral dysbiosis and promote eubiosis.

### BACKGROUND ART

### Oral dysbiosis / caries, periodontal diseases and halitosis / current treatments

The oral microbiota is known as a diverse microbial community with 100-200+ bacterial species per individual, of the 700+ that have been identified globally in the oral cavity. Inter-individual variation results from differences in age, host factors (e.g., genetic and immunity), environment and habits. The oral cavity offers several distinct habitats for microbial colonization and biofilm formation, like the teeth, tongue, buccal mucosa (interior of cheeks), lip palate and gingiva, and the species proportion in each habitat is significantly different. The saliva is inoculated with bacteria from all oral surfaces and, after biofilm removal (e.g., by oral hygiene), bacteria from saliva rapidly start forming new biofilms on the cleared surface.

Compared to other communities of the human body, the oral microbiota remains relatively stable in healthy adults over time for periods of years. Despite the stability of the oral microbiota, it is a living ecosystem and its composition and activity can undergo fluctuations. Certain disease drivers can lead to perturbations in species and functions of the oral microbiome that can trigger the development of oral diseases. These host-microbial perturbations associated with diseases are known as dysbiosis and are caused by a shift in microbial composition and activity. Dysbiosis can be caused by physiological changes resulting from, e.g., age and salivary gland dysfunction or lifestyle adoptions, like diet, poor hygiene, or smoking.

During many years, it was considered that there were "pathogenic" bacteria in the microbiota which caused infections and diseases. Now, it is known that the bacteria that were considered as pathogens are part of the resident microbiota and present in a healthy state, albeit in lower numbers. Oral disease develops because of a deleterious change in the balance of the microbiota resulting in an increase in the abundance of disease-associated species, rather than a result of an exogenous pathogen causing an infection. In dysbiosis, disease-associated bacteria can grow to higher proportions than under healthy conditions. Additionally, bacteria can switch metabolism and activate functions that contribute to dysbiosis and disease development. In summary, perturbations can lead to oral disease when disease drivers are strong or persistent enough.

Resilience is the capability to resist or recover from perturbations when disease drivers are present and this capacity differs between susceptible and tolerant individuals. In caries e.g., the main disease drivers are fermentable carbohydrates, like sugars. The oral microbiota can ferment these carbohydrates into organic acids, mainly lactate, which cause a fall in the local pH. Over time this results in a microbiota that is more acid-tolerant and more efficient at fermenting carbohydrates, generating a positive feedback loop that can cause enamel demineralization if pH levels get under pH ~5.5. Certain species are associated with the dysbiotic state of supragingival dental plaque observed in caries, including acid-resistant representatives of *Lactobacillus, Streptococcus, Veillonella, Oribacterium, Atopobium, Bifidobacterium, Actinomyces,* and certain yeasts. Importantly, other health-associated species decrease in number as the caries disease develops, including *Neisseria* spp., *Rothia* spp. and *Kingella* spp.

In periodontal diseases, the main disease driver is an innate host immune activation by accumulated dental plaque (i.e. biofilms on tooth surfaces), due to a lack of oral hygiene. In this case, there is also a positive feedback loop that can disrupt the microbiota and lead to periodontal diseases. In this loop, plaque accumulation causes an increase of anaerobic conditions and, subsequently, a growth of anaerobic species. Additionally, the host responds to accumulated bacteria with gingival inflammation that selects for inflammation-tolerant species. The inflammation also results in a slight increase in temperature and more leakage of gingival crevicular fluid (GCF, i.e., a serum-like fluid that leaks out of the gingival crevice). GCF contains a large amount of serum proteins and, unintentionally, serves as nutrition for proteolytic species. The degradation of proteins, results in a neutral or slightly alkaline pH, stimulating the growing of alkalophilic species.

Periodontal diseases begin with bacterial, biofilm-induced inflammation of the soft tissues surrounding the teeth (i.e., gingivitis). Dental plaque appears in different shades of white and is combined with food debris typically found at the gingival margin bordering teeth. Biofilm is also commonly found between teeth, where its removal requires additional efforts by using, e.g., dental floss and interproximal brushes. Regular oral hygiene is necessary to prevent inflammation as abstaining from oral hygiene results in gingivitis without exceptions, generally after a period of 2-3 weeks. Repeated or long lasting episodes of gingivitis can result in periodontitis, which is chronic and destructive inflammation in which host tissue is lost.

The dysbiotic subgingival plaque microbiota associated with periodontitis is complex. Classic bacteria associated with periodontitis (i.e., consistently more abundant in disease) include *Porphyromonas gingivalis, Treponema denticola, Tannerella forsythia, Fusobacterium nucleatum, Prevotella intermedia, Parvimonas micra* and *Aggregatibacter actinomycetemcomitans.* However, in a recent systematic review, 17 other species were associated to the disease, including (other) species from the genera *Eubacterium, Selenomonas, Dialister, Peptostreptococcus, Alloprevotella, Porphyromonas, Treponema* and *Prevotella* (Pérez-Chaparro et al., 2014). Importantly, also in periodontal disease development health-associated species are reduced or lost, and these include representatives of *Neisseria, Rothia,* or *Kingella,* among others. In respect to this, *Neisseria* and *Rothia* correlate with anti-inflammatory mediators, which indicates that these species prevent harmful inflammation. In the case of peri-implantitis, which consists in a pathological condition occurring in tissues around dental implants, the associated microbiology has been found to be extremely similar to that of periodontitis, and it is also considered to be the outcome of a microbial dysbiosis, leading to a strong inflammation of the peri-implant mucosa and progressive loss of supporting bone.

Bad breath, also known as halitosis, is a symptom in which a noticeably unpleasant breath odor is present. Halitosis is intra-oral in 90% of the cases and is mostly caused by changes in tongue microbiota composition and activity, leading to a microbial dysbiosis. This dysbiotic state causes bacterial degradation of sulphur-containing amino acids that results in the production of volatile sulphur compounds (VSCs) such as hydrogen sulfide (H₂S), methyl mercaptan and, to a lesser extent, dimethylsulfide. Apart from the tongue microbiota, halitosis has also been associated with periodontitis and periodontal pockets can be a source of VSC formation. Nevertheless, most individuals with halitosis do not suffer from periodontitis and their tongue coating is the only source of VSCs. Different studies with different detection methods have identified a broad range of bacteria associated with VSC production and halitosis. Some bacteria that have been consistently associated with the dysbiotic state of the tongue microbiota are representatives of *Prevotella, Fusobacterium, Porphyromonas* and *Leptotrichia,* and the classic halitosis biomarker *Solobacterium moorei.* Interestingly, S. *moorei* has also been associated with periodontitis. Like for caries and periodontal diseases, there are some health-associated species that could prevent halitosis, including *Rothia* spp. and *Neisseria* spp. The affection of halitosis has a significant impact - personally and socially - on those who suffer from it and are estimated to be the third-most-frequent reason for seeking dental aid, following tooth decay and periodontal disease.

Notably, the health-associated and disease-associated bacterial communities differ among different surfaces and their corresponding disease(s). Nevertheless, some overlap can be found between different diseases. For example, *Fusobacterium nucleatum* and *Porphyromonas gingivalis* are associated with inflammation in periodontitis, but also VSCs production in halitosis. Importantly, two oral genera consistently associated with health are *Rothia* and *Neisseria*. Representatives of these genera consistently are more abundant in health than disease and decrease as disease develops, regardless of the surface. This is relevant from a caries, gingivitis, periodontitis, periimplantitis and halitosis point of view. A third genus is *Kingella* that is associated with dental and periodontal health. An increase in these three nitrate-reducing genera can be considered as eubiosis of oral biofilms, which refers to a microbiota composition with higher levels of beneficial bacteria.

Periodontal treatment typically involves the physical removal of the biofilm and results in reduction of inflammation and improvement in the overall periodontal condition. Additional treatment modalities include surgical debridement, use of tetracycline or local application of statin agents, or prescription of systemic antibiotics. Antibacterial oral rinses are often also used, such as chlorhexidine gluconate, triclosan, triclosan plus zinc citrate or fluoride. They have a broad spectrum of antimicrobial activity against oral pathogens and therefore mouthwashes are used to treat different oral diseases (e.g. periodontal diseases, caries and halitosis). However, these antimicrobial compounds have different relevant side effects such as irritation and damage of the oral mucosa, discoloration and staining of the teeth, alteration of taste perception, endocrine disruption, or antibiotic resistance.

Precisely, one of the most relevant undesirable effects of antiseptics is associated to the alterations caused in the oral microbiota as a result of their unselective antiseptic effect. Importantly, the oral microbiota contributes to systemic nitric oxide levels by reducing nitrate to nitrite. It was shown that an antiseptic mouthwash increased blood pressure by disrupting nitrate reduction by oral bacteria. Additionally, dietary nitrate intake stimulates nitrate reduction by the oral microbiota, which has several beneficial effects, including the lowering of blood pressure, the increase of sport performance and antidiabetic effects. In light of this, antiseptic mouthwash has shown to interfere with sport performance. Additionally, over-the-counter mouthwash correlated with diabetes and pre-diabetes development.

In summary, since current antiseptics do not have a distinct bacterial cell target upon which to act, (long-term) use of antiseptic agents, especially at high concentrations, can remove biofilm and/or kill disease-associated bacteria, but simultaneously kill health-associated bacteria and disrupt the natural and beneficial properties of the resident oral microflora. Additionally, a disrupted microbiota, which normally protects host surfaces, can allow the colonization of (opportunistic) pathogens that cause diseases, such as candidiasis and other fungal infections. Therefore, there is currently no treatment to reduce dysbiosis associated to periodontal diseases, while antiseptic treatments have important negative side effects.

In case of halitosis, current treatments also include physical or chemical means to decrease the numbers of bacteria, products to mask the smell, or chemicals to alter the odor creating molecules. Antibacterial mouth rinses may help. They often contain antibacterial agents including cetylpyridinium chloride, chlorhexidine, zinc gluconate, essential oils, hydrogen peroxide, and chlorine dioxide, which have the same problems as mentioned before for periodontal diseases and thus, dysbiosis is not solved and beneficial oral bacteria may also be killed.

Current thinking in preventive dentistry contends that modifying or modulating the oral biofilm, rather than fully eliminating it, is the most promising strategy to prevent oral diseases. However, there are very limited data for the positive effect of prebiotics to modulate oral biofilms. The case with the highest degree of evidence is arginine, an amino acid that several health-associated bacteria are able to convert into ammonia, which due to its alkali properties, is able to buffer salivary and plaque pH. As a consequence of this, a high oral arginylotic activity has been found to be related to low caries experience. However, arginine has not been proposed to be effective against other oral diseases like periodontitis or halitosis, which are normally favored by alkaline environments and by the presence of high protein or amino acid levels. In fact, clinical evidence shows that arginine improves caries risk at the expense of increasing the levels of several bacteria strongly associated to periodontal diseases and halitosis, like *Treponema, Eubacterium or Prevotella* (Koopman et al., 2016). This implies that arginine administration may in fact increase dysbiosis in the oral cavity and could only be prescribed to prevent dental caries. Thus, there is a need for finding pre- and probiotic compositions that prevent an oral disease while not increasing the bacteria associated to other oral diseases.

In conclusion, it is believed that currently there are no treatments directed to reduce dysbiosis related to oral diseases without negative side-effects, so it is desirable to look for alternatives or improved products focusing on the dysbiosis management.

### Nitrate pathway in the oral cavity

Humans have low amounts of nitrate in their body as certain human cells produce nitric oxide from amino acids that oxidizes to nitrate. Nitrate concentrations are boosted with nitrate (NO₃⁻) from our diet. We get over 90% from nitrate from fruits and vegetables and particularly high amounts are found in leafy greens and beetroots. The human body alone cannot do anything of significance with nitrate. However, certain oral bacteria convert the nitrate into nitrite and the human body can effectively convert nitrite into nitric oxide by several enzymatic and non-enzymatic processes. Discoverers relating to nitric oxide (NO) won a Nobel Prize in 1998 and this important molecule is involved in many important functions of the human body, e.g.: the communication of neurons, the antimicrobial activity of the stomach, and the regulation of blood pressure by vasodilation.

Different research groups have focused on the systemic, mainly cardiovascular, benefits of nitrate, but studies that investigate the effects of nitrate inside the mouth are limited.

As discussed below and without being limited to theory, the present inventors believe that no prior art document directly and unambiguously describes use of nitrate to prevent or reduce dysbiosis and promote eubiosis of dental plaque and other oral biofilms.

In the cardiovascular field, it is herein discussed the content of articles Velmurugan et al., 2016 and Vanhatalo et al., 2018.

Velmurugan et al., 2016 describes a clinical trial focusing on the cardiovascular benefits of dietary nitrate, wherein oral bacterial profiles in saliva were measured. After 6 weeks of daily nitrate-rich beetroot juice consumption, containing 372 mg per serving (i.e., 1.7 times the Acceptable Daily Intake, ADI, which is 222 mg for an adult of 60 kg), 78 bacterial taxa were affected, and 2 nitrate-reducing species, *Rothia mucilaginosa* and *Neisseria flavescens,* increased notably. No other changes in bacterial species are mentioned in this document. They conclude that sustained dietary nitrate ingestion improves vascular function in hypercholesterolemic patients. These changes are associated with alterations in the saliva microbiome and, in particular, nitrate-reducing genera.

Vanhatalo et al., 2018 describes changes in oral microbiota detected in saliva after nitrate supplementation and its effects on vascular endothelial function and therefore blood pressure. They examined the relationships between the oral microbiota and physiological indices of NO bioavailability and possible changes in these variables following 10 days of beetroot juice supplementation. After 10 days, the salivary microbiome was altered compared to placebo by increasing the relative abundances of *Rothia* (+127%) and *Neisseria* (+351%), and decreasing *Prevotella* (-60%) and *Veillonella* (-65%). NO₃⁻ supplementation increased plasma concentration of nitrite (NO₂⁻) and reduced systemic blood pressure in old, but not young participants. High abundances of *Rothia* and *Neisseria* and low abundances of *Prevotella* and *Veillonella* were correlated with greater increases in plasma [NO₂⁻] in response to nitrate supplementation. It is noted that in this study, beetroot juice is administered during 10 days and reading carefully the article, it is confirmed that they have only found these significant changes of these bacteria in saliva, because measurements of the tongue are only taken at time 0, but not later: "Oral swabs of the tongue dorsum were collected at baseline. Saliva samples (~ 1 ml) were collected by expectoration, without stimulation, over a period of 5 min on three occasions following placebo and beetroot supplementation periods." On the contrary:
- The results on bacterial changes obtained by the present inventors are in a biofilm, while the results of Vanhatalo, as well as Velmurugan et al., are in saliva.
- Oral diseases are biofilm-mediated diseases (Kuang et al., 2018), thus are caused by different biofilms. The content in saliva does not correlate with the composition of any specific oral biofilm (see Mira 2018, or Simón-Soro et al., 2013); thus, microbial changes in saliva as a consequence of nitrate supplementation cannot predict changes in a specific biofilm nor predict health outcomes in biofilm-mediated diseases.
- Saliva samples in Vanhatalo were collected on days 8, 9 and 10 of each supplementation period. It was a cross-sectional design, with 10 days of treatment, washout period of 3-47 days (average 18 days) and a second 10-day treatment. The supplementation of nitrate in Vanhatalo is during 10 days.
- The study of Vanhatalo is in the context of cardiovascular diseases.
- The results on bacterial changes of the present inventors are remarkably different from the obtained in Vanhatalo as will be explained hereinafter.
- Vanhatalo use an extremely high dose of nitrate (i.e., 770 mg per day), which is around 3.5 times de ADI (i.e., 222 mg for an adult of 60 kg). In the present invention the physiological concentration range of saliva (EXAMPLE 1) and the ADI for nitrate composition intake (EXAMPLES 2 and 4) are respected, and the effects are observed with a single low dose of nitrate.

Koopman et al., 2016 reads in the abstract: "Nitrate is emerging as a possible health benefactor. Especially the microbial conversion of nitrate to nitrite in the oral cavity and the subsequent conversion to nitric oxide in the stomach are of interest in this regard. Yet, how nitrate influences the composition and biochemistry of the oral ecosystem is not fully understood. To investigate the effect of nitrate on oral ecology, the authors performed a 4-week experiment using the multiplaque artificial mouth (MAM) biofilm model." They applied 5 mM nitrate pulses of 6 minutes to 1-4 week old oral microcosms from two individuals, which were grown with the continuous supply of 1 mM nitrate, and each of them responded differently to nitrate in bacterial compositional changes. An effect on pH buffering, ammonia or lactate production was not detected and the number of participants was too low to conclude how the biofilm composition changes. Koopman does therefore not provide any results that could be interpreted as evidence for that supply of nitrate could reduce dysbiosis, promote eubiosis by eliminating oral pathogens and/or buffering pH and/or reducing lactate production.

Jockel-Schneider et al., 2016 describes that gingival inflammation in patients with chronic gingivitis was reduced after 14 days of nitrate intake. The article reads on page 607: "As this trial primarily focused on the clinical impact of the ingestion of dietary nitrate, it may only be speculated which of the aforementioned mechanisms and pathways contributed to the present findings." It is noted that this study is essentially clinical and oral microbiome is not analyzed.

Li et al., 2007 states that "anaerobic incubation of saliva containing a mixture of oral bacteria in the presence of nitrate/nitrite substrates and glucose resulted in a higher pH than was found in controls in the absence of nitrate/nitrite". It must keep in mind, that all saliva samples from 13 different donors used in their *in vitro* experiments are modified before usage. Specifically, all samples were diluted with equal volumes of water, degassed with nitrogen or enriched with oxygen, and the pH of the samples was adjusted to 7.0 by the addition of 2 mol NaOH and/or HCl. Furthermore, in most of the experiments, the saliva was pre-incubated for 12 h at 30°C before adding glucose (110 mM = 2%) and nitrate or nitrite (both 1.5 mM). Pre-incubation for 12 h at a different temperature from the human body will substantially change the composition of the microorganisms in the sample, giving a selective advantage of a subgroup of species that grow or survive best under those experimental conditions. Therefore, the observations in their modified samples do not necessarily reflect what would happen in the initial sample and certainly not what would happen in the oral cavity. In the other remaining experiment, where the samples were not pre-incubated after the initial modifications, they centrifuge and wash the saliva three times in PBS before resuspending the salivary pellet (containing the microorganisms) in PBS to the original volume, meaning that all other salivary components (including nutrients and pH buffering salts) are discarded. Once again, this does not reflect the real *in vivo* situation. By modifying the saliva samples in their study, the *in vitro* conditions are not a good representation of the *in vivo* situation. Additionally, by measuring the effect of microorganisms in saliva, the possible effects of oral biofilm (e.g., dental plaque and tongue coating) metabolism on salivary pH are not considered. Finally, the authors only found an effect of nitrate on acidification when samples were grown without oxygen. However, in the oral cavity there are niches with different levels of oxygen. Therefore, it can be concluded that the unnatural experimental conditions in the work by Li et al., make it impossible to predict the natural activity of oral biofilms exposed to nitrate. Rosier et al., 2018 indicates that "Another potential prebiotic is nitrate, but current *in vivo* evidence in humans is limited. In a recent clinical trial focusing on the cardiovascular benefits of dietary nitrate, oral bacterial profiles were measured (Velmurugan et al., 2016). After 6 weeks of daily nitrate-rich beetroot juice consumption, 78 bacterial taxa were affected, and 2 nitrate-reducing species, *Rothia mucilaginosa* and *Neisseria flavescens,* increased notably". These measurements, however, were performed on saliva, not on oral biofilms and in the context of clinical work to test cardiovascular health, not oral health. In addition, the changes observed were measured after a long-term daily high dose supplementation (6-weeks) and therefore the short-term effect of nitrate supplementation (i.e. <24 h) remains unknown. This review also mentions that "prebiotics can drive beneficial changes in the oral microbiome and could increase resistance to dysbiosis and recovery of health", but only provide evidence for arginine.

In summary, the articles discussed above do not mention changes in biofilms, only in saliva. They also focus on long-term effects of nitrate supplementation (between 1-6 weeks). Therefore, the present inventors believe that they are the first to observe changes in the same bacteria and other more relevant bacteria and bacterial functions in biofilms grown *in vitro* after 5 h and 9 h, or on humans after less than 24 h, as well as to propose its beneficial effect on all biofilm-mediated oral diseases.

The only article showing changes in a biofilm (tongue sample) is from Burgleigh et al., 2019. They observed that after 7 days of beetroot consumption, there is an increase in the salivary pH and that bacteria change on the tongue: *Neissera* increases and *Prevotella, Actinomyces and Streptococcus* decreases. It is noted that:
- It is a change after daily nitrate consumption during 7 days (long-term effect) and herein it is shown after 5 hours (short-term, immediate effect).
- Burgleigh uses an extremely high dose of nitrate (i.e., 770 mg per day: 385 mg in the morning and 385 mg in the afternoon), which is around 3.5 times ADI. Again, in the present invention, the physical concentration range of saliva (EXAMPLE 1) and the ADI for nitrate composition intake (EXAMPLES 2 and 4) is respected and the effects are observed with a single low dose of nitrate.
- The placebo used in the study of Burgleigh is nitrate depleted beetroot juice containing high amount of sugar, that without the presence of nitrate significantly decreases the pH of oral biofilms. It has been showed that a low pH selects for specific acid-tolerant microorganisms, which could affect their results, especially because they give two doses of (placebo) juice per day and frequency of sugar intake is an important factor in microbiota modulation and caries development.
- They relate the changes observed in the tongue microbiota to caries and periodontal diseases, while the skilled in art knows that said diseases are caused by changes in dental plaque, not in tongue.
- In the discussion they make some confusing associations such as that the observed changes in microbiota could prevent acidification and that acidification is linked to caries and periodontal diseases. This cannot be considered true: acidification is only linked to caries and would prevent the growth of periopathogens instead of stimulating it. Additionally, *Prevotella* is not linked to acidification in any way. Conversely, *Prevotella* is a proteolytic species and proteolytic metabolism increases the pH (Takahashi 2005). Therefore, the association of Burgleigh with an increase of *Prevotella* and acidification is wrong.

Patent JP 2015 157768 A discloses a composition comprises Rothia and nitrate or nitrate ions for administration to the oral cavity for treating periodontal disease.

As seen, the prior art is confusing in using bacterial composition in saliva and not in oral biofilms, which is where oral diseases take place. In addition, when oral biofilms are considered, they use tongue biofilms, which are not relevant for caries or periodontal diseases. Furthermore, changes in salivary or tongue bacterial composition are studied only after long-term (>1 week) supplementation in the context of cardiovascular health clinical studies and not designed to study the effect of nitrate on oral health through bacterial dysbiosis. The prior art is also unclear when describing the underlying functions that change after nitrate supplementation, either by showing conflicting evidence or by not providing evidence for the mechanism involved in reducing dysbiosis, whereas the present inventors show that this is achieved by several functions like ammonia production, nitric oxide production or lactate depletion. Also relevant, the potential benefits of nitrate for oral health are never proposed for all oral diseased together, and when they are proposed individually, disease drivers for an oral disease are confusingly used to refer to other oral diseases (e.g. erroneously indicating that acidic pH may cause periodontal disease). In summary and without being limited to theory, the present inventors believe that no prior art document directly and unambiguously describes the use of nitrate for preventing or reducing bacterial dysbiosis or promoting eubiosis from a caries, periodontal diseases and halitosis point of view. Further it is also herein demonstrated that nitrate can have a prebiotic acute effect by promoting health-associated bacteria and reducing disease-associated bacteria and functions in biofilms, which derives in a beneficial action against all biofilm-mediated oral diseases, including caries, periodontitis, halitosis and peri-implantitis.

### SUMMARY OF THE INVENTION

The problem to be solved by the present invention may be seen as the provision of a method of reducing or preventing oral dysbiosis and increasing oral eubiosis, and thereby providing an improved method for treatment or prevention of a biofilm-mediated oral disease such as e.g. caries, periodontal diseases (e.g. gingivitis, periodontitis or peri-implantitis) and halitosis.

As discussed in the working Examples herein, the inventors identified that nitrate may be used for reducing dysbiosis and promoting eubiosis in oral biofilms.

As discussed above, the present inventors believe that no prior art document directly and unambiguously describes the use of nitrate for reducing dysbiosis and promoting eubiosis in oral biofilms.

It is evident that the herein discussed novel use of nitrate for reducing dysbiosis and promoting eubiosis may be seen as a contribution to the art that changes the behavior of the skilled person; for instance, based on the teaching herein it is plausible that e.g. a novel toothpaste comprising a herein relevant amount of nitrate (e.g. instead of or in addition to today used fluoride) would be suitable for treatment and/or prevention of carries, periodontal diseases or halitosis due to reducing dysbiosis and promoting eubiosis.

In contrast to the limitations mentioned above in the case of arginine as a treatment, it was surprising for the present inventors that nitrate administration in oral biofilms provided a pH buffering effect and a reduction in caries-associated bacteria while at the same time reducing the levels of periodontitis-associated and halitosis-associated bacteria. Therefore, the present inventors believe that in the light of evidence provided herein, nitrate can be considered a product that truly reduces dysbiosis and/or promotes an eubiotic bacterial composition in oral biofilms, unlike arginine that increases bacteria associated with periodontal diseases and halitosis.

Remarkably, this makes nitrate treatment an effective treatment for several diseases at once, which may be seen as a great contribution to the art, e.g. in the oral care industry because with a single composition (e.g. toothpaste) it is possible to treat and prevent both caries and periodontal diseases, as well as halitosis.

In summary, based on the results provided herein one may find it plausible that nitrate could be used as a prebiotic to improve global oral health by e.g. reducing or preventing oral dysbiosis and increasing oral eubiosis, and thereby providing an improved method for treatment or prevention of a biofilm-mediated oral disease such as e.g. caries, periodontal diseases (e.g. gingivitis, periodontitis or peri-implantitis) and halitosis.

Accordingly, a first aspect of the invention relates to an oral composition comprising nitrate for use in an acute treatment or prevention of oral dysbiosis in a mammal, wherein the acute treatment or prevention of oral dysbiosis comprises changing the bacterial composition and functions of oral biofilms in a mammal, and decreasing the amount of disease-associated bacteria and increasing the amount of health-associated bacteria; wherein the composition is orally administered to the mammal and thereby increases the concentration of nitrate in the saliva of the mouth; wherein the amount of nitrate per dose of composition is at least 3 micrograms when the oral composition is a topically applied composition, and at least 12 micrograms when the oral composition is an ingested composition; and wherein the acute treatment or prevention has effects before 24 hours of a biofilm-mediated oral disease.

The term "dysbiosis" is used to describe a shift of bacterial species and functions associated with a disease. Different diseases, such as caries, gingivitis, periodontitis, peri-implantitis and halitosis have different dysbiotic compositions. Additionally, while caries, gingivitis and periodontitis result from a dysbiotic composition of dental biofilms (dental plaque), halitosis results from a dysbiotic composition of the tongue biofilm (tongue coating) and peri-implantitis from one of a biofilm on an implant.

In dysbiosis, apart from an increase in disease-associated species and functions, health-associated species and functions are lost. If any oral biofilm is removed, bacteria from the saliva form a new biofilm on this surface. In EXAMPLE 1 and 6, the inventors obtained the first evidence that the presence of nitrate acutely improves the composition of this new biofilm (e.g., dental plaque after tooth brushing or tongue coating after tongue scraping) by reducing dysbiosis and increasing health-associated species and functions.

EXAMPLE 2 and 7 provide the first *in vivo* evidence that a nitrate-rich supplement affects bacterial activity directly after a single intake (i.e. an acute effect). This effect was shown to happen via topic application and via ingestion of the product. Thus, it is shown that nitrate provides resilience against dysbiosis. Specifically, inventors showed that a nitrate-containing supplement prevents or limits a pH drop due to sugar consumption directly (1 h), 1 h 45 min and 4 h after supplement intake. This is the first *in vivo* evidence that nitrate prevents caries-associated metabolism. The effect was the strongest after 4 h when the oral microbiota has had most time to change due to nitrate (reducing caries-associated dysbiosis or increasing dental-health associated eubiosis). Importantly, unlike other nutrients, nitrate and nitrite levels in saliva are still elevated after 4 h due to the plasma nitrate-recycling activity of the salivary glands.

As understood by the skilled person in the present context, the use of nitrate for reducing dysbiosis and promoting eubiosis in oral biofilms in the terms of the first aspect of the invention, is a clearly different use of the nitrate in e.g. above discussed prior art uses. The differences between the present invention and the prior art documents are explained in detail in this description. It is herein remarked that the prior art is referred to a long-term effect of daily nitrate doses above the ADI and the most relevant observations are in saliva samples. In this sense, it is important to keep in mind that oral bacteria form biofilms on all oral surfaces. Biofilm on the teeth (dental plaque) and on the tongue (lingual layer) are seen with the eye because this is where more bacteria accumulate. That is why they are the most important biofilms for oral health, but there are also biofilms inside the cheeks, the palate, etc. There is an important relationship between saliva and oral biofilms: on one hand, bacteria of all biofilms and sometimes the respiratory tract (resulting form, e.g., nasal drip or coughing) enter into the saliva, resulting in ten-million to one hundred-million bacteria per ml of saliva; on the other hand, when a biofilm from a surface is removed, e.g. with oral hygiene, saliva bacteria do not take long to colonize the surface and form a new biofilm. However, the environmental conditions on each oral surface determine that bacteria grow well, and that is why the lingual layer is very different than the dental plaque (in a healthy person, specific bacteria can be abundant in the dental plaque and of low abundance in the lingual layer). Thus, both biofilms start with bacteria in saliva, but the final product is very different. It is important to keep in mind that dental plaque is the cause of caries and periodontal diseases while lingual biofilm is the cause of halitosis. The cause of the disease is not a particular composition of bacteria in the saliva. Therefore, the prior art observations in saliva samples cannot lead to a consequence into a biofilm-mediated disease. In contrast, the present inventors have seen that nitrate has a clear effect in changing the composition of oral biofilms, thereby getting a treatment or a prevention of biofilm-mediated oral diseases. The *ex vivo* biofilm model used by the inventors reflect any biofilm formed by saliva; i.e. any oral biofilm.

Also described in the present disclosure, but not part of the invention, is a composition comprising nitrate for use in the treatment or prevention of halitosis and wherein the composition is orally administered to the mammal and thereby increases the concentration of nitrate in the saliva of the mouth. By decreasing VSC producing bacteria, the amount of VSC production will inevitably decrease.

The inventors have also found that individuals have different nitrate reduction capacities (NRC, i.e., the capacity of their oral microbiota to reduce nitrate into nitrite) and that this capacity can be stimulated with nitrate-reducing probiotics (EXAMPLE 3). Even in an individual with a low to undetectable NRC, the addition of nitrate-reducing probiotics resulted in a significant NRC *in vitro.* To obtain potential probiotics, 62 nitrate reducing strains were isolated and the 10 best nitrate reducers were selected, which were all *Rothia* spp. From these 10 strains, 7 isolates with different properties were selected (five *Rothia mucilaginosa* strains, one *R*. *dentocariosa* and one *R*. *aeria* strain). Specifically, these 7 isolates reduced nitrate and nitrite at different rates at established pH levels (e.g, some reduced nitrate better at an acidic pH and others at a neutral pH). Each isolate is suitable to treat and prevent dysbiotic states and improve resilience against different oral diseases (e.g., caries has an acidic pH, while periodontitis has a neutral to slightly alkaline pH). Additionally, isolates that reduce nitrate, but do not further reduce most of the nitrite are suitable to prevent and treat systemic conditions (e.g., hypertension and diabetes) as the nitric oxide levels in the body increase when swallowing nitrite.

Also described in the present disclosure, but not part of the invention, is a composition comprising nitrate and/or a bacterial strain belonging to *Rothia, Neisseria* or *Kingella* genera, for use in increasing the nitrate-reduction capacity of a mammal, by acutely increasing the amount of nitrate-reducing bacteria in oral biofilms, thereby getting a treatment or prevention of a disease or state that benefits from nitric oxide supply. In respect to this, the inventors have therefore identified particular bacteria with beneficial features to be used as probiotics in e.g. individuals with poor capacity of nitrate-reduction. Thus, another aspect of the invention relates to a composition comprising a bacterial strain wherein the bacterial strain belongs to *Rothia* genus, and wherein the *Rothia* bacterial strain:
a) reduces 100% of nitrate after 7 h of incubation at 37 °C starting with an optical density (OD) of 0.01 in BHI medium with 6.5 mM nitrate;
b) reduces more than 15% of nitrate after 4 h of incubation at 37 °C starting with an OD of 0.01 in BHI medium with 6.5 mM nitrate;
c) does not decrease the pH of BHI medium with 6.5 mM nitrate after 7 h of incubation at 37°C starting with an OD of 0.01 below pH 6.8;
d) grows to an optical density over 0.7 after 7 h of growth in BHI medium with 6.5 mM nitrate at 37 °C starting with an optical density OD of 0.01; and
e) is able to colonize an *in vitro* oral biofilm grown from human saliva during 5 h at 37°C when adding 1:1 *Rothia* bacterial strain in BHI (OD 0.40):saliva inoculum, reaching a proportion of more than 10% of total bacteria in the formed biofilm.

Another aspect of the invention relates to a composition comprising a bacterial strain selected from the group consisting of strain deposited in the Spanish Type Culture Collection (CECT) under the accession numbers CECT 9999, CECT 30000, CECT 30001, CECT 30002, CECT 30003, CECT 30004, CECT 30005, or combinations thereof. Other related aspects of the invention refer to the compositions comprising the bacterial strains for use as medicaments. This aspect can alternatively be formulated as a method for probiotic treatment, comprising administering in a need thereof an effective amount of a composition comprising at least one bacterial strain. The term "probiotic" as used herein, refers to live microorganisms that, when administered in adequate amounts, confer a health benefit on the host.

Also described in the present disclosure, but not part of the invention, is a method for selecting a therapeutic treatment or a preventive strategy for a biofilm-mediated oral disease or a disease or state that benefits from nitric oxide supply, the method comprising:
i) measuring the nitrate-reduction capacity of a subject in an oral sample;
ii) classifying the subject according to the degree of nitrate-reduction capacity of the subject, wherein, when the nitrate-reduction capacity is measured by adding 0.05 ml of 80 mM nitrate to a fasting oral sample of 0.45 ml to reach a final volume of 0.5 ml and a final nitrate concentration of 8 mM and incubating for 2 hours at 37 °C,
ii.1) a decrease of the amount of nitrate of the oral sample below 57 mg/l is indicative of a poor nitrate-reduction capacity,
ii.2) a decrease of the amount of nitrate of the oral sample between and including 57 mg/l and 175 mg/l is indicative of an intermediate nitrate-reduction capacity, and
ii.3) a decrease of the amount of nitrate of the oral sample above 175 mg/l is indicative of a good nitrate-reduction capacity; and
iii) selecting a therapeutic treatment or a preventive strategy according to the nitrate-reduction capacity, wherein:
   iii.1) a subject with a poor nitrate-reduction capacity is administered with a composition comprising nitrate and a bacterial strain belonging to *Rothia* or *Neisseria* genera,
   iii.2) a subject with an intermediate nitrate-reduction capacity is administered with a composition comprising nitrate and/or a bacterial strain belonging to *Rothia* or *Neisseria* genera, and
   iii.3) a subject with a good nitrate-reduction capacity is administered with a composition comprising nitrate.

### DEFINITIONS

All definitions of herein relevant terms are in accordance of what would be understood by the skilled person in relation to the herein relevant technical context.

Oral (i.e. relating to the mouth) diseases include dental diseases (e.g. caries) or periodontal diseases (e.g. gingivitis, periodontitis or periimplantitis) and halitosis (a symptom in which a noticeably unpleasant breath odor is present).

Oral biofilm or plaque is a biofilm or mass of bacteria that grows on surfaces within the mouth. Oral biofilm growth is important for oral diseases. Oral diseases are dependent on the niche (gums for periodontitis, tongue for halitosis, teeth for caries) and are mediated by biofilms.

The term "dysbiosis", also called dysbacteriosis, is used to describe a shift (or imbalance) of bacterial species and functions associated with a disease. The microbiota has a commensal relationship to the host; the bacteria thrive in the rich environment of the mouth while the host benefits from multiple functions provided by the bacteria. The homeostatic balance of the oral microbiota is extremely beneficial to the host, however if there is a change in the microbial composition that causes a drastic imbalance between the beneficial (i.e. health-associated) and potentially pathogenic (i.e. disease-associated) bacteria, the mouth becomes vulnerable to pathogenic insult with microbial alterations. This imbalance in the microbial equilibrium is termed "dysbiosis", which has been further defined as a disturbance to microbiota homeostasis due to an imbalance in the flora itself, changes in their functional composition and metabolic activities, or changes in their local distribution. In general, dysbiosis can be categorized into three different types: 1) Loss of beneficial organisms and/or functions, 2) Excessive growth of potentially harmful organisms and/or functions, and 3) Loss of overall microbial diversity. It has been found that these three types are not mutually exclusive and can occur at the same time, which is most often the case. More details about the concept of dysbiosis are described in DeGruttola et al., 2016.

Dysbiosis can be considered as a clinical entity or a pre-disease state, thus subject to be treated or prevented. For example, in a healthy individual, sugar consumption can increase cariogenic species and dysbiotic lactate production, decreasing the pH to levels that can demineralize enamel. In a healthy individual, these changes can be reversed rapidly (resilience). However, if sugar is consumed continuously, over time dysbiosis can become stable due to a more cariogenic microbiota (i.e., more species adapted to sugar metabolism) and caries can form. One approach would to be increase resilience in healthy individuals by increasing eubiosis (prevention), while another approach would be to decrease dysbiosis in caries-active individuals (treatment). The same holds for increasing eubiosis and decreasing dysbiosis related to periodontal diseases and halitosis.

The term "eubiosis" (also called "probiosis") of oral biofilms refers to a microbiota composition with higher levels of beneficial bacteria and/or bacterial activity, while disease-associated species are present, but in a lower abundance. Eubiosis includes more resilience to diseases, which means more resistance to disease drivers (i.e. a protective effect to any factor that can cause disease) and a quicker recovery from a perturbation caused by a disease driver. More details about the concept of eubiosis are described in lebba et al., 2016 and dysbiosis and resilience in Rosier et al., 2018.

The composition according to the invention reduces or prevents oral dysbiosis Synonyms of the expression "reducing dysbiosis" in this description are "reversing dysbiosis", "modulating dysbiosis", "modulating oral biofilm composition", or has an "anti-dysbiosis effect". In particular, reducing dysbiosis is related to "decreasing bacteria/bacterial activities that result in volatile sulfur compound production", "decreasing bacteria/bacterial activities that lower the pH", "decreasing bacteria/bacterial activities that cause inflammation", as well as "increasing bacteria/bacterial activities that prevent volatile sulfur compound production", "increasing bacteria/bacterial activities that prevent a pH drop", "increasing bacteria/bacterial activities that prevent inflammation".

Further, the composition according to the invention increases oral eubiosis. Synonyms of the expression "increasing eubiosis" are "improving homeostasis and symbiosis", "stimulating a healthy composition of the oral biofilms". It is characterized by a better resilience, meaning a better recovery of and resistance to disease drivers resulting from microbiota activity. This also includes "decreasing bacteria/bacterial activities that result in volatile sulfur compound production", "decreasing bacteria/bacterial activities that lower the pH", "decreasing bacteria/bacterial activities that cause inflammation", as well as "increasing bacteria/bacterial activities that prevent volatile sulfur compound production", "increasing bacteria/bacterial activities that prevent a pH drop", "increasing bacteria/bacterial activities that prevent inflammation".

Decreasing dysbiosis and increasing eubiosis is often used interchangeably. However, in a healthy individual without dysbiosis, it should be stated that if the composition improves, eubiosis increases, with the benefits (i.e. resilience, protection) described above.

Different diseases, such as caries, gingivitis, periodontitis, peri-implantitis and halitosis have different dysbiotic compositions. While caries, gingivitis and periodontitis result from a dysbiotic composition of dental biofilms (dental plaque), halitosis results from a dysbiotic composition of the tongue biofilm (tongue coating) and peri-implantitis from one of a biofilm on an implant. Dental biofilm or plaque is a biofilm or mass on the teeth - including subgingival plaque or supragingival plaque. The biofim on the tongue is called tongue coating and is thickest on the back of the tongue.

The term "biofilm-mediated disease" refers to a disease that is initiated and developed by the activity of microbial biofilms rather than by planktonic or intracellular microorganisms. In biofilm-mediated diseases, where the biofilm is formed by microbiota species, a larger proportion of disease-associated bacteria and bacterial activity, as well as a lower proportion of health-associated bacteria and bacterial activity, increases the risk and severity of the pathology.

According to the art, the term "prebiotic" relates to a compound that induce/promote the growth or activity of beneficial microorganisms such as bacteria and fungi.

Nitrate reduction capacity (NRC) is the capacity of an individual's oral microbiota to reduce nitrate into nitrite.

The term "disease-associated bacteria" refers to bacteria which are present in higher relative abundance in certain disease or, accordingly, lower relative abundance in health at the surface where disease can develop. Sometimes functions of disease-associated bacteria have been identified that contribute to an oral disease and, therefore, "disease-associated bacteria" can also be called "pathogenic", "cariogenic" or "periopathogenic" bacteria.

The term "health-associated bacteria" refers to bacteria which are present in lower relative abundance in certain disease and, accordingly, higher relative abundance in health at the surface where disease can develop. Sometimes functions of health-associated bacteria have been identified that can prevent oral disease development and, therefore, "health-associated bacteria" can also be called "beneficial bacteria", "probiotic bacteria" or "probiotics".

Note that some genera have health-associated and disease-associated species, but the genera can still be health-associated or disease-associated. For example: *Streptococcus mutans* is caries-associated, while *Streptococcus dentisani* is health-associated (caries free-associated). However, the genus *Streptococcus* always increases in caries (different cariogenic *Streptococcus* increase a lot, while *S*. *dentisani* may be decreasing), so the genus is disease-associated.

The term "disease or state that benefits from nitric oxide supply" relates to any condition or state of the human body that improves when systemic levels of nitric oxide increase (e.g. hypertension - more examples are described hereinafter -). An increase of systemic nitric oxide levels can be achieved by stimulating nitrate-reduction by the oral microbiota with nitrate (prebiotic) or nitrate-reducing bacteria (probiotics).

Throughout the description and claims the word "reduce" and derivates (e.g., reduction or reducing) in combination with "nitrate" or "nitrite" refers to bacterial conversion of nitrate to nitrite, or the bacterial or chemical conversion of nitrite to nitric oxide or ammonium. Only if not combined with "nitrate" or "nitrite", the word "reduce" and derivates are used as normally to indicate a "decrease" or "lowering" in something.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### DESCRIPTION OF THE DRAWINGS

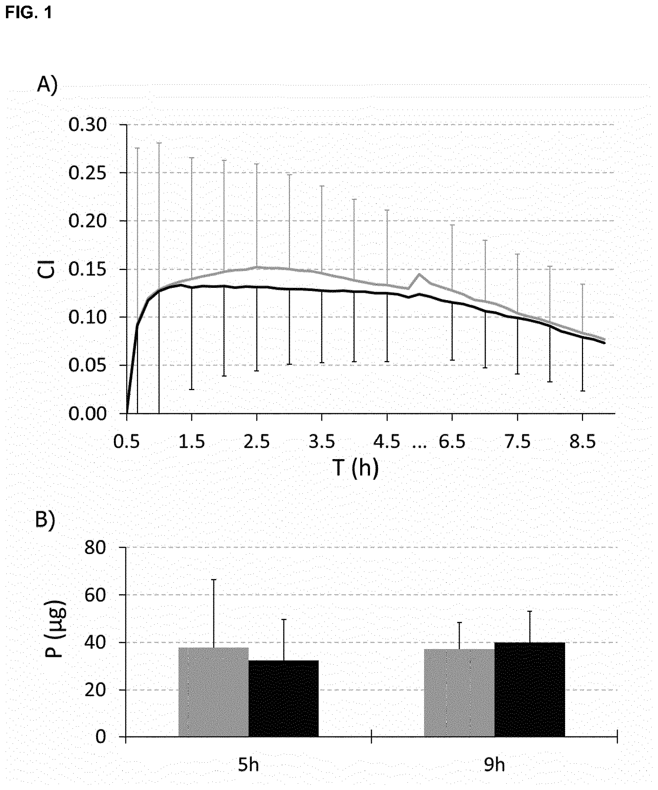

**FIG. 1****:** Biofilm quantification. Biofilms grown with saliva as inoculum in the 6.5 mM nitrate condition (black) and the control condition (grey). All values presented are averages of 12 donors (D13-D25) with their corresponding standard deviations. A: plot shows averages of biofilm mass, expressed as Cell Index (CI) values over time (T), as indicated by the xCELLigence system, after normalization with microorganisms-free filtered saliva. Measurements were taken every 10 minutes. Error bars with standard deviations are only shown at half an hour intervals for clarity. During all biofilm growth experiments, at 5h and 9h, samples were taken for protein quantification. B: Protein quantification of the biofilms harvested at 5h and 9h. P: protein. No significant changes were observed between the two conditions.

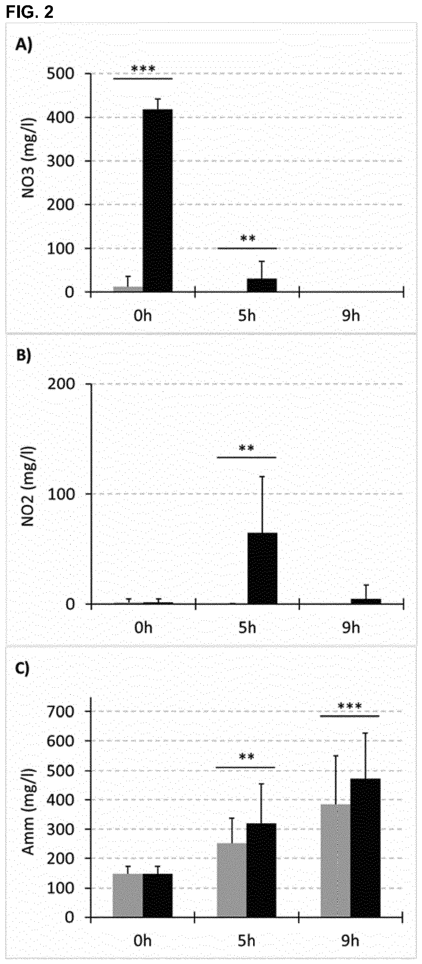

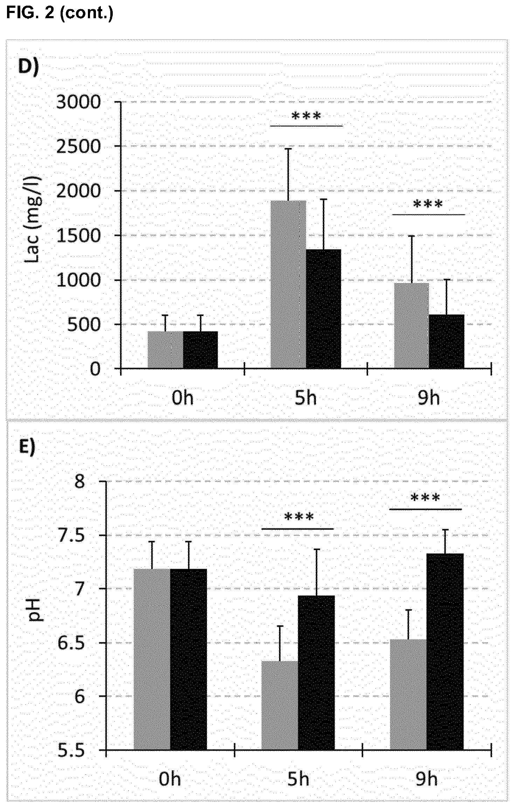

**FIG. 2****:** Nitrate (A), nitrite (B), ammonium (C), lactate (D) and pH (E) measurements in oral biofilms grown *in vitro* with or without nitrate. Barplots show averages and standard deviations of measurements in supernatant samples from 12 donors (D1-D12) of the 6.5 mM nitrate (black) and the control (grey) conditions at different times of biofilm growth (0h, 5h and 9h). NO3: nitrate. NO2: nitrite. Amm: ammonium. Lac: lactate. *** p < 0.005 ** p < 0.01, according to a Wilcoxon test.

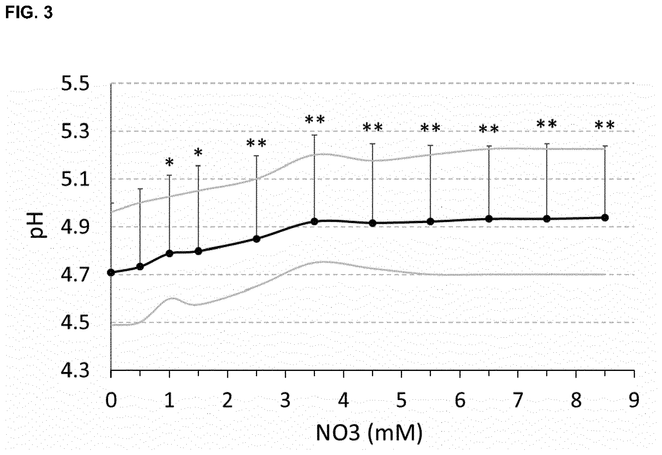

**FIG. 3****:** Salivary acidification is inhibited by nitrate. Saliva of 9 donors (D25-D33) was incubated for 5h with 0.2% glucose and a concentration range of nitrate (0.5-8.5 mM), which is within the physiological range of human saliva. In this plot, averages (black dots) with standard deviations, as well as upper and lower quartiles (grey lines) are shown. NO3: nitrate. All the different concentrations of nitrate were compared with 0 mM nitrate and significance was marked with * for p < 0.05 and ** for p < 0.01 according to a Wilcoxon test.

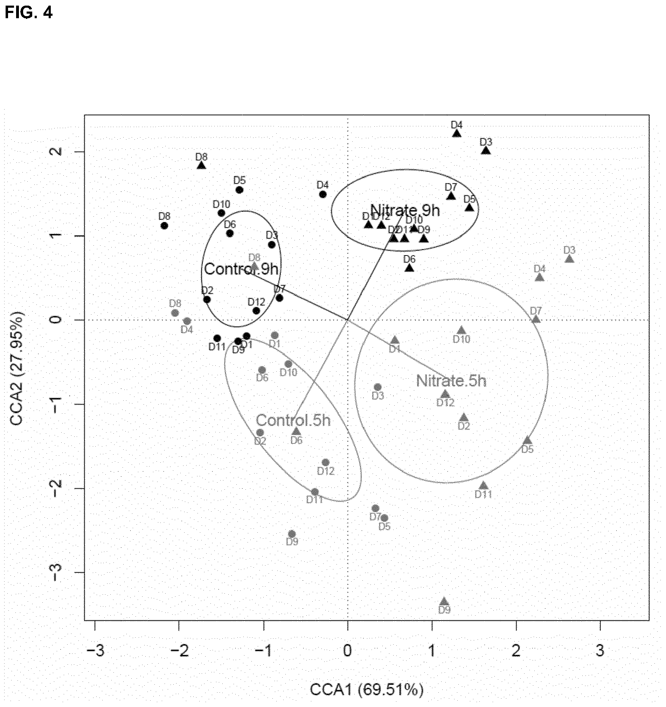

**FIG. 4****:** Differences in bacterial composition, as shown by Canonical Correspondence Analysis (CCA) for control (circles) and 6.5 mM nitrate (triangles) biofilms at 5h (grey) and 9h (black) of growth. Both Adonis and CCA p-values (0.0017 and 0.001, respectively) suggest statistically significant differences between the four groups. The first constrained component clearly separates the two experimental conditions (control and nitrate), whereas the second component reflects variability due to time (5h and 9h), showing that both nitrate influences bacterial composition at the two timepoints of biofilm development.

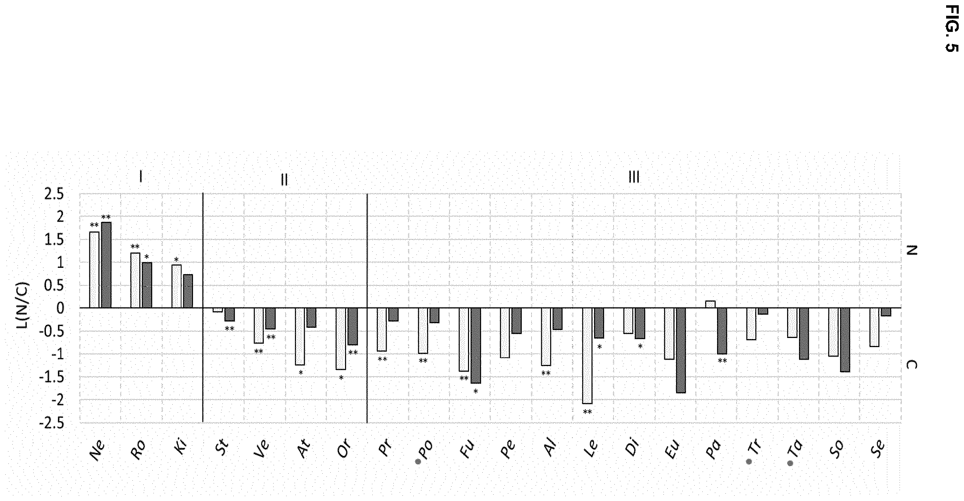

**FIG. 5****:** Changes in biofilm bacterial composition under nitrate conditions. Bar graphs show the log2 value of the ratio [average abundance nitrate condition]/[average abundance control condition] of the 12 donors. Genera shown are those significantly different between the nitrate and control conditions at 5h or 9h (* unadjusted p < 0.05 and ** unadjusted p < 0.01 according to a Wilcoxon test) or where a trend of change was observed. Group I include bacterial genera associated with oral health, group II are genera associated with caries and group III are genera associated with periodontitis and/or halitosis. Each group is sorted from most abundant to least abundant genera. Ne: Neisseria. Ro: *Rothia. Ki: Kingella. St: Streptococcus. Ve: Veillonella. At: Atopobium. Or. Oribacterium. Pr. Prevotella. Po: Porphyromonas. Fu: Fusobacterium. Pe: Peptostreptococcus. AI: Alloprevotella. Le: Leptotrichia. Di: Dialister. Fu: Eubacterium. Pa: Parvimonas. Tr. Treponema. Ta: Tannerella. So: Solobacterium. Se: Selenomonas.* Grey circles are placed before the genera of the periodontitis-associated red-complex bacteria (Po, Tr and Ta). L(N/C): Log2(nitrate/control). Light grey bars: 5h biofilms; dark grey bars: 9h biofilms.

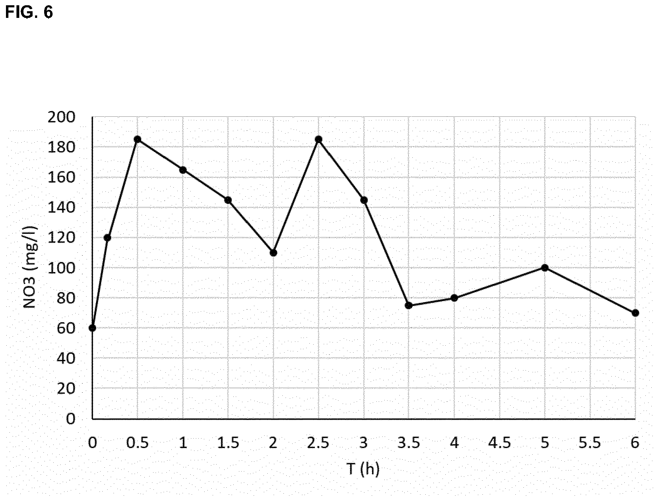

**FIG. 6****:** Nitrate levels in saliva collected in the morning in a healthy donor under fasting conditions, after intake of a nitrate-rich supplement (220 mg nitrate in 200 ml water) right after 0h. Two peaks are observed, illustrating the direct increase of nitrate due to the topical supplement contact (0.5 h) and the indirect increase due to the salivary gland activity that recycles nitrate from plasma (2.5 h). NO3: nitrate. T: time.

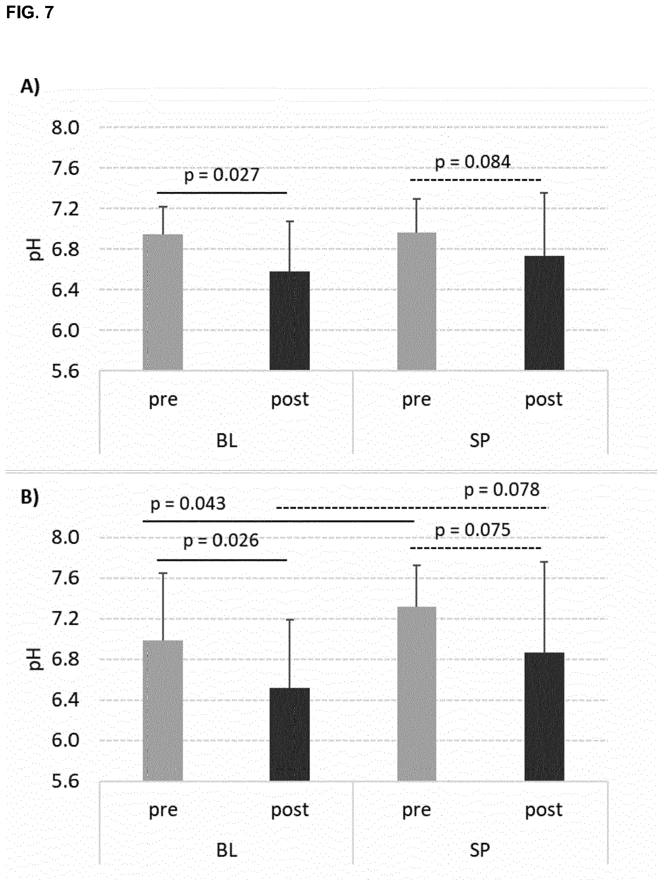

**FIG. 7****:** Pre- and post-sugar mouth rinse salivary pH values before and after a single dose of a nitrate-rich supplement. Data show the acute effect on bacterial activity after the nitrate composition is topically administered (A, study 2: effect 1 h after 300 mg nitrate in 70 ml intake, n = 6) and ingested (B, study 3: effect 4h after 220 mg nitrate in ≥150 ml intake, n = 6). BL: baseline. SP: after supplement. Pre: pre-sugar mouth rinse. Post: post-sugar rinse. P-values determined with a Wilcoxon test in SPSS (v25).

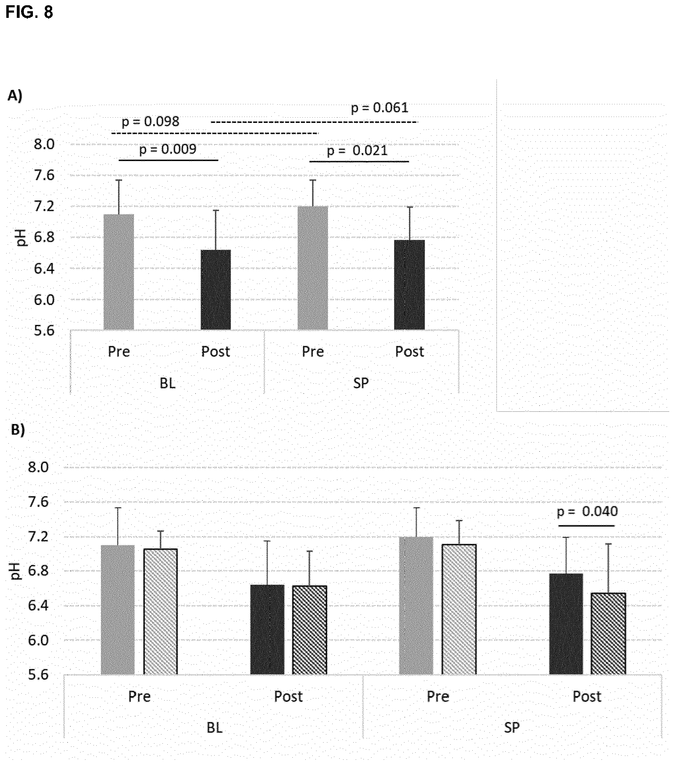

**FIG. 8****:** Pre- and post-sugar mouth rinse salivary pH values before and after a single dose of a nitrate-rich supplement. A: data show the acute effect on bacterial activity after the nitrate composition is ingested (study 4: 1 h 45 min after 250 mg nitrate in 200 ml intake, n = 12). B: bars show the difference in pH of this nitrate supplement with a placebo supplement (striped bars). BL: baseline. SP: after supplement. Pre: pre-sugar rinse. Post: post-sugar rinse. P-values determined with a Wilcoxon test in SPSS (v25).

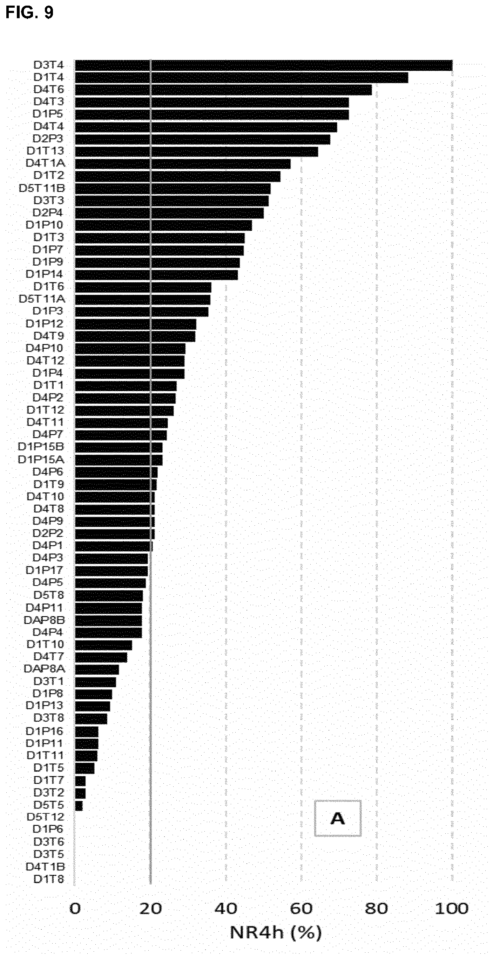

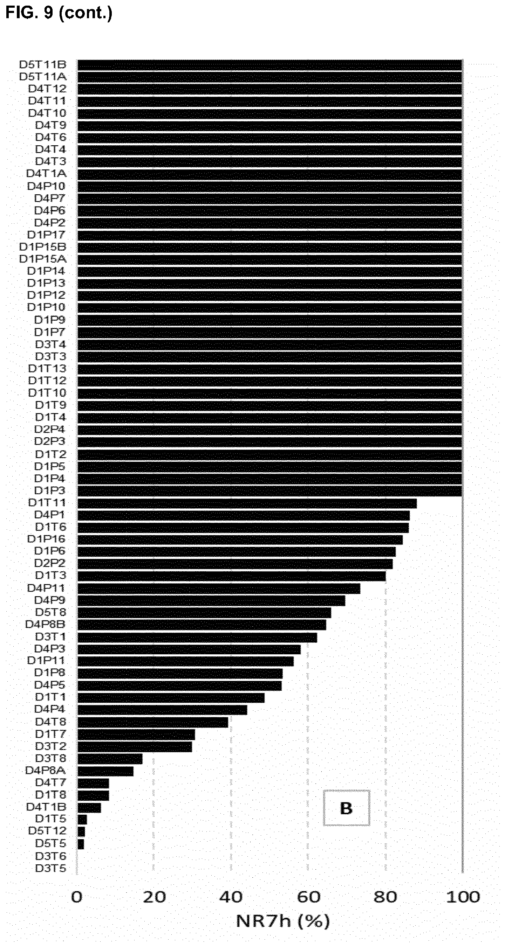

**FIG. 9****:** Percentage of nitrate reduced by all 67 oral bacterial isolates after 4 hours (A) and 7 hours (B) of incubation at 37 °C. Bars represent the percentage of initial nitrate that had been used up after 4h or 7h of incubation. The isolates that were selected as potential probiotics reduced at least 20% of the nitrate after 4h and 100% after 7h (grey lines). Other isolates, which are below the grey lines in A or B were not selected. On the y-axis are the names of the 67 isolates. NR4h: nitrate reduced after 4 hours. NR7h: nitrate reduced after 7 hours.

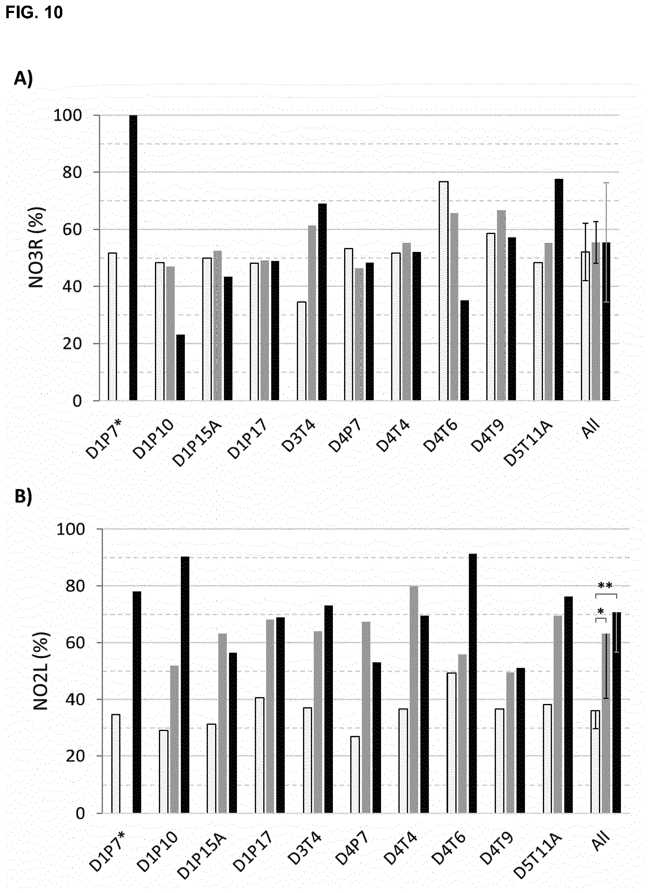

**FIG. 10****:** Percentage of (A) nitrate reduced and (B) nitrite left by 10 selected isolates after 5 hours of incubation with nitrate at different pH levels. Light grey bars with black borders are pH 6, dark grey bars are pH 7 and black bars are pH 7.5. A: The bars represent percentages of initial nitrate that had been used up (100% = all nitrate was used up). B: The percentage of nitrite left was calculated as nitrite detected after 5h, which is indicated as a percentage of the used up nitrate at this timepoint (100% is = no nitrite was further reduced to other compounds such as nitric oxide, 0% = all nitrite further reduced), taking into account nitrate to nitrite conversion is a 1:1 molar reaction. D1P7, D1P10, D1P15A, D1P17, D3T4, D4P7, D4T4, D4T6, D4T9 and D5T11A: selected isolate names. D1P7*: the pH 7 sample of D1P7 was lost. NO3R: nitrate reduced. NO2L: nitrite left. All: bars represent averages of all the isolates with their standard deviations. *p < 0.05, ** p <0.01 according to a Wilcoxon test in SPSS (v25).

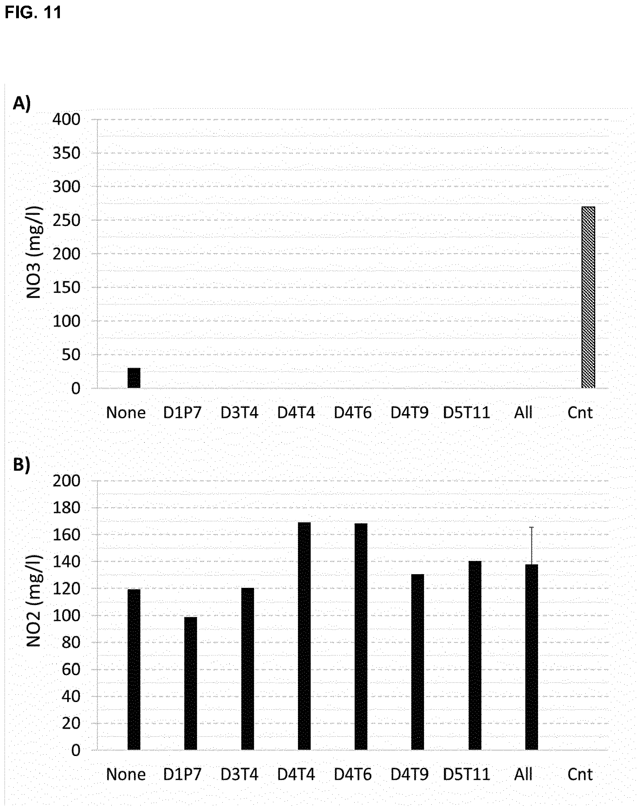

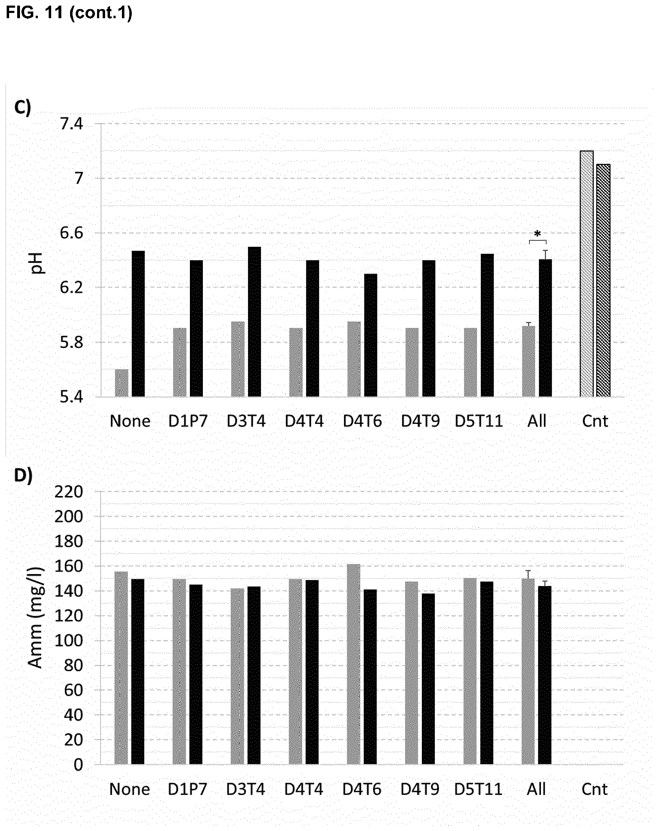

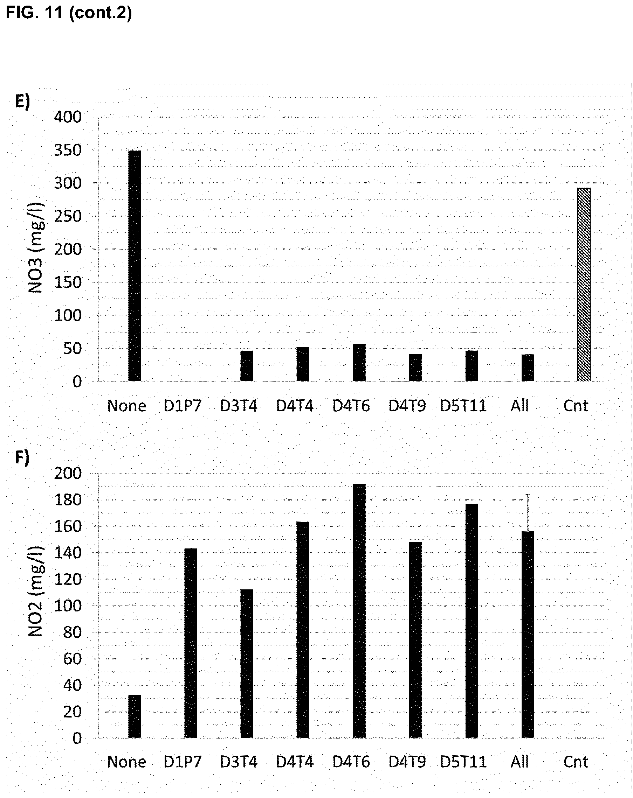

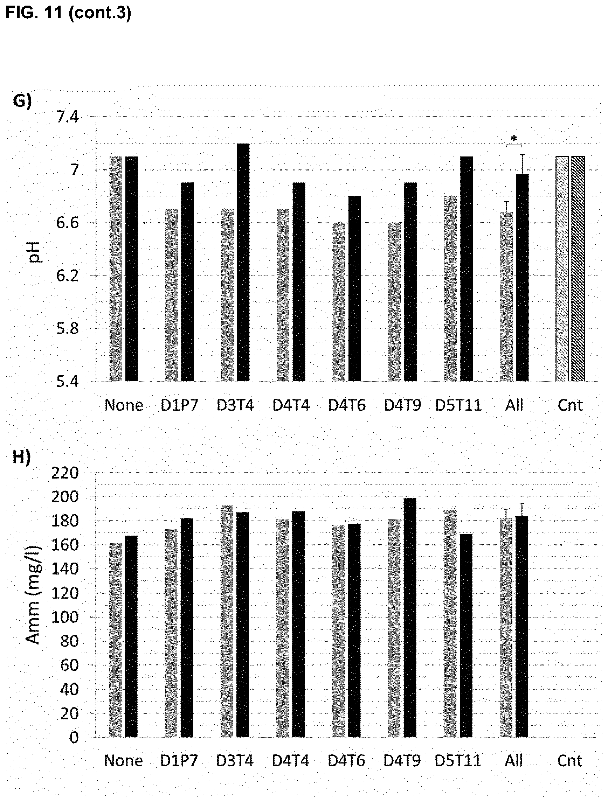

**FIG. 11****:** Nitrate (A & E), nitrite (B & F), pH (C & G) and ammonium (D & H) measurements after 5 h 30 min of biofilms grown from saliva with different probiotics in the absence (control: grey bars) or presence (nitrate: black bars) of 6.5 mM nitrate. Donor 1 (A-D) reduces nitrate and has a more acidic salivary pH, while donor 2 (E-H) does not reduce nitrate and has a neutral salivary pH. None: no probiotic was added. D1P7, D3T4, D4T4, D4T6, D4T9, D5T11: the biofilms were grown with one of these probiotics. All: the average of the different probiotics with standard deviations (*p < 0.05 between control and nitrate conditions according to a Wilcoxon test in SPSS v25). Cnt: measurements in the culture medium with (black striped bars) or without (grey striped bars) 6.5 mM nitrate. When there is a black or grey bar missing, the measurement was 0. NO3: Nitrate. NO2: Nitrite. Amm: ammonium. D5T11 in FIG. 11 corresponds to D5T11A.

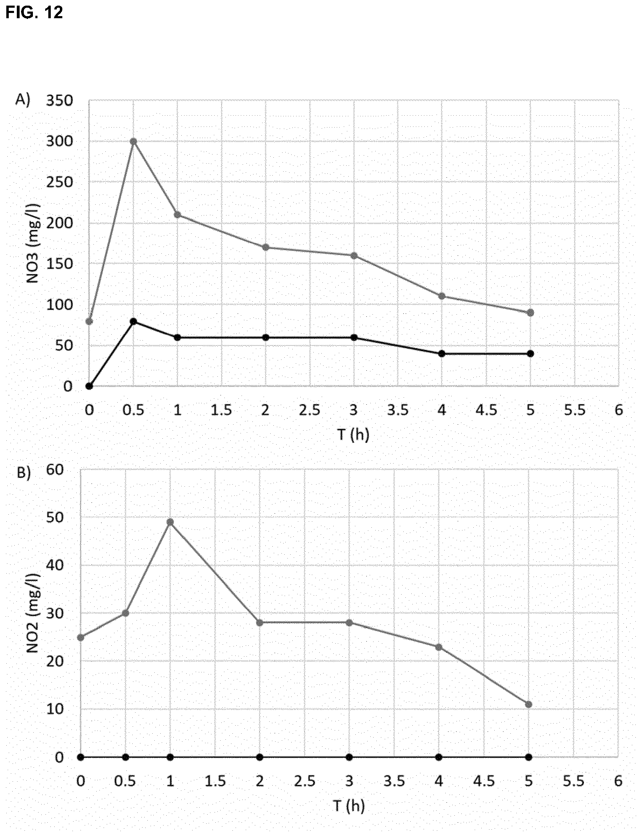

**FIG. 12****:** Nitrate (A) and nitrite (B) levels in saliva collected in the morning in two healthy donors under fasting conditions after intake of a Beetroot extract containing 3% nitrate (Beta vulgaris) dose containing 220 mg nitrate, which was dissolved in 200 ml water. The beetroot extract was taken right after 0h. One donor (D1, good nitrate reducer, grey line) appears to reduce the nitrate from the supplement, producing nitrite. The salivary nitrate of the other donor (D25, bad nitrate reducer, black line) does not increase as much and there is no nitrite detected, indicating a lack of nitrate reduction. NO3: nitrate. NO2: nitrite. T: time.

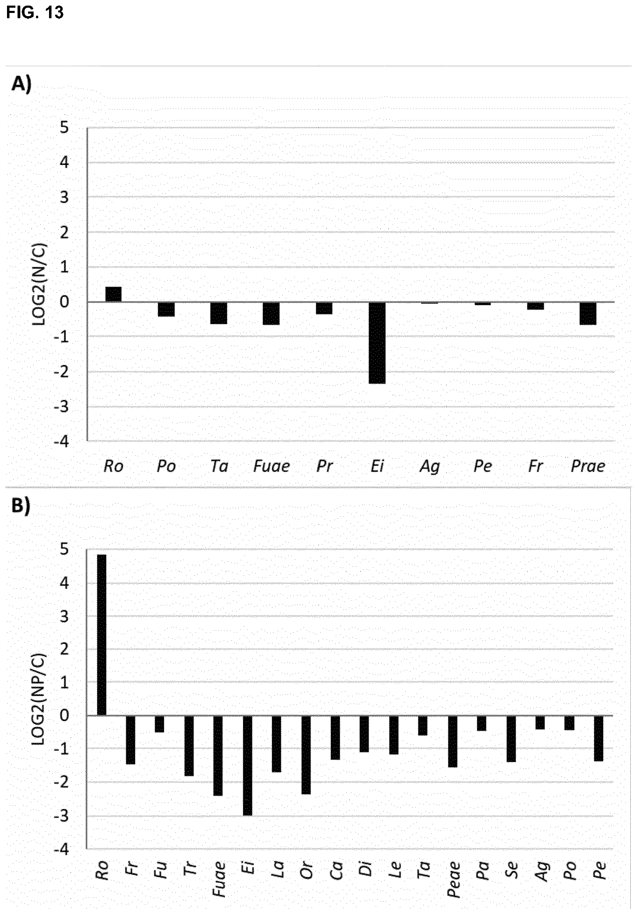

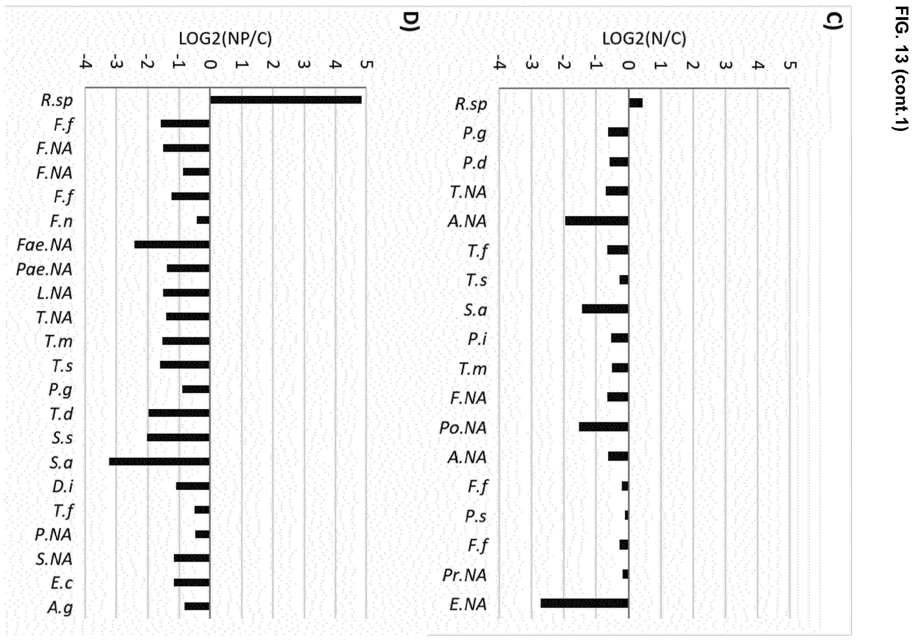

**FIG. 13****:** Differences in ex *vivo* periodontal biofilm bacterial composition under nitrate (N) and nitrate plus probiotic (NP) conditions compared to control biofilms without nitrate and without probiotic (C). The probiotic added in the NP condition was *Rothia aeria* D1P7 (CECT9999). Bar graphs show the log2 value of the ratio [average abundance nitrate condition]/[average abundance control condition] of the biofilms grown from subgingival samples from 11 donors with periodontal disease after 7 hours of anaerobic growth. Changes in Genera (Panels A and B) and Species (panels C and D) are shown, sorted from most different to least different bacteria between nitrate and control conditions. Genera and species with positive values are those at higher levels after nitrate treatment relative to their levels in the control (no nitrate) condition. Genera and species with negative values are those at lower levels after nitrate treatment relative to their levels in the control (no nitrate) condition. L(N/C): Log2(mean abundance nitrate/mean abundance control). L(NP/C): Log2(mean abundance nitrate+probiotic/mean abundance control).

| | | | |
|---|---|---|---|
| GENUS NAMES | (A-Z) | SPECIES NAMES | (A-Z) |
| Ag = | *Aggregatibacter* | Ag.NA = | *Aggregatibacter_NA* |
| Ca = | *Campylobacter* | Al. NA = | *Alloprevotella_NA* |
| Di = | *Dialister* | A.g = | *Anaeroglobus_geminatus* |
| Ei = | *Eikenella* | D.i = | *Dialister_invisus* |
| Fr = | *Fretibacterium* | E.c = | *Eikenella_corrodens* |
| Fuae = | *Fusobacteriaceae_NA* | E.NA = | *Eikenella_NA* |
| Fu = | *Fusobacterium* | F.fa = | *Fretibacterium_fastidiosum* |
| La = | *Lachnoanaerobaculum* | F.fe = | *Fretibacterium_feline* |
| Le = | *Leptotrichia* | Fr.NA = | *Fretibacterium_NA* |
| Or = | *Oribacterium* | Fuae.NA = | *Fusobacteriaceae_NA* |
| Pa = | *Parvimonas* | Fu.NA = | *Fusobacterium_NA* |
| Peae = | *Peptostreptococcaceae_NA* | F.n = | *Fusobacterium_nucleatum* |
| Pe = | *Peptostreptococcus* | L.NA = | *Leptotrichia_NA* |
| Po = | *Porphyromonas* | P.NA = | *Parvimonas_NA* |
| Pr = | *Prevotella* | P.s = | *Peptostreptococcus_stomatis* |
| Prae = | *Prevotellaceae_UCG-001* | P.g = | *Porphyromonas_gingivalis* |
| Ro = | *Rothia* | Po.NA = | *Porphyromonas_NA* |
| Se = | *Selenomonas* | P.d = | *Prevotella_dentalis* |
| Ta = | *Tannerella* | P.i = | *Prevotella_intermedia* |
| Tr = | *Treponema* | Pr.NA = | *Prevotella_NA* |
| | | Pae.NA= | *Prevotellaceae_NA* |
| | | R.NA = | *Rothia_NA* |
| | | S.a = | *Selenomonas_arfemidis* |
| | | S.NA = | *Selenomonas_NA* |
| | | S.s = | *Selenomonas_sputigena* |
| | | T.f = | *Tannerella_forsythia* |
| | | T.NA = | *Tannerella_NA* |
| | | T.d = | *Treponema_denticola* |
| | | T.m = | *Treponema_maltophilum* |
| | | T.s = | *Treponema_socranskii* |

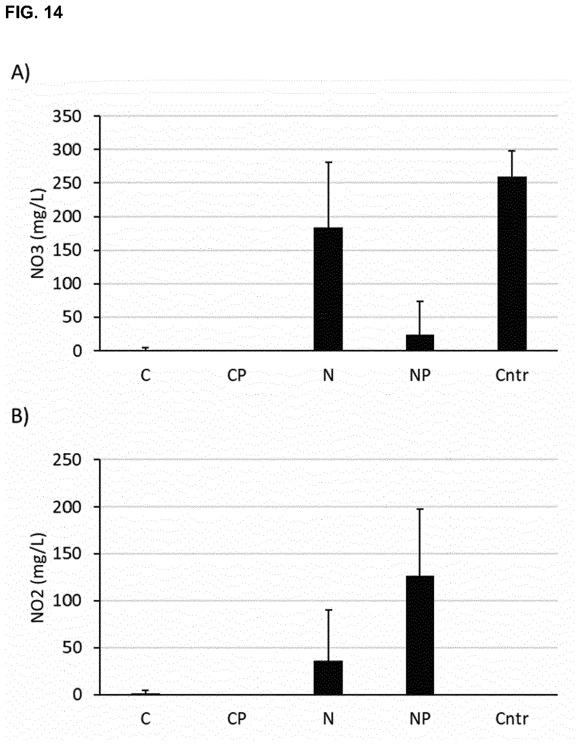

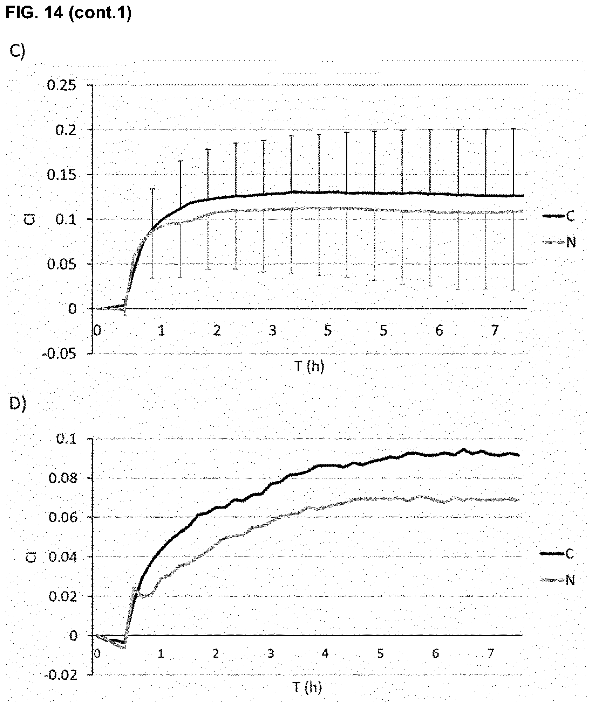

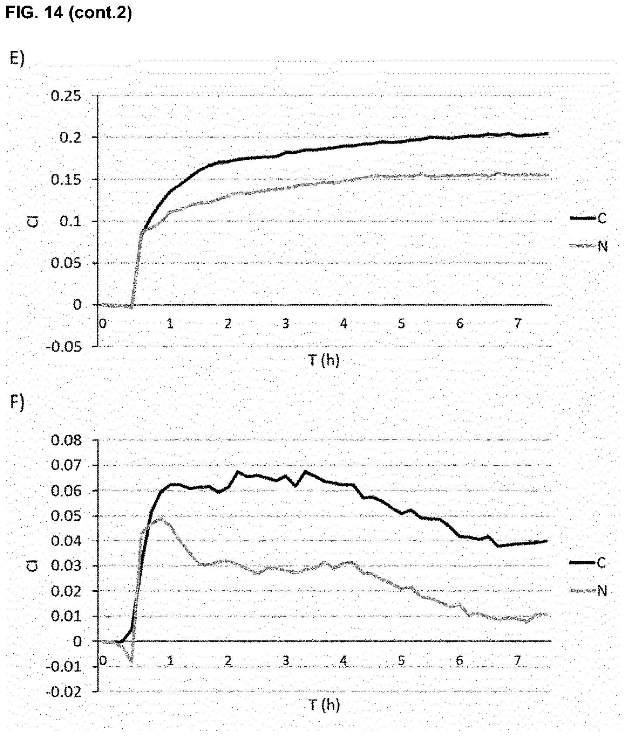

**FIG. 14****:** Nitrate reduction capacity in periodontal samples grown *ex vivo.* Bar graphs show the amount of nitrate left (FIG. 14A) and nitrite produced (FIG. 14B) by biofilms derived from periodontal samples (n=11). Biofilms were grown anaerobically for 7 hours in the absence of nitrate (Control condition, C), in the absence of nitrate but with the probiotic isolate *Rothia aeria* D1P7 (Control+Probiotic condition, CP), with 6.5 mM nitrate (prebiotic Nitrate condition, N), or with nitrate plus the probiotic isolate *Rothia aeria* D1P7 (symbiotic Nitrate+Probiotic condition, NP). The amount of nitrate added is indicated by the Cntr bar (250 mg/L). NO3: nitrate; NO2: nitrite. FIG. 14C-F show biofilms grown for 7h from subgingival samples of patients with periodontitis as inoculum in the 5 mM nitrate condition (N, black) and the control condition with 0 mM nitrate (C, grey). FIG. 14C: the average growth curves of all patients. The values presented are averages of all 11 patients with their corresponding standard deviations. FIG. 14D: the individual growth curves of patient 4. FIG. 14E: the individual growth curves of patient 5. FIG. 14F: the individual growth curve of patient 6. The plots show biofilm mass, expressed as Cell Index (CI) values over time (T) in hours (h), as indicated by the xCELLigence system, after normalization with BHI medium with 5 mM nitrate (for the N condition) or 0 mM nitrate (for the C condition). Measurements were taken every 10 minutes, but in FIG. 14C, error bars with standard deviations are only shown at half an hour intervals for clarity.

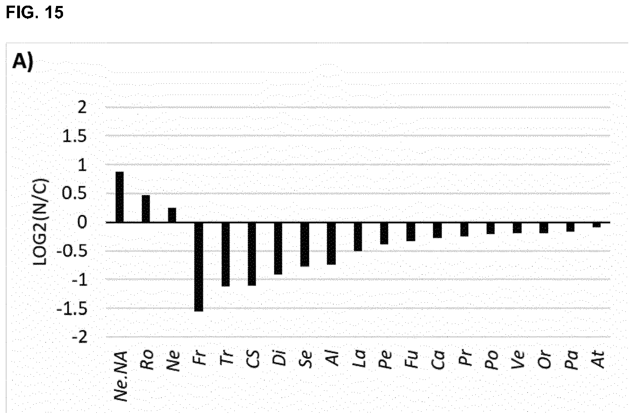

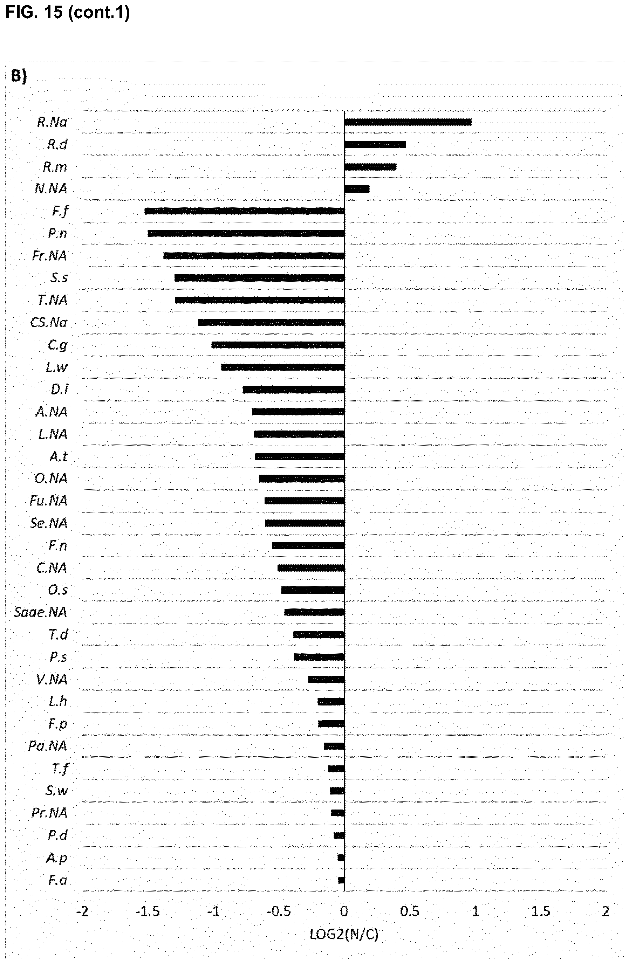

**FIG. 15****:** Bacterial composition in saliva samples collected after 4 hours of ingesting a nitrate-rich supplement (study 3: 4h after 220 mg nitrate in ≥150 ml intake, n=6). Bacterial composition was obtained by Illumina sequencing of the 16S rRNA gene. Graphs show bacteria at the genus (panel A) and species-level (panel B) assignment. Genera and species with positive values are those at higher levels after nitrate ingestion relative to 4 hours after ≥150 ml water consumption on a control day (without nitrate). Bacteria are sorted from most different to least different between 4-hour samples and baseline. Log2(N/C): Log2(mean abundance nitrate/mean abundance control).

| | | | |
|---|---|---|---|
| FIG. 15A: | | FIG. 15B: | |
| Ne.NA *=* | *Neisseriaceae* Not Assigned genus | F.a = | *Filifactor alocis* |
| Ro *=* | *Rothia* | A.p *=* | *Atopobium parvulum* |
| Ne *=* | *Neisseria* | P.d *=* | *Prevotella denticola* |
| Fr *=* | *Fretibacterium* | Pr.NA *=* | *Prevotella* Not Assigned species |
| Tr *=* | *Treponema* | S.w *=* | *Scardovia wiggsiae* |
| CS *=* | *Candidates Saccharimonas* | T.f *=* | *Tannerella forsythia* |
| Di *=* | *Dialister* | Pa.NA *=* | *Parvimonas* Not Assigned species |
| Se *=* | *Selenomonas* | F.p *=* | *Fusobacterium periodonticum* |
| Al *=* | *Alloprevotella* | L.h *=* | *Leptotrichia hongkongensis* |
| La *=* | *Lactobacillus* | V.NA = | *Veillonella* Not Assigned species |
| Pe *=* | *Peptostreptococcus* | P.s *=* | *Peptostreptococcus stomatis* |
| Fu *=* | *Fusobacterium* | T.d *=* | *Treponema denticola* |
| Ca *=* | *Campylobacter* | Saae.NA = | *Saccharimonadaceae* Not Assigned species |
| Pr *=* | *Prevotella* | O.s = | *Oribacterium sinus* |
| Po *=* | *Porphyromonas* | C.NA = | *Campylobacter* Not Assigned species |
| Ve *=* | *Veillonella* | F.n = | *Fusobacterium nucleatum* |
| Or *=* | *Oribacterium* | Se.NA = | *Selenomonas* Not Assigned species |
| Pa *=* | *Parvimonas* | Fu.NA = | *Fusobacterium* Not Assigned species |
| At *=* | *Atopobium* | O.NA = | *Oribacterium* Not Assigned species |
| | | A.t *=* | *Alloprevotella tannerae* |
| | | L.NA *=* | *Lactobacillus* Not Assigned species |
| | | A.NA *=* | *Alloprevotella* Not Assigned species |
| | | D.i *=* | *Dialister invisus* |
| | | L.w *=* | *Leptotrichia wadei* |
| | | C.g *=* | *Campylobacter gracilis* |
| | | CS.Na = | *Candidates Saccharimonas* Not Assigned species |
| | | T.NA *=* | *Treponema* Not Assigned species |
| | | S.s *=* | *Selenomonas sputigena* |
| | | Fr.NA *=* | *Fretibacterium* Not Assigned species |
| | | P.n *=* | *Prevotella nigrescens* |
| | | F.f *=* | *Fretibacterium fastidiosum* |
| | | N.NA *=* | *Neisseria* Not Assigned species |
| | | R. m *=* | *Rothia mucilaginosa* |
| | | R.d *=* | *Rothia dentocariosa* |
| | | R.Na *=* | *Rothia* Not Assigned species |

### DETAILED DESCRIPTION OF THE INVENTION

### Composition comprising nitrate for use in reducing or preventing oral dysbiosis and/or increasing oral eubiosis

The main features of the invention have already been explained in the above Summary of the invention and Definitions sections.

As discussed, the composition of the invention not only reduces oral dysbiosis but also increases oral eubiosis, which may be considered as a great contribution to the art.

Health-associated bacteria in oral biofilms are *Neisseria, Rothia* and *Kingella.*

Caries-associated bacteria in oral biofilms are *Streptococcus, Veillonella, Oribacterium* and *Atopobium.*

Periodontal diseases/halitosis-associated bacteria in oral biofilms are *Porphyromonas, Fusobacterium, Leptotrichia, Prevotella, Treponema, Tannerella, Alloprevotella, Peptostreptococcus, Dialister, Eubacterium, Parvimonas, Selenomonas,* and *Solobacterium.*

Classic bacteria associated with periodontitis include *Porphyromonas gingivalis, Treponema denticola, Tannerella forsythia, Fusobacterium nucleatum, Prevotella intermedia, Parvimonas micra* and *Aggregatibacter actinomycetemcomitans.* However, in a recent systematic review, 17 other species were associated to the disease, including (other) species from the genera *Eubacterium, Selenomonas, Dialister, Peptostreptococcus, Alloprevotella, Porphyromonas, Treponema* and *Prevotella. Porphyromonas, Fusobacterium, Leptotrichia* and *Prevotella* are also associated with halitosis - bad breath resulting from microbial production of volatile sulfur compounds (VSCs). Additionally, a classic biomarker for halitosis is the VSC producing *Solobacterium moorei,* which has also been associated with periodontitis.

As discussed in the "Results and Conclusions" sections of working EXAMPLE 1 herein, the inventors made an *ex vivo* study to test the effects of nitrate on oral biofilm growth and identified e.g. that after an "acute" treatment (5 h and 9 h after nitrate addition), the administered nitrate:
- increases the amount of at least one health-associated bacteria of oral biofilms selected from the group consisting of *Neisseria, Rothia* and *Kingella* in the oral biofilms, and/or
- decreases the amount of at least one caries-associated bacteria of oral biofilms selected from the group consisting of *Streptococcus, Veillonella, Oribacterium* and *Atopobium* in the oral biofilms, and/or
- decreases the amount of at least one periodontal diseases/halitosis-associated bacteria of oral biofilms selected from the group consisting of *Porphyromonas, Fusobacterium, Leptotrichia, Prevotella, Treponema, Tannerella, Alloprevotella, Peptostreptococcus, Dialister, Eubacterium, Parvimonas, Selenomonas,* and *Solobacterium* in the oral biofilms.

Thus, in a particular embodiment, the administered nitrate increases the amount of at least one health-associated bacteria of oral biofilms selected from the group consisting of *Neisseria, Rothia* and *Kingella* in the oral biofilms, thereby increasing oral eubiosis. The increase in the amount of these bacteria is beneficial for all oral diseases. It is the first time that it's shown that a compound increases the levels of health-associated bacteria by several fold already after 5 h. This is a unique property of nitrate and has not been shown for any other compound before.

In a more particular embodiment, the administered nitrate increases the amount of at least one health-associated bacteria of oral biofilms selected from the group consisting of *Neisseria* and *Rothia* in the oral biofilms. More particularly, the administered nitrate increases the amount of *Neisseria* and *Rothia* bacteria in the oral biofilms.

In a particular embodiment, the administered nitrate decreases the amount of at least one caries-associated bacteria of oral biofilms selected from the group consisting of *Streptococcus, Veillonella, Oribacterium* and *Atopobium* in the oral biofilms. It is the first time that it is shown that a compound decreases all of these caries-associated bacteria together with their metabolite lactate (the main acid involved in caries development), which shows a strong anticariogenic potential of nitrate. Additionally, it is surprising that this happens after a single dose of nitrate <24h.

In a more particular embodiment, the administered nitrate decreases the amount of at least one caries-associated bacteria of oral biofilms selected from the group consisting of *Streptococcus, Veillonella,* and *Oribacterium* in the oral biofilms. More particularly, the administered nitrate decreases the amount of *Streptococcus, Veillonella, and Oribacterium bacteria* and more particularly also of *Atopobium* bacteria in the oral biofilms. Remarkably, this list includes not only bacteria classically associated to caries but also to other bacteria more rare or recently related to the disease with the use of modern sequencing techniques e.g. *Oribacterium* and *Atopobium.*

In another embodiment, the administered nitrate decreases the amount of at least one periodontal diseases/halitosis-associated bacteria of oral biofilms selected from the group consisting of *Porphyromonas, Fusobacterium, Leptotrichia, Prevotella, Treponema, Tannerella, Alloprevotella, Peptostreptococcus, Dialister, Eubacterium, Parvimonas, Selenomonas,* and *Solobacterium* in the oral biofilms. Remarkably, this list includes not only bacteria classically associated to periodontitis-like red complex bacteria *(Porphyromonas, Treponema,* and *Tannerella)* but also to other bacteria more recently related to the disease with the use of modern sequencing techniques e.g. *Eubacterium* and *Alloprevotella.* It is the first time that it is shown that a compound decreases all of these periodontal diseases- and halitosis-associated bacteria together, which shows a strong prebiotic potential of nitrate against these diseases. This is a unique property of nitrate and has not been shown for any other compound before. Furthermore, it is the first time that different caries-associated and periodontitis-associated bacteria decrease at the same time. In light of this, it is the first time that any compound decreases periopathogenic bacteria, while increasing ammonium and decreasing lactate. Finally, it is surprising that all this happens after a single dose of nitrate <24h.

In a more particular embodiment, the administered nitrate decreases the amount of at least one periodontal diseases/halitosis-associated bacteria of oral biofilms selected from the group consisting of *Porphyromonas, Fusobacterium, Leptotrichia, Prevotella, Treponema, Tannerella* and *Alloprevotella* in the oral biofilms. More particularly, the administered nitrate decreases the amount of *Porphyromonas, Fusobacterium, Leptotrichia, Prevotella, Treponema, Tannerella* and *Alloprevotella* bacteria and more particularly also of one or more of *Peptostreptococcus, Dialister, Eubacterium, Parvimonas, Selenomonas,* and *Solobacterium* bacteria in the oral biofilms.

In a particular embodiment, the administered nitrate:
- increases the amount of at least one health-associated bacteria of oral biofilms selected from the group consisting of *Neisseria, Rothia* and *Kingella* in the oral biofilms, and
- decreases the amount of at least one caries-associated bacteria of oral biofilms selected from the group consisting of *Streptococcus, Veillonella, Oribacterium* and *Atopobium* in the oral biofilms, and
- decreases the amount of at least one periodontal diseases/halitosis-associated bacteria of oral biofilms selected from the group consisting of *Porphyromonas, Fusobacterium, Leptotrichia, Prevotella, Treponema, Tannerella, Alloprevotella, Peptostreptococcus, Dialister, Eubacterium, Parvimonas, Selenomonas,* and *Solobacterium* in the oral biofilms.

As discussed, the administration of nitrate changes the bacterial composition of the oral biofilms but also the functions. Thus, as is seen in EXAMPLE 1, in particular embodiments:
1) The administered nitrate decreases lactate production and increases the level of ammonium in oral biofilms, relevant to prevent and treat caries development;
2) The administered nitrate reduces volatile sulfur compounds (VSCs) and VSCs-producing bacteria involved in halitosis and periodontitis;
3) Impressively, even though ammonium increases, periodontal diseases/halitosis-associated bacteria decrease, while they like a neutral or slightly alkaline pH. This is why it can be said that nitrate stimulates a general symbiotic composition, and does not prevent one disease while pushing toward another disease (that is the case of the use of arginine);
4) The administered nitrate increases nitrite production, relevant for systemic conditions and states that benefit from increased systemic nitric oxide levels;
5) The administered nitrate increases the nitrate reduction capacity of the oral microbiota by increasing nitrate reducing bacteria associated to oral health; and/or
6) The administered nitrate decreases bacteria (e.g., *Porphyromonas, Treponema, Tannerella*) that trigger gingival inflammation and increases bacteria (e.g., *Rothia* and *Neisseria*) that reduce gingival inflammation.

Regarding point (2), CSVs such as hydrogen sulfide (H₂S) and methyl mercaptan (CH₃SH), on the one hand, are the direct cause of halitosis and, on the other hand, damage periodontal tissue in periodontitis, because these gases, apart from smelling bad, are genotoxic and cause inflammation. This is why there is a clear correlation between halitosis and periodontitis. As discussed in EXAMPLE 1, nitrate reduces the bacteria that produce these gases. Apart from this, nitrate changes the metabolism of the microbial community, because bacteria can use sulfate or nitrate and nitrate gives more energy than sulfate. In other words, nitrate is favored when present and when observing nitrate reduction of a bacterial community, like in EXAMPLE 1, it can be concluded that sulfate reduction to H₂S, which some bacteria convert to CH₃SH, is inhibited. Therefore, the final products of sulfate reduction, H₂S and CH₃SH (smelly and toxic) are being replaced by nitric oxide (antimicrobial and beneficial). Regarding the bacteria, the administration of nitrate gives an advantage to nitrate-reducing and/or nitric oxide resistant bacteria and a disadvantage to those that reduce sulfate and/or that are sensitive to nitric oxide. In summary, it the results of EXAMPLE 1 show that there is an important shift from bacteria and metabolism associated with halitosis and periodontitis towards bacteria and metabolism associated with health.

Thus, in a particular embodiment, the composition comprising nitrate is used as a prebiotic to decrease VSC-producing bacteria and VSC production.

The effects of nitrate in changing the bacterial composition and functions of the oral biofilms may be tested by any method known in the art. A suitable method is the one described in EXAMPLE 1.

In an embodiment, the biofilm-mediated oral disease is caries and the administered nitrate decreases the amount of at least one caries-associated bacteria selected from the group consisting of *Streptococcus, Veillonella, Oribacterium* and *Atopobium* in the oral biofilms.

In an embodiment, the biofilm-mediated oral disease is a periodontal diseases/halitosis-associated bacteria of oral biofilms selected from the group consisting of *Porphyromonas, Fusobacterium, Leptotrichia, Prevotella, Treponema, Tannerella, Alloprevotella, Peptostreptococcus, Dialister, Eubacterium, Parvimonas, Selenomonas,* and *Solobacterium* in the oral biofilms.

One embodiment relates to the composition comprising nitrate herein provided for use in reducing the biofilm quantity (i.e. dental plaque or tongue coating mass), thereby getting an acute treatment or prevention with effects before 24 hours of a biofilm-mediated oral disease, and wherein the composition is orally administered to the mammal and thereby increases the concentration of nitrate in the saliva of the mouth. Such effect has been demonstrated in e.g. EXAMPLE 6.

In an embodiment, the mammal is a human. In another embodiment, the mammal is an animal such as a cat, a dog, a horse, a cow, a pig, a goat, a sheep, a donkey, a buffalo, an ox, a llama or a camel. In another embodiment, the biofilm-mediated oral disease is a periodontal disease or halitosis.

### Probiotic bacteria for increasing the nitrate-reduction capacity of a mammal

In EXAMPLE 3, it is shown that individuals have different nitrate reduction capacities (NRC, i.e., the capacity of their oral microbiota to reduce nitrate into nitrite) and it is obtained *in vitro* evidence that this capacity can be stimulated with nitrate-reducing probiotics. Furthermore, the inventors have identified particular bacteria with beneficial features to be used as probiotics in e.g. individuals with poor capacity of nitrate-reduction. Even in an individual with a low to undetectable NRC, the addition of nitrate-reducing probiotics resulted in a significant NRC *in vitro.*

Depending on the NRC of each individual, the composition to be administered will comprise nitrate without probiotic bacteria, probiotic bacteria without nitrate or nitrate and probiotic bacteria. Thus, as discussed above, an aspect of the invention relates to a composition comprising nitrate and/or a bacterial strain belonging to *Rothia, Neisseria* or *Kingella* genera, for use in increasing the nitrate-reduction capacity of a mammal, by acutely increasing the amount of nitrate-reducing bacteria in oral biofilms, thereby getting a treatment or prevention of oral diseases benefiting from nitrate-reduction (caries, periodontal diseases and halitosis), and a disease or state that benefits from an increase systemic nitric oxide levels.

It is believed that there is no prior art reporting nitrate-reducing probiotics able to restore or improve the NRC of an individual. The inventors have identified particular bacteria with relevant nitrate-reduction capacities in different conditions and with other relevant features to be used as probiotics. Thus, another aspect of the invention relates to a composition comprising a bacterial strain that has the following properties:
a) reduces 100% of nitrate after 7 h of incubation at 37 °C starting with an optical density (OD) of 0.01 in BHI medium with 6.5 mM nitrate;
b) reduces more than 15% of nitrate after 4 h of incubation at 37 °C starting with an OD of 0.01 in BHI medium with 6.5 mM nitrate;
c) does not decrease the pH of BHI medium with 6.5 mM nitrate after 7 h of incubation at 37°C starting with an OD of 0.01 below pH 6.8;
d) grows to an optical density over 0.7 after 7 h of growth in BHI medium with 6.5 mM nitrate at 37 °C starting with an optical density OD of 0.01; and
e) is able to colonize an *in vitro* oral biofilm grown from human saliva during 5 h at 37°C when adding 1:1 bacterial strain in BHI (OD 0.40):saliva inoculum, reaching a proportion of more than 10% of total bacteria in the formed biofilm. Details of the steps above described are in EXAMPLE 3 section 21-25.

It is understood that the bacterial strain is an isolated bacteria strain. As understood by the skilled person in the present context the term "isolated" relates to that the bacteria strain has been isolated from its natural environment - i.e. it is not present in its natural environment, so it is free from other organisms and substances present in the natural environment.

In a particular embodiment, the bacterial strain belongs to *Rothia* genus and has the features mentioned above (a)-(e). In other embodiments the bacterial strain belongs to *Rothia aeria, Rothia dentocariosa,* and *Rothia mucilaginosa.*

Based on the assays described above (steps (a)-(e)) and e.g in EXAMPLE 3, the skilled person is routinely able to repeat the assay to objectively determine whether a particular strain is encompassed by the present invention. Thus, another aspect of the invention relates to a method of screening of bacteria with nitrate-reduction capacities, the method comprising the steps mentioned above (a)-(e).

Through the Examples, different strains are provided for complying with the above-mentioned features. Further to said strains, by means of the method described in detail, it is plausible to identify and isolate other strains within a pool of bacteria, with the same features. It is also demonstrated by means of the Examples, that these features are beneficial for the purposes of the invention. Thus, although some specific strains have been tested and identified with these beneficial features, there is no reason to limit the scope of the invention to such strains because all the steps of the method to get other good strains are plausibly described herein. Therefore, the invention also provides a pool of strains other than the used in the Examples that have the same features. It is important to note that not all the bacterial strains will have the mentioned features; thus, the invention provides a method to recognize them. Similarly, it is relevant to note that the method described above is not limiting the scope of the invention. The assay is one suitable to test the desired features.

Particular bacterial strains were isolated from donor subjects without caries and periodontitis. Plaque or tongue coating samples were collected by a dentist and resuspended in 1 mL of PBS. Identification at species level was performed by sequencing 16S rRNA gene as described in EXAMPLE 3 section 23.

The strains were deposited in the Spanish Type Culture Collection (Universitat de València, Campus de Burjassot, Edif. de Investigación, 46100 Burjassot, València, Spain) on October 23, 2019 (23.10.2019). The deposited strains are viable and keep all their features related to their deposit.

In particular embodiments, the bacterial strain is one selected from the group consisting of:
- *Rothia aeria* deposited in the Spanish Type Culture Collection under the accession number CECT 9999 (internal code D1P7). Its 16S rRNA sequence corresponds to SEQ ID NO: 1, and when comparing to public databases, it shows a 99.72% nucleotide identity with *Rothia aeria* A1-17B.
- *Rothia dentocariosa* deposited as CECT 30000 (internal code D1P17). Its 16S rRNA sequence corresponds to SEQ ID NO: 2, and when comparing to public databases, it shows a 99.87% nucleotide identity with *Rothia dentocariosa* ATCC 17931.
- *Rothia mucilaginosa* deposited as CECT 30001 (internal code D3T4). Its 16S rRNA sequence corresponds to SEQ ID NO: 3, and when comparing to public databases, it shows a 99.07% nucleotide identity with *Rothia mucilaginosa* DSM 20746.
- *Rothia mucilaginosa* deposited as CECT 30002 (internal code D4T4). Its 16S rRNA sequence corresponds to SEQ ID NO: 4, and when comparing to public databases, it shows a 99.20% nucleotide identity with *Rothia mucilaginosa* DSM 20746.
- *Rothia mucilaginosa* deposited as CECT 30003 (internal code D4T6). Its 16S rRNA sequence corresponds to SEQ ID NO: 5, and when comparing to public databases, it shows a 99.20% nucleotide identity with *Rothia mucilaginosa* DSM 20746.
- *Rothia mucilaginosa* deposited as CECT 30004 (internal code D4T9). Its 16S rRNA sequence corresponds to SEQ ID NO: 6, and when comparing to public databases, it shows a 99.20% nucleotide identity with *Rothia mucilaginosa* DSM 20746.
- *Rothia mucilaginosa* deposited as CECT 30005 (internal code D5T11A). Its 16S rRNA sequence corresponds to SEQ ID NO: 7, and when comparing to public databases, it shows a 99.07% nucleotide identity with *Rothia mucilaginosa* DSM 20746.

The above strains were isolated from the oral cavity (P = dental plaque, T = tongue as indicated in the internal code D-Number-(P or T)-Number) in healthy individuals without dental caries, gum diseases nor halitosis, with healthy blood pressure and without visible oral mucosa alterations.

An aspect of the invention relates to a composition comprising a bacterial strain selected from the group consisting of strain deposited in the Spanish Type Culture Collection (CECT) under the accession number CECT 9999, CECT 30000, CECT 30001, CECT 30002, CECT 30003, CECT 30004, CECT 30005, or combinations thereof. In a particular embodiment, the composition comprises from 10⁴ to 10¹³ cfu/g of cells of at least one of the mentioned bacterial strains. Compositions of the present invention may comprise a single strain or be a combination of different bacterial strains.

It is clear that by using the deposited strains as starting material, the skilled person in the art can routinely, by conventional mutagenesis or re-isolation techniques, obtain further variants or mutants thereof that retain or enhance the herein described relevant features and advantages of the strains forming the composition of the invention. Thus, the invention also relates to variants of strains disclosed herein. As used herein, the term "variant" or "mutant" of a strain refers to any naturally-occurring or specifically developed strain obtained from the reference strain X, mainly by mutation, that maintains the features mentioned above. For example, the 16S rRNA gene of a "variant" strain as contemplated herein may share e.g. about 85 percent, 86 percent, 87 percent, 88 percent, 89 percent, 90 percent, 91 percent, 92 percent, 93 percent, 94 percent, 95 percent, 96 percent, 97 percent, 98 percent or 99 percent sequence identity with the 16S rRNA sequence SEQ ID NO: 1-SEQ ID NO 7 of a strain disclosed herein. In one particular embodiment, the mutants are obtained by using recombinant DNA technology. In another embodiment, the mutants are obtained by random mutagenesis. Thus, another aspect of the invention relates to a method to obtain a mutant of a deposited strain, wherein the method comprises using the deposited strain as starting material and applying mutagenesis, and wherein the obtained variant or mutant further retains or enhances at least the features of the deposited strain.

In a particular embodiment, the strains have been fermented in an artificial medium and submitted to a post-treatment after the fermentation, to obtain bacterial cells, and the resulting bacterial cells are in a liquid medium or in a solid form. Particularly, the post-treatment is selected from the group consisting of: drying, freezing, freeze-drying, fluid bed-drying, spray-drying and refrigerating in liquid medium, and more particularly, is freeze-drying.

The strains of the invention are produced by cultivating the bacteria in a suitable artificial medium and under suitable conditions. By the expression "artificial medium" for microorganisms is to be understood a medium containing natural substances, and optionally synthetic chemicals such as the polymer polyvinyl alcohol, which can reproduce some of the functions of serums. Common suitable artificial media are nutrient broths that contain the elements including a carbon source (e.g. glucose), a nitrogen source (e.g. amino acids and proteins), water and salts needed for bacterial growth. Growth media can be liquid form or often mixed with agar or other gelling agent to obtain a solid medium. The strains can be cultivated alone to form a pure culture, or as a mixed culture together with other microorganisms, or by cultivating bacteria of different types separately and then combining them in the desired proportions. After cultivation, and depending on the final formulation, the strains may be used as purified bacteria, or alternatively, the bacterial culture or the cell suspension may be used, either as such or after an appropriate post-treatment. In this description, the term "biomass" is understood the bacterial strains culture obtained after cultivation.

By the term "post-treatment" is to be understood in the context of the present invention, any processing carried out on the biomass with the aim of obtaining storable bacterial cells. The objective of the post-treatment is decreasing the metabolic activity of the cells in the biomass, and thus, slowing the rate of cellular deleterious reactions. As a result of the post-treatment, the bacterial cells can be in solid or liquid form. In solid form, the stored bacterial cells can be a powder or granules. In any case, both the solid and liquid forms containing the bacterial cells are not present in the nature, hence, are not naturally-occurring, since they are the result of artificial post-treatment process(es). The post-treatment processes may in particular embodiments require the use of one or more of so-called post-treatment agent. In the context of the present invention, the expression "post-treatment agent" refers to a compound used to perform the herein described post-treatment processes. Among the post-treatment agents are to be included, without limitation, dehydrating agents, bacteriostatic agents, cryoprotective agents (cryoprotectants), inert fillers (also known as lyoprotectants), carrier material (also known as core material), etc., either used alone or in combination.

There are two basic approaches to decrease the metabolic activity of the bacterial cells, and thus, two approaches to carry out the post-treatment. The first one is decreasing the rate of all chemical reactions, which can be done lowering the temperature by refrigerating or freezing using refrigerators, mechanical freezers, and liquid nitrogen freezers. Alternatively, decreasing the rate of all chemical reactions can be achieved by adding substances that inhibit the growth of the bacterial cells, namely a bacteriostatic agent, abbreviated Bstatic.

The second approach to carry out the post-treatment is to remove water from the biomass, a process which can involve sublimation of water using a lyophilizer. Suitable techniques to remove water from the biomass are drying, freeze-drying, spray-drying or fluid bed-drying. Post-treatments that result in solid form may be drying, freezing, freeze-drying, fluid bed-drying, or spray-drying.

The post-treatment is preferably freeze-drying, which involves the removal of water from frozen bacterial suspensions by sublimation under reduced pressure. This process consists of three steps: pre-freezing the product to form a frozen structure, primary drying to remove most water, and secondary drying to remove bound water. Due to objective and expected variability of industrial processes for manufacturing and isolation of lyophilized bacterial cultures, the latter commonly contain certain amount of inert filler also known as lyoprotectant. Its role is to standardize the content of live probiotic bacteria in the product. The following inert fillers in commercially available lyophilized cultures are used: sucrose, saccharose, lactose, trehalose, glucose, maltose, maltodextrin, corn starch, inulin, and other pharmaceutically acceptable non-hygroscopic fillers. Optionally, other stabilizing or freeze-protecting agents like ascorbic acid, are also used to form a viscous paste, which is submitted to freeze-drying. In any case, the so-obtained material can be grinded to appropriate size, including to a powder.

The strains forming the composition of the invention are preferably in the form of viable cells. However, the strains of the invention can also be in the form of non-viable cells such as killed cultures or compositions containing beneficial factors (such as enzymes and antibacterial peptides) produced by the strains identified herein. This could include thermally killed micro-organisms or micro-organisms killed by exposure to altered pH, sonication, radiation or subjection to pressure. With non-viable cells product preparation is simpler, cells may be incorporated easily into commercial products and storage requirements are much less limited than viable cells.

### Medicalloral care uses of the compositions of the invention

As said, the first aspect of the invention relates to compositions comprising nitrate (and particularly can also comprise probiotic bacteria) for use in reducing or preventing oral dysbiosis and/or increasing oral eubiosis, by changing the bacterial composition and functions thereof of oral biofilms in a mammal, by decreasing the amount of disease-associated bacteria and bacterial functions and increasing the amount of health-associated bacteria and bacterial functions, thereby getting an acute treatment or prevention with effects before 24 hours of a biofilm-mediated oral disease, and wherein the composition is orally administered to the mammal and thereby increases the concentration of nitrate in the saliva of the mouth. Alternatively, this aspect can be formulated as the use of a composition of the invention for the manufacture of a medicament for reducing or preventing oral dysbiosis and/or increasing oral eubiosis in the terms described above. Also alternatively, the invention provides a method for reducing or preventing oral dysbiosis and/or increasing oral eubiosis in the terms described above in a mammal, including a human, comprising administering to said mammal in need thereof the defined composition.

In a particular embodiment, the biofilm-mediated oral disease is selected from the group consisting of a periodontal disease, halitosis and caries.

Periodontal diseases, also known as gum diseases, are a set of inflammatory conditions affecting the tissues surrounding the teeth. In its early stage, called gingivitis, the gums become swollen, red, and may bleed. In its more serious form, called periodontitis, the host tissue is lost resulting from destructive inflammation and proteolytic activity of the dysbiotic microbiota. Due to the proteolytic activity of bacteria that metabolize gingival crevicular fluid proteins and tissue-breakdown products, the pH stays neutral or slightly alkaline. In periodontitis, the gums can pull away from the tooth, bone can be lost, and the teeth may loosen or fall out. Bad breath may also occur. When this disease is associated with an implant it is called peri-implantitis in which bone loss tends to develop faster.

More particularly, the periodontal disease is selected from the group consisting of periodontitis, gingivitis and periimplantitis.

In a particular embodiment, the biofilm-mediated oral disease is halitosis. This disease causes a noticeably unpleasant breath odour. Halitosis is intra-oral in 90% of the cases and is mostly caused by changes in tongue microbiota composition and activity, leading to a microbial dysbiosis. This dysbiotic state causes increased proteolytic activity and/or bacterial degradation of sulphur-containing amino acids that results in the production of volatile sulphur compounds such as hydrogen sulfide (H2S), methyl mercaptan and, to a lesser extent, dimethylsulfide. Due to the proteolytic activity the pH stays neutral or slightly increases. Additionally, less of the VSCs neutralized by metabolism of a dysbiotic tongue community, which contributes to VSC release and bad breath.

In a particular embodiment, the biofilm-mediated oral disease is caries. Due to the saccharolytic activity (i.e., the microbial metabolism of carbohydrates, mostly sugars), lactate is produced and the pH decreases. When the pH decreases below a critical level of around pH 5.5, the enamel is demineralized and caries can develop.

In an embodiment, the mammal is a human. In another embodiment, the mammal is an animal such as a cat, a dog, a horse, a cow, a pig, a goat, a sheep, a donkey, a buffalo, an ox, a llama or a camel. In another embodiment, the biofilm-mediated oral disease is a periodontal disease or halitosis.

The invention also relates to compositions comprising at least one bacterial strain defined above. Particularly, said compositions are for use in treating or preventing a biofilm-mediated oral disease such as periodontal diseases, halitosis and caries. The identified *Rothia* bacterial strains improve resilience against different oral biofilm-mediated diseases since they present NRC in very different conditions associated to different diseases (e.g., caries has an acidic pH, while periodontitis has a neutral to slightly alkaline pH). For example, the identified isolates reduce nitrate at any pH. Thus, each strain is useful to treat or prevent caries, a periodontal disease and halitosis at the same time. Therefore, a product comprising one of the identified strains is useful to treat or prevent any biofilm-mediated disease. Additionally, nitrate-reduction by the oral microbiota increases the amount of systemic nitric oxide levels and, thus, each strain is useful to prevent systemic conditions that benefit from nitric oxide.

However, the inventors have seen that some bacterial strains perform much better (i.e. have "super-NRC") in particular conditions, and thus are particularly good for a specific disease (see Table 3 of EXAMPLE 3). Thus, in particular embodiments:
- Bacterial strains D1P7/CECT9999, D4T4/CECT30002, D4T6/CECT30003, and D4T9/CECT3004, have a good NRC (i.e., above the median) at pH 6, therefore being particularly suitable for the treatment of caries.
- Bacterial strains D1P7/CECT9999, D3T4/CECT30001, and D4T9/CECT3004 have a good NRC (i.e., above the median) at pH 7.5 and also reduce nitrite well at this pH, therefore being particularly suitable for the treatment of periodontal diseases and halitosis.
- Bacterial strains D1P17/CECT30000, D3T4/CECT30001, D4T4/CECT30002, D4T6/CECT30003 and D5T11A/CECT30005 produce most nitrite (i.e., above the median) at all or 2 out of 3 tested pH levels (i.e., pH 6, pH 7 and/or pH 7.5), therefore being particularly suitable for the treatment of systemic conditions or states that benefit from nitric oxide as nitrite can be swallowed to increase systemic nitric oxide levels.

The identified *Rothia* bacterial strains are useful in improving the NRC of an individual, and thus, are useful in the treatment or the prevention of a disease or state that benefits from nitric oxide supply other than a biofilm-mediated oral disease. In particular embodiments, the identified strains are useful for treating or preventing a cardiovascular disease, metabolic syndrome, diabetes, erectile dysfunction, urinary tract infection, improving sport performance, improving/increasing the antimicrobial activity of the stomach, improving/increasing the antimicrobial activity of the macrophages. Particularly, the cardiovascular disease is hypertension, so that the identified strains lower the blood pressure. The identified strains also improve endothelial function.

### Topically applied compositions (direct effect) and ingested compositions (indirect effect)

As understood by the skilled person in the present context, if the composition is a topically applied composition (i.e. its use is in the oral cavity, thus performing a "direct effect", such as the case of a toothpaste) comprising herein relevant amount of nitrate then the use of the composition automatically/inherently fulfills the requirements of the first aspect; i.e. the toothpaste is a composition that dissolves and/or disintegrates in the mouth and thereby increases the concentration of nitrate in the saliva of the mouth due to the presence of the nitrate in the composition as such. The same goes for instance if the composition is e.g. mouthwash, juice or a suitable gel (e.g. nitrate gel to periodontal pockets).

On the other hand, the composition can be an ingested composition e.g. an enteric-coated tablet that is not almost dissolved/disintegrated before it has passed the gastric environment. Also in this case, it is understood that such an enteric-coated tablet composition would be a composition within the scope of the first aspect, since it is a composition that does thereby increase the concentration of nitrate in the saliva of the mouth due to the nitrate-recycling activity of the salivary glands. This can be named "indirect effect".

Therefore, in a particular embodiment, the composition is a topically applied composition selected from the group consisting of:
- a toothpaste;
- a mouthwash;
- an oral gel;
- a dental floss;
- a tongue paste;
- a food extract;
- a chewing gum;
- a chewing tablet; and
- a supplement powder; or
the composition is an ingested composition selected from the group consisting of:
- tablets, pills or capsules;
- a food extract;
- a chewing gum;
- a chewing tablet;
- a pet and livestock food or snack;
- starch- and sugar-containing products;
- a supplement powder; and
- parenteral nutrition for intravenous application.

It can be seen that there are some products form with both topically and ingested administration; e.g. a food extract, a chewing gum, a chewing tablet or a supplement powder. In this case, the composition (e.g. a juice) is first in contact with the mouth and part of the composition dissolves and/or disintegrates in the mouth and thereby increases the concentration of nitrate in the saliva of the mouth due to the presence of the nitrate in the composition as such. And the composition (e.g. a juice) is then swallowed, introduced into the digestive tract and the increase in the concentration of nitrate in the saliva of the mouth is due to the nitrate-recycling activity of the salivary glands.

In a particular embodiment, the composition is a food supplement comprising a nitrate-rich vegetable extract, an antioxidant and/or a nitrate-reductase enzyme cofactor.

A nitrate-rich vegetable extract includes an extract from a plant or from fruits. Examples of vegetables rich in nitrate are green, leafy vegetables such as spinach, Swiss chard, mustard greens, arugula, kale and lettuce. Other vegetables rich in nitrate are beetroot, radishes, broccoli, celery, or cabbage. Examples of fruits rich in nitrate are banana or grapes. In an embodiment, the composition is a food supplement comprising a nitrate-rich vegetable extract.

In a particular embodiment, the nitrate-rich vegetable extract is a beetroot extract. In other embodiments, nitrate is in form of a salt such as sodium nitrate or potassium nitrate.

In a particular embodiment, the composition comprising the nitrate-rich vegetable extract further comprises a nitrate-reductase enzyme cofactor. Examples of cofactors are molybdenum or copper.

More particularly, the nitrate-rich vegetable extract is a beetroot extract, the antioxidant is vitamin C and the nitrate-reductase enzyme cofactor is molybdenum, a salt thereof or a molybdenum-rich vegetable extract, e.g. a kidney bean powder. Particularly, said composition is in liquid form (e.g. a beetroot juice adding the mentioned elements) or also in the form of e.g. a pill, tablet, chewing gum, powder, orodispersible or effervescent tablet to be dissolved with water.

An example of product based on nitrate-rich vegetable extract is described in EXAMPLE 4.1. Also as an example of administration form, a subject can brush their teeth in the morning like usual. Then, from a jar with 92 g of beetroot extract/vitamin C/molybdenum supplement (Table 5), a dose is taken of 11.5 g by using a plastic spoon provided with the jar and filling it until the 25 ml line. The dose is dissolved in 200 ml water, mixed and ingested. A dose of supplement contains 250 mg nitrate naturally present in the beetroot extract and the current daily-recommended doses of vitamin C (80 mg) and molybdenum (50 µg) for adults. This product is supplied in a single dose per day, preferably in the morning, in the form of a food supplement as a vegetable extract powder, and provides an immediate (within an hour, due to the retention of nitrate in the oral cavity during swallowing) and an acute but indirect effect (between 1 and 6 hours) due to nitrate recycling, during which a drop pH after a meal is diminished and therefore protection against oral diseases (such as dental caries) is provided.

### Acute treatment or prevention with effects before 24 hours

As mentioned, the provided results show a direct (immediate) topic effect of the nitrate when applied (as a supplement, toothpaste, etc), and a short-term effect (a few hours) when the composition is swallowed and recycled by the body to concentrate in the saliva. The advantage of having elevated nitrate concentrations for several hours in the saliva is that the microbiota changes towards a more health-associated composition and activity in the presence of nitrate as observed *ex vivo* in EXAMPLE 1 after 5h. In both cases, the effects are acute within 24 h post-treatment with the composition of the invention.

Without being limited to theory, it was a surprise for the present inventors that there was an improvement (alkalization) of the salivary pH both before and after a sugar rinse directly (1 h) to 4 h after taking a nitrate composition. The sugar rinse simulates a meal, where it is known that the pH decreases - accordingly, EXAMPLE 2 surprisingly shows that by administration of nitrate may provide direct and acute protection from a pH drop e.g. after a meal. Further, the results provide *in vivo* evidence that the levels of nitrate in saliva remain at high concentrations (i.e., above fasting nitrate levels of a donor) for a period of at least 6 hours. This demonstrates that e.g. a food supplement, a toothpaste or a tablet containing nitrate has a positive effect to reduce oral dysbiosis after a single dose and within 24 h, i.e. an acute effect, as opposed to the current state of the art where changes in oral microbiota composition *in vivo* are shown only after 1-4 weeks treatment, i.e. a chronic effect.

The provided results (EXAMPLE 2) show a positive fast effect, as opposed to all clinical trials with a nitrate supplement of the discussed prior art, where participants did the treatment for at least one week, and up to 1.5 months, taking daily higher doses (e.g., 385-770 mg per day). The inventors have seen that with a single dose below the ADI (e.g., <222 mg for an adult of 60 kg and <252 mg for an adult of 70 kg), the patient improves its pH within a few hours (*in vivo* and ex *vivo*)*.* This would allow a treatment for an immediate effect (e.g. an "anti-caries pill") to be taken e.g. on the morning and providing protection against caries during all the day.

In a particular embodiment, the effects of the treatment are seen 24 h after consumption of the composition of the invention with a direct and indirect acute effect; more particularly, effects are seen within 1-12 h after the consumption of a composition; more particularly effects are seen within 1-9h; more particularly effects are seen within 1h, 2h, 3h, 4h, 5h, 6h, 7h, 8h, 9h, 10h, 11h, 12h, 13h, 14h, 15h, 16h, 17h, 18h, 19h, 20h, 21h, 22h or, 23h.

In the *ex vivo* experiments provided EXAMPLE 1, it was observed that a concentration of 6.5 mM of nitrate in a small volume of 250 µl, was enough to drastically change oral biofilm composition and activity towards health-associated eubiosis after 5h and 9h. It was also showed that adding much lower concentrations of nitrate in the micromolar range prevented a pH drop caused by sugar. Based on these results, it may be established that the minimum dose of nitrate for a composition with a direct effect (i.e., topical application in the mouth) should result in 0.1 mM nitrate in the saliva (in addition to the nitrate that may already be present naturally). Taking into account that we have a minimum of ~0.5 ml of saliva in the mouth and the molecular weight of nitrate is 62 g/mol, the dose to achieve an increase in nitrate concentration of 0.1 mM would be 3.1 µg of nitrate (3.1 µg in 0.5 ml [x2000] = 6.2 mg in 1L [÷62] = 0.1 mM nitrate). Around 75% of the nitrate is removed from the body by the kidneys into the urine, while the other 25% is recycled by the salivary glands into saliva. Therefore, a dose of nitrate for an indirect acute effect (i.e., resulting from ingestion and recycling of the salivary glands) should be four times as high to reach an additional 0.1 mM of nitrate in the saliva, which is 12.4 µg of nitrate. Both doses (for direct and indirect applications) can be a pure nitrate salt or any form of vegetable nitrate in the presence or absence of other molecules.

Accordingly, in an embodiment, the salivary concentration of nitrate in the mouth after administration of the composition on top of fasting salivary levels is: at least 0.1 mM more 30 s after administration when the composition is a topically applied composition and, at least 0.1 mM more at least 2 h after administration when the composition is an ingested composition. This expresses the increase of salivary concentration of nitrate in the mouth after administration of the composition on top of fasting salivary levels; i.e. as relative value before and after administration.

Alternatively, the amount of salivary concentration of nitrate in the mouth after administration of the composition can be expressed in absolute values. Thus, taking into account that fasting salivary nitrate levels start at 0.1 mM, in an embodiment, the salivary concentration of nitrate in the mouth after administration of the composition is: at least 0.2 mM 30 s after administration when the composition is a topically applied composition and, at least 0.2 mM 2 h after administration when the composition is an ingested composition. In each of the cases, if the fasting salivary nitrate level is higher than 0.1 mM, the absolute value detected after the composition would be 0.1 mM above the detected fasting levels (fasting salivary nitrate expressed in mM + 0.1 mM). If, e.g., 0.5 mM nitrate is detected during fasting, the absolute value would be 0.6 mM 30 s after administration when the composition is a topically applied composition and, at least 0.6 mM 2 h after administration when the composition is an ingested composition. In a particular embodiment, the salivary concentration of nitrate in the mouth after administration of the composition is at least 3.5 mM 30 s after administration when the composition is a topically applied composition and, at least 3.5 mM 2 h after administration when the composition is an ingested composition.

To achieve said concentrations of nitrate in the saliva, a certain amount of nitrate has to be administered, that depend on the type of product, the administration schedule and the time of the treatment.

In this sense, the amount of nitrate per dose of composition is at least 3 µg when the composition is a topically applied composition, and at least 12 µg when the composition is an ingested composition. These are the minimum amounts of nitrate to observe the desired effects. In particular embodiments, the amount of nitrate per dose of composition is between 3 µg and 222 mg when the composition is a topically applied composition, and between 12 µg and 222 mg (and more particularly 35 mg) when the composition is an ingested composition. In more particular embodiments, optimal results are achieved when the amount of nitrate per dose of composition is 190, 195, 200, 205, 210, 215, or 220 mg.

### Compositions comprising probiotic bacteria

In an embodiment, the composition comprising nitrate of the first aspect, is administered in combination with at least a bacterial strain belonging to *Rothia* genus, wherein the *Rothia* bacterial strain has the features mentioned before (a)-(e). Particularly, the composition comprising nitrate is administered in combination with at least a bacterial strain selected from the group consisting of CECT 9999, CECT 30000, CECT 30001, CECT 30002, CECT 30003, CECT 30004, and CECT 30005, or combinations thereof.

The bacterial strains and nitrate of the present invention can be formulated for a separate, sequential, concomitant administration or in admixture. The administration regimens and forms will be determined by the skilled in the art e.g. according to the disorder to be treated. Administration of nitrate and bacteria can be administered separately (waiting a time interval between the administration of the bacteria and nitrate), sequentially (one after the other), concomitantly (simultaneously) or in admixture (together). In one embodiment, nitrate and the strains of the invention are administered within a time interval no longer than 12 hours. Particularly, the time interval is no longer than 6 hours. More particularly, the time interval is no longer than 1 hour. More particularly, the time interval is no longer than 5 minutes. In a more particular, embodiment the therapeutic regimen is based on the simultaneous administration of nitrate and the strains. One particular administration regimen consists in administering nitrate and subsequently administering the strain/s of the present invention. In some embodiments, nitrate and the strain/s are administered at the same time. When nitrate and the strain/s are administered to the patient at the same time, they can be administered as separate forms or as a part of a single composition. When the products are administered in separate dosage forms, the dosage forms can be in the same or different containers.

Described hereinafter are particular products comprising nitrate and/or the bacterial strains of the invention. The type of product will also depend e.g. on the disease to be treated.

### Oral care products

In particular embodiments, the compositions of the invention are in the form of oral care product, comprising nitrate and/or the strain/s, together with pharmaceutically excipients, or cosmetically acceptable excipients, or other edible ingredients. It is understood that the compositions of the present invention will be in an effective amount. The term "oral care product" refers to products used for keeping the mouth and teeth clean to prevent dental problems, most commonly, dental cavities, gingivitis, periodontal (gum) diseases and bad breath. In this sense, the oral hygiene product is not intentionally swallowed for systemic administration of particular therapeutic agents, but instead is retained in the oral cavity for a time sufficient to contact substantially all of the dental surfaces and/or oral tissues for purposes of oral activity. Non-limiting examples of such products are toothpastes, dentifrices, tooth powders, topical oral gels, mouth rinses, denture products, mouth sprays, chewing gums, dental floss, dental tapes, blasting powder, polishing pastes, dental varnishes, fissure sealants, filling materials, oral cream or gel, candy, lozenges, oral dispersible tablet or strip, or powder that may be sprinkled directly into the oral cavity.

In a particular embodiment, the oral care product is selected from the group consisting of a toothpaste; a mouthwash; an oral gel, e.g. for applying in the periodontal pockets; a chewing gum; and a chewing tablet.

The oral care products may additionally comprise flavoring and taste-masking agents. Non-limiting examples of these agents for use in oral care products include cinnamic aldehyde, eugenol, euca-lyptol, menthol, N-ethyl-p-menthane-3-carboxamide, anethole, peppermint oil, spearmint oil and corn mint oil. The oral care products may optionally include humectants, gelling agents, abrasives, fluoride sources, desensitizing agents, flavorings, colorings, sweeteners, preservatives, structuring agents, surfactants, anti-calculus agents and anti-plaque agents. The oral care products may also comprise other orally active agents, such as teeth whitening actives, including bleaching or oxidizing agents like peroxides, perborates, percarbonates, peroxyacids, persulfates, metal chlorites, and combinations thereof. Teeth color modifying substances may also be considered among the oral care actives useful in the present invention.

The formulation of toothpastes is well-known by those skilled in the art. In the toothpaste compositions, it is preferable to use nonionic (e.g. fatty acids esters with sugars) or amphoteric (e.g. coco-derived betaines) surfactants, since anionic surfactants have a negative effect on the delicate epithelial tissue of the gums. In the case of toothpastes, the use of sodium bicarbonate to neutralize oral acidity is also particularly preferred. In addition, toothpastes can contain thickening agents such as xanthan gum, abrasive silica fillers, and other supplementary agents in addition to those normally used in the toothpaste industry. Preferably, the bacteria is encapsulated or protected in other form to be introduced in a toothpaste.

As known by those skilled in the art "mouthwash", "mouth rinse", "dental rinse", "oral rinse" or "mouth bath" as used herein refers to a liquid composition which is held in the mouth passively or swilled around the mouth by contraction of the perioral muscles and/or movement of the head, and may be gargled, where the head is tilted back and the liquid bubbled at the back of the mouth. Usually mouthwashes are an antiseptic solution intended to reduce the microbial load in the oral cavity, although other mouthwashes might be given for other reasons such as for their analgesic, anti-inflammatory or anti-fungal action. Additionally, some rinses act as saliva substitutes to neutralize acid and keep the mouth moist in xerostomia (dry mouth). In addition to water, polyhydroxylated compounds such as glycerine or glycols (e.g., propylene glycol, nonionic surfactants, etc.) and other additives to improve appearance, flavor, and preservation can be included.

A typical formulation of chewing gum comprises gum base, sweeteners, softeners/plasticizers, flavors, colors, and, typically, a hard or powdered polyol coating. The gum base is considered proprietary information within each gum-manufacturing company but the three main components making up all gum bases are resin (e.g. terpene, which is the main component), wax (which softens the gum) and elastomer (which adds flexibility).

The sprays are compositions equal or similar to mouthwashes but dispensed in spray bottles for convenient application of the dose needed to moisten and protect the mouth without requiring subsequent rinsing.

Oral gels include polymers that allows direct, stable application to the oral cavity. In relation to these polymers, for the purposes of this invention it is preferable to use a combination of polymers generically known as polycarbophil and carbomer, since they keep the gel structure stable for very prolonged times under extreme temperature conditions. The gels can also include a quantity of a natural, noncariogenic sweetener, such as sorbitol.

### Pharmaceutical products and food supplements

In particular embodiments, the compositions of the invention are formulated as pharmaceutical products, particularly, as food supplements. The term "pharmaceutical product" is understood in its widely meaning in this description, including any composition that comprises an active ingredient, in this case, the strains of the invention preferably in form of composition and optionally at least one antiseptic, together with pharmaceutically acceptable excipients. This term is not limited to medicaments.

The term "pharmaceutically acceptable" is art-recognized, and includes to compounds, materials, compositions, carriers, vehicles and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts. Some non-limiting examples of materials which may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; cocoa butter and suppository waxes; oils, such as peanut oil, cottonseed oil, sunflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other nontoxic compatible substances employed in pharmaceutical formulations.

Excipients are selected, without limitation, from the group comprising: fillers/diluents/bulking agents, binders, antiadherents, disintegrants, coatings, anti-caking agents, antioxidants, lubricants, sweeteners, flavors, colors, tensides and other classes of pharmaceutically and veterinary acceptable excipients.

Fillers are selected, without limitation, from the group comprising: inulin, oligofructose, pectin, modified pectins, microcrystalline cellulose, lactose, starch, maltodextrin, saccharose, glucose, fructose, mannitol, xylitol, non-crystallizing sorbitol, calcium carbonate, dicalcium phosphate, other inert inorganic and organic pharmacologically acceptable fillers, and mixtures of these substances. At dosage form of oral suspension, fillers or diluents are selected from the group comprising: vegetable oil, oleic acid, oleyl alcohol, liquid polyethylene glycol, other pharmacologically acceptable inert liquids, or mixtures of these substances.

Binders are used in solid dosage forms, e.g. to hold the ingredients in a tablet together, to ensure that tablets and granules can be formed with required mechanical strength, and to give volume to low active dose tablets. Binders in solid dosage forms like tablets are: lactose, sucrose, corn (maize) starch, modified starches, microcrystalline cellulose, modified cellulose (e.g. hydroxypropyl methylcellulose (HPMC) and hydroxyethylcellulose), other water soluble cellulose ethers, polyvinylpyrrolidone (PVP) also known as povidone, polyethylene glycol, sorbitol, maltitol, xylitol and dibasic calcium phosphate; other suitable pharmacologically acceptable binders, or mixtures of these substances.

Antiadherents are used to reduce the adhesion between the powder (granules) and the punch faces and thus prevent sticking to tablet punches. They are also used to help protect tablets from sticking. The most commonly used is magnesium stearate.

As disintegrants and superdisintegrants in solid dosage forms like tablets and capsules, the following substances, without limitation, are used: cross-linked polyvinylpyrrolidone, sodium starch glycolate, sodium carboxymethyl cellulose, calcium carboxymethyl cellulose, and formaldehyde-casein, other suitable pharmacologically acceptable disintegrant and superdisintegrant, or their mixtures.

Coatings in the case of solid dosage forms, such as tablets and granules for capsules filling, protect the ingredients from deterioration by moisture in the air, make large, unpleasant-tasting tablets easier to swallow and/or in the case of enteric coatings ensure intact passage through a strong acidic medium of gastric juice (pH around 1), and which allow release in duodenum or ileum (small intestine). For most coated tablets, a cellulose ether hydroxypropyl methylcellulose (HPMC) film coating is used. Occasionally, other coating materials are used, e.g. synthetic polymers and co-polymers like polyvinylacetate phthalate (PVAP); co-polymers of methyl acrylate-metacrylic acid; co-polymers of methyl metacrylate-metacrylic acid; shellac, corn protein zein or other polysaccharides; waxes or wax-like substances such as beeswax, stearic acid; higher fatty alcohols like cetyl or stearyl alcohol; solid paraffin; glycerol monostearate; glycerol distearate, or their combinations. Capsules are coated with gelatin or hydroxypropyl methylcellulose.

Enteric coatings control the rate of drug release and determine where the drug will be released in the digestive tract. Materials used for enteric coatings include fatty acids, waxes, shellac, plastics, and plant fibers and their mixtures, also in combination with other above mentioned coatings.

An anticaking agent is an additive placed in powdered or granulated materials to prevent the formation of lumps (caking) and for easing packaging, transport, and consumption. As anti-caking agents in solid dosage forms like tablets, capsules, or powders, the following are used: magnesium stearate, colloidal silicon dioxide, talc, other pharmacologically acceptable anticaking agents, or their mixtures.

Lubricants are used in solid dosage forms, in particular in tablets and capsules, to prevent ingredients from clumping together and from sticking to the tablet punches or capsule filling machine, and also in hard capsules. As lubricants talc or silica, and fats, e.g. vegetable stearin, magnesium stearate or stearic acid, and mixtures thereof, are the most frequently used lubricants in tablets or hard gelatin capsules.

Sweeteners are added to make the ingredients more palatable, especially in solid dosage forms, e.g. chewable tablets, as well as in liquids dosage forms, like cough syrup. Sweeteners may be selected from artificial, natural or synthetic or semi-synthetic sweeteners; non-limiting examples of sweeteners are aspartame, acesulfame potassium, cyclamate, sucralose, saccharine, sugars or any mixture thereof,

Flavors can be used to mask unpleasant tasting active ingredients in any dosage form. Flavorings may be natural (e.g. fruit extract) or artificial. For example, to improve: (1) a bitter product, mint, cherry or anise may be used; (2) a salty product, peach or apricot or liquorice may be used; (3) a sour product, raspberry; and (4) an excessively sweet product, vanilla.

Except auxiliary substances from the class of excipients, the formulation from the present invention can contain other pharmacologically active or nutritive substances including, but not limited, to vitamins, such as vitamin D (calciferol) in the pharmaceutically acceptable chemical form, salt or derivatives; minerals in the form of pharmaceutically and nutritive acceptable chemical form; and L-amino acids. Regarding the preparation of the formulations of the present invention is within the scope of ordinary person skilled in the art and will depend upon the final dosage formulation. For instance, and without limitation, when the final dosage forms is an oral solid one, such as tablets, capsules, powder, granules, oral suspension, etc. the process for preparation of solid dosage forms of the formulation includes homogenization of: (1) the active ingredient(s), comprising at least one or more post-treated probiotic bacteria of the invention in an effective amount; (2) with one or more excipients to form homogeneous mixture which is, e.g. according to requirements, subjected to lubrication with magnesium stearate or other lubricants yielding final dosage form of powder. Such homogeneous powder is filled into ordinary gelatin capsules or, alternatively, into gastro-resistant capsules. In the case of tablets, they are manufactured by direct compression or granulation. In the first case, a homogeneous mixture of active ingredients and suitable excipients such as anhydrous lactose, non-crystallizing sorbitol, and others is prepared. In the second case, tablets are processed on the mixture in granulated form. Granules are prepared by granulation process of active ingredients of the formulation with suitable fillers, binders, disintegrants, and small amount of purified water. Such prepared granules are sieved and dried until the water content of <1 % w/w.

Regarding the process for preparation of liquid dosage forms (e.g. oral suspension), it involves homogenization of the active ingredient(s) of the formulation comprising at least one or more post-treated probiotic bacteria of the invention in an effective amount in an inert liquid diluent (filler) such as various vegetable oils like sunflower, soybean or olive oil; oleic acid; oleyl alcohol; liquid polyethylene glycols like PEG 200, PEG 400 or PEG 600; or other inert pharmacologically acceptable liquids. The process further involves treatment of homogeneous mixture with one or more processes selected from the group comprising: (1) stabilization of the formulation, by addition and homogenization of suspension stabilizers like beeswax, colloidal silicon dioxide, etc.; (2) sweetening of the formulation; by addition and homogenization of sweetener; (3) flavoring of the formulation, by addition and homogenization of flavoring. Such forms of the formulation can contain also other excipients or ingredients, usually employed in the art.

The pharmaceutical product can adopt different forms or names depending on the product approval route and also depending on the country. For instance, a medicament is a particular pharmaceutical product. A medical food is another particular pharmaceutical product. The terms "medical food" or "food for special medical purposes" are used in some countries to refer to a food specially formulated and intended for the dietary management of a disease that has distinctive nutritional needs that cannot be met by normal diet alone. They are defined in regulations such as the Food and Drug Administration's 1988 Orphan Drug Act Amendments in the United States, and the Commission Directive 1999/21/EC in Europe. Medical foods are distinct from the broader category of food supplements and from traditional foods that bear a health claim. Thus, in a particular embodiment, the strains of the invention are formulated as a medical food.

A food supplement, also known as dietary supplement or nutritional supplement is considered another particular pharmaceutical product. This is a preparation or product intended to supplement the diet, made from compounds usually used in foodstuffs, which provide nutrients or beneficial ingredients that are not usually ingested in the normal diet or may not be consumed in sufficient quantities. Mostly, food supplements are considered as food products, but sometimes they are defined as drugs, natural health products, or nutraceutical products. In the sense of the present invention, food supplements also include nutraceuticals. Food supplements are usually sold "over the counter", i.e. without prescription. If the food supplement adopts the form of a pill, a capsule a tablet or a powder, it comprises excipients which are the same as the used in medicaments. A food supplement however, can also adopt the form of a food product which is fortified with some nutrients (e.g. a bar or yoghurt).

Thus, in a particular embodiment, the compositions of the invention are formulated as a food supplement. The food supplement can be administered as such, can be mixed with a suitable drinkable liquid, such as water, yoghurt, milk or fruit juice, or can be mixed with solid or liquid food. In this context the food supplement can be in the form of tablets or lozenges, pills, capsules, granules, powders, suspensions, sachets, sweets, bars, syrups and corresponding administration forms, usually in the form of a unit dose. More particularly, the compositions of the invention are formulated as tablets, pills, capsules, a food extract (particularly based on beetroot extract as explained above), and a supplement powder.

The compositions of the invention can be also included in a variety of food products, such as a milk products (a yogurt, a cheese, a fermented milk, a milk powder, a milk based fermented product, an ice-cream, a fermented cereal based product, a milk based powder), bread, bars, spreads, biscuits and cereals, a beverage, different types of oil, or a dressing. The term "food product" is used herein in its broadest meaning, including any type of product, in any form of presentation, which can be ingested by an animal, but excluding pharmaceutical and veterinary products. Examples of other food products are meat products, chocolate spreads, fillings and frostings, chocolate, confectionery, baked goods, sauces and soups, fruit juices and coffee whiteners. Particularly interesting food products are food supplements and infant formulas. The food product preferably comprises a carrier material such as oat meal gruel, lactic acid fermented foods, resistant starch, dietary fibres, carbohydrates, proteins and glycosylated proteins. In a particular embodiment the strains of the invention are encapsulated or coated.

### Examples of particular products

In particular embodiments, the composition also comprises molybdenum and/or vitamin C and/or (other) antioxidants and/or any carbon source for nitrate-reducing bacteria.

Examples of such products, ingredients contained and administration form are described in EXAMPLE 4:
In a particular embodiment, the composition of the invention is in the form of a chewing tablet, which provides a direct and an indirect effect. In particular, the chewing tablet is e.g. the one described in EXAMPLE 4.2. Chewing tablets (e.g. 0.5-3 g) containing nitrate (e.g., 222 mg if 1 dose, 111 mg if 2 doses, 74 mg if 3 doses) may be consumed daily by chewing and swallowing after breakfast and oral hygiene in the morning and, if divided in two doses, also after lunch and, if divided in three doses, also after dinner and oral hygiene at the end of the day. Some variants of the tablets of 1, 2 or 3 doses also contain e.g. 80 mg, 40 mg or 26.67 mg vitamin C, respectively, and/or e.g. 50 µg, 25 µg or 16.67 µg molybdenum. Finally, some variants of the tablets contain daily acceptable amounts of commercially available anti-oxidants, which were divided over 1, 2 or 3 doses. This product is ideal for an acute direct and indirect effect on oral diseases, e.g. during a period of 0-6 h after consumption.

In particular, the chewing tablet is an anti-caries chewing tablet as e.g. the one described in EXAMPLE 4.3. Tablets (e.g. 0.5-3 g) containing e.g. an amount between 3.1 µg-74 mg nitrate are consumed by swallowing before a meal. A maximum of three tablets could be consumed per day and it is recommended to consume them 1h before the three meals or snacks with most sugar, preferably each in a different part of the day (morning, afternoon and evening). This product is useful for an acute indirect effect on oral diseases, e.g. during a period of 0-6 h after consumption, as well as to improve all health conditions that are influenced by a deficit of nitric oxide.

In a particular embodiment, the composition of the invention is in the form of a chewing gum which provides a direct and an indirect effect. In particular, the chewing gum is e.g. the one described in EXAMPLE 4.3. Chewing gums (e.g. 1-2 g) containing e.g. an amount between 3.1 µg-37 mg nitrate were consumed by swallowing before a meal. A maximum of six chewing gums could be consumed per day and it was recommended to consume them right after meals or snacks, preferably at least one in a different part of the day (morning, afternoon and evening). Other molecules were added based on EXAMPLE 4.2. This is ideal for an acute indirect effect on oral diseases, e.g. during a period of 1-6 h after ingestion, as well as to improve all health conditions that are influenced by a deficit of nitric oxide.

In a particular embodiment, the composition of the invention is in the form of toothpaste, which provides a direct effect. In particular, the toothpaste is e.g. the one described in EXAMPLE 4.5. A toothpaste dose of e.g. 0.2-1 g containing e.g. an amount between 3.1 µg-74 mg of nitrate, e.g. 26.67 mg vitamin C and e.g. 16.67 µg molybdenum and other molecules based on EXAMPLE 4.2 is used by individuals like normally without exceeding three times of toothbrushing per day. This is administered when brushing, as with a standard toothpaste, by contact with the teeth and gum, which provides a topic application of nitrate directly to oral biofilms, being part of the nitrate also retained in the oral cavity until saliva clearance eliminates it. It is recommended that the mouth is not washed or only briefly rinsed once with water after application.

In a particular embodiment, the composition of the invention is in the form of a mouthwash, which provides a direct effect. In particular, the mouthwash is e.g. the one described in EXAMPLE 4.6. Mouthwash volume to be taken is e.g. 5-30 ml, particularly 15 ml. An oral rinse for e.g. 1-60s is made by which a topic application of the nitrate product is given to tongue, teeth, oral mucosa and gums, and nitrate is therefore directly provided to oral biofilms, being part of the nitrate also retained in the oral cavity until saliva clearance eliminates it. It is recommended that the mouth is not washed after application.

In a particular embodiment, the composition of the invention is in the form of oral gel for periodontal pockets, which provides a direct effect. In particular, the oral gel is e.g. the one described in EXAMPLE 4.7. A buccoadhesive gel is applied with a syringe by a professional inside the periodontal pockets of patients with a periodontal disease, containing a concentration of e.g. an amount between 3.1 µg-222 mg nitrate in the entire volume applied over one to several pockets, with or without a nitrate-reducing probiotic. It is applied inside the pockets at the basal, treatment and follow-up visits of the patient as an initial treatment. It is recommended not to eat or drink for an hour after application. A maintenance treatment can be combined, in which a daily nitrate supplement or tablet is provided for e.g. 1 to 4 weeks in the morning. When the composition comprises probiotic bacteria, the product is not recommended for immunosuppressed patients.

In a particular embodiment, the composition of the invention is in the form of daily dose capsule or pill, which provides an indirect effect. In particular, the capsule or pill is e.g. the one described in EXAMPLE 4.8. Capsules (e.g. 0.1-3 g) containing nitrate (e.g., 222 mg if 1 dose, 111 mg if 2 doses, 74 mg if 3 doses) were consumed daily by ingestion before breakfast and oral hygiene in the morning and, if divided in two doses, also before lunch and, if divided in three doses, also before dinner and oral hygiene at the end of the day. Some variants of the capsules of 1, 2 or 3 doses also contained e.g. 80 mg, 40 mg or 26.67 mg vitamin C, respectively, and/or 50 µg, 25 µg or 16.67 µg molybdenum. Finally, some variants of the capsules contained daily acceptable amounts of commercially available anti-oxidants, which were divided over 1, 2 or 3 doses. To choose the optimal combinations and amounts of molecules, the different capsules were tested. This is ideal for an acute direct and indirect effect on oral diseases, e.g. during a period of 0-6 h after ingestion.

In particular, the pill, capsule or chewing tablet is an anti-halitosis pill to take before a meeting. A pill like the one described above could be taken, e.g., in the morning before a meeting that will take place 2-4 hours later. For a rapid effect before a meeting, the nitrate may be in the form of a chewing tablet with a high dose (e.g., 222 mg) and taken just before the meeting.

In a particular embodiment, the composition of the invention comprises probiotic bacteria and is applied with a ferule. In particular, the composition is e.g. the one described in EXAMPLE 4.9. A nitrate-reducing probiotic is provided in a lyophilized form with vitamin C and molybdenum, as well as a thickening agent. This is mixed with water and applied in the teeth with a ferule for 5-30 minutes, to allow bacterial colonization of the dental biofilm. This is applied at night at least 30 minutes after standard oral hygiene, avoiding eating or drinking for at least an hour after application. This is especially suited to treat and prevent dental diseases (caries or gum diseases). In another particular mode of application, the probiotic preparation is applied to the tongue for e.g. 1-5 minutes, which is especially suited to treat halitosis. This product is not recommended for immunosuppressed patients.

In a particular embodiment, the composition of the invention is in the form of parenteral nutrition for intravenous application at Intensive Care Units (ICUs), which provides an indirect effect. In particular, the parenteral nutrition is e.g. the one described in EXAMPLE 4.10. Parenteral nutrition (a patient-dependent dose, e.g. 10-1600 ml) containing nitrate (e.g., an amount between 12.4 µg-222 mg) in the form of a salt or vegetable extract may be used to prevent oral disease development in ICU patients. Some variants of the parenteral nutrition also contain extra vitamin C, antioxidants and molybdenum. Some variants are more suitable for babies, some for toddlers, some for infants, some for teenagers, and some for adults.

In a particular embodiment, the composition of the invention is in the form of candy for children and adults, such as the ones described in EXAMPLE 4.11.

In a particular embodiment, the composition of the invention is in the form of starch- and sugar-containing product, such as the ones described in EXAMPLE 4.12.

In a particular embodiment, the composition of the invention is in the form of pet and livestock food and snacks, such as the ones described in EXAMPLE 4.13.

In a particular embodiment, the composition of the invention is in the form of tongue paste, such as the ones described in EXAMPLE 4.15.

In a particular embodiment, the composition of the invention is in the form dental floss, such as the ones described in EXAMPLE 4.16.

### Method of selecting a therapeutic treatment or a preventive strategy

The present invention also contemplates the selection of personalized therapies or preventive strategies in accordance with the nitrate-reduction capacity (NRC) of a subject to be treated. The herein provided EXAMPLE 5 shows that subjects can be stratified or segmented according to their NRC. Then, a personalized therapy or a preventive strategy can be established according to their NRC.

Thus, another aspect of the invention relates to a method for selecting a therapeutic treatment or a preventive strategy for a biofilm-mediated oral disease or a disease or state that benefits from nitric oxide supply. The method comprises a first step (i) wherein the nitrate-reduction capacity of a subject in an oral (preferably saliva) sample is measured. In a second step (ii), the subject is classified according to the degree of nitrate-reduction capacity of the subject.

Nitrate-reduction capacity of a subject can be measured by e.g. the method described in EXAMPLE 5. For example, a 1:10 dilution is made of a sterile 80 mM (4960 mg/l) nitrate in water solution in a fasting saliva sample, leading to an added concentration of 8 mM (496 mg/l). For example, 0.05 ml of 80 mM nitrate are added to a fasting saliva sample of 0.45 ml to reach a final volume of 0.5 ml, resulting in a final nitrate concentration of 8 mM. One sample is directly frozen at -20°C and another one is incubated at 37°C for 2 hours. Nitrate is measured in both samples as described in EXAMPLE 5 and the difference of the mg/l between the two samples, corresponds to the nitrate reduced.

With nitrate values, the subject is classified according to their degree of nitrate-reduction capacity, wherein:
ii.1) a decrease in the amount of nitrate relative to a non-incubated oral sample below 57 mg/l (or below 15% expressed as percentage of reduction) is indicative of a poor nitrate-reduction capacity,
ii.2) a decrease in the amount of nitrate relative to a non-incubated oral sample between and including 57 mg/l and 175 mg/l (between and including 15% and 35%) is indicative of an intermediate nitrate-reduction capacity, and
ii.3) a decrease in the amount of nitrate relative to a non-incubated oral sample above 175 mg/l (or above 35%) is indicative of a good nitrate-reduction capacity.

The method further comprises (iii) selecting a therapeutic treatment or a preventive strategy according to the nitrate-reduction capacity, wherein:
iii.1) a subject with a poor nitrate-reduction capacity is administered with a composition comprising nitrate and a bacterial strain belonging to Rothia or Neisseria genera,
iii.2) a subject with an intermediate nitrate-reduction capacity is administered with a composition comprising nitrate and/or a bacterial strain belonging to Rothia or Neisseria genera, and
iii.3) a subject with a good nitrate-reduction capacity is administered with a composition comprising nitrate.

A biofilm-mediated oral disease or a disease or state that benefits from nitric oxide supply has been defined above. The subject is particularly a mammal, and more particularly a human. In particular, a subject with a good nitrate-reduction capacity will most likely benefit from the administration of a composition comprising nitrate given that the subject already has bacteria to perform that function and therefore only needs the substrate of the reaction. Conversely, a subject with a normal nitrate-reduction capacity will most likely benefit from the administration of a composition comprising nitrate and/or a bacterial strain belonging to *Rothia* or *Neisseria* genera, given that both the levels of the substrate and the levels of bacteria can improve the NRC. And a subject with a poor nitrate-reduction capacity will most likely benefit from the administration of a composition comprising nitrate and a bacterial strain belonging to *Rothia* or *Neisseria* genera, given this subject needs both to be able to reduce nitrate.

In a particular embodiment the step (i) of the method, i.e. measuring the nitrate-reduction capacity of a mammal in an oral sample, comprises the step of:
1) collecting a salivary sample;
2) adding a known amount of nitrate;
3) incubating at 37°C for at least an hour; and
4) measuring the amount of nitrate left in the salivary sample;
wherein a higher amount of nitrate left is indicative of a poor capacity of nitrate-reduction.

An example of method for measuring the nitrate-reduction capacity of a mammal in an oral sample is described in EXAMPLE 5.

Particularly, the compositions comprising nitrate and/or a bacterial strain belonging to *Rothia* or *Neisseria* genera are the ones described in the above sections.

The method selecting a suitable therapeutic treatment or a preventive strategy can alternatively be formulated as a method for selecting a subject suffering from a biofilm-mediated oral disease or a disease or state that benefits from nitric oxide supply to receive a therapeutic treatment or a preventive strategy selected from the different combinations of a composition comprising nitrate and/or a bacterial strain belonging to *Rothia* or *Neisseria* genera, the method comprising (i) measuring the nitrate-reduction capacity of a mammal in an oral sample, and (ii) selecting said subject to receive said therapeutic treatment or preventive strategy based on the nitrate-reduction capacity explained above (i.e. poor, normal and good nitrate-reduction capacity). This can be also referred to a method of stratification or segmentation of subjects according to their NRC.

Thus, herein is provided a method for re-establishing NRC of a subject in the oral cavity.

For the avoidance of doubt the methods of the invention do not involve diagnosis practiced on the human or animal body; the methods are particularly conducted on a sample that has previously removed from the subject.

### EXAMPLES

### EXAMPLE 1: Ex vivo study - effects of nitrate on oral biofilm growth of healthy human individuals

### Materials and methods

### 1. Unstimulated saliva sampling

For this study, adults who reported to be systemically healthy were recruited at the Centre for Public Health Research (CSISP-FISABIO, Valencia, Spain). Individuals were excluded if they reported to have caries or any history of periodontitis.

Unstimulated saliva was collected from three groups of donors in the morning by drooling (Navazesh & Christensen, 1982) in a sterile tube in a quiet room until a volume of 5 ml was reached. The first and the second group both consisted of 12 donors (D1-D12 and D13-D24, respectively). These donors were instructed to have a normal breakfast and abstain from oral hygiene in the morning before saliva collection. First, the saliva of D1-D12 was collected at least one hour after breakfast and used as inoculum for *in vitro* biofilm growth. Then, this experiment was repeated with the saliva of D13-D24 to perform additional measurements. The third group consisted of 9 donors (D25-D33) who were asked to donate saliva while fasting (i.e., abstaining from breakfast and oral hygiene). The saliva of this group was used to determine the effect of nitrate on acidification due to glucose fermentation.

The fresh unstimulated saliva was always directly used in the experiments or kept at 4°C for at most 1 h before usage. All donors gave informed consent prior to collection of the clinical material and the protocol was approved by the Ethical Committee of DGSV-CSISP (Valencian Health Authority).

### 2. In vitro oral biofilm growth and impedance-based quantification

Biofilms of D1-D12 were grown from unstimulated saliva in 'E-Plate 96' 96-well plates (ACEA Biosciences) in the xCELLigence system (ACEA Biosciences). Each E-Plate 96 is coated with a golden layer at the bottom of the wells that is connected to microelectrodes, allowing the measurement of biofilm growth in real-time (Mira et al., 2019). The impedance formed by biofilm adherence has been shown to proportional to the quantity of single-species biofilms, and a measure of biofilm mass is provided by the corresponding Cell Index, expressed in arbitrary units (Ferrer et al., 2017).

BHI medium (Biolife) with an additional 0.05 mg/L haemin, 0.005 mg/L menadione and 0.2 mM vitamin K (all Sigma-Aldrich) was prepared of which 100 µl was added to each well for background impedance measurements. An additional 25 µl of a 65 mM nitrate in water or just water were added to each well of the nitrate or control condition, respectively. Then, 125 µl freshly collected saliva was added, leading to a final concentration of 6.5 mM nitrate in the nitrate condition. The E-Plate 96 was placed in the xCELLigence system inside an incubator at 37°C. Every 10 minutes, a Cell Index measurement was taken. All experiments were performed without agitation and anaerobic conditions were favored by sealing the wells with adhesive aluminum foil (VWR), which previously allowed the growth of strictly anaerobic bacteria (data not shown). For 0h measurements, 1:1 medium and saliva mixtures were used. Then, duplicates of supernatant and biofilm were taken after 5h and 9h of growth. The supernatant was sampled and stored at -20°C until pH, nitrate, nitrite, ammonium and lactate measurements. After this, a PBS washing step was performed and duplicates of biofilms were resuspended together in 200 µl PBS for storage at -20°C until DNA isolation for sequencing.

The biofilms of the second group of 12 donors (D13-D24) were grown identically to the first group, sampled without a PBS washing step, and stored individually in 75 µl PBS to quantify protein and DNA at 5h and 9h. It was observed that nitrate affects the impedance of the xCELLigence system and this effect depended on the saliva of the donor. Therefore, for D13-D24, controls with microorganism-free filtered saliva were used to normalize the cell-index measurements. For this, the saliva was first filtered with a 5 µm filter and then with 0.1 µm filter. All samples were immediately stored at -20°C in Eppendorf tubes until further analyses.

### 3. Incubating saliva with nitrate and glucose

The unstimulated saliva of D25-D33 was used to test the effect of different concentrations of nitrate on a pH drop caused by 0.2% of glucose after 5 h of incubation. For each donor, 187 µl of saliva and 22 µl of glucose (2% diluted in water) was added per well of a standard 96-well plate. Then, 11 µL of water without or with different concentrations of nitrate was added, leading to final concentrations of 0 mM, 0.5 mM, 1 mM, 1.5 mM, 2.5 mM, 3.5 mM, 4.5 mM, 5.5 mM, 6.5 mM, 7.5 mM and 8.5 mM of nitrate. The plate was sealed with adhesive aluminum foil (VWR) and incubated during 5 h at 37°C. After incubation, the samples were stored at -20°C until pH measurements.

### 4. Nitrate, nitrite, ammonium, lactate and pH measurements

For the nitrate, nitrite, ammonium, lactate and pH measurements, the RQflex^{®} 10 Reflectoquant^{®} (Merck Millipores), a reflectometer that measures the reflection of color changing test strips, was used. The strips for pH (Merck Millipores ref. 1.16996.0001) had a range from pH 4 to 9 and incubation time of 10 seconds on the strip, the strips for nitrate (ref. 1.16995.0001) had a range of 3-90 mg/l and incubation time of 1 min, the strips for nitrite (ref. 1.16973.0001) a range of 0.5-25 mg/l and incubation time of 15 sec, the strips for ammonium (ref. 1.16899.0001) a range of 5-20 mg/l and 4 min incubation time, and the strips for lactate (ref. 1.16127.0001) a range of 3-60 mg/l and incubation time of 6 min. Accuracy of all reflectometer methods was confirmed by the use of controls with known concentrations of the different measured compounds.

In the case of nitrate, nitrite, ammonium and lactate measurements, the supernatant of the cultures was diluted 1:10 with water to minimize the interference of medium compounds. In some cases, the supernatant had to be diluted more for the compounds to be in the detection range of the strips. For pH measurements, undiluted supernatant was used. For all measurements except ammonium, 15 µl of (diluted) supernatant was added to two reactive patches on a strip, excess liquid was removed by tipping the side of the strip on a tissue, the corresponding incubation time was applied for each measurement, and the strip was put inside the reflectometer.

Nitrite was measured first. The diluted supernatants in which 0.5 mg/l or more nitrite was detected were treated with amidosulfuric acid to remove nitrite before nitrate measurements. For this, 35 µl of diluted supernatant was mixed with 1.5 µl amidosulfuric acid solution (10%), and directly added to the strips.

For ammonium measurements, aliquots were made of 50 µl diluted supernatant to which 10 µl of reagent 1 of the kit was added first and resuspended well. Then, 15 µl of a freshly made mixture of one scoop of reagent 2 (both provided with the kit) dissolved in 1.25 ml water was added directly and resuspended well. This solution was then directly added to the strips and incubated. Five seconds before the measurements, excess liquid was removed. All measured concentrations were adjusted by dilution factors.

### 5. Biofilms protein quantification

Biofilms grown for 5h and 9h were resuspended in 75 µl PBS. For protein quantification, the Bradford protein assay was applied, which is based on the colour change of the Coomassie Brilliant Blue dye (G-250) when bound to proteins. Duplicates of 15 µl of resuspended pellet were added to different wells of a standard 96-well plate. Then, 240 µl of Bradford Reagent (Sigma-Aldrich), containing G-250, was added and, after 5 minutes of incubation in the dark, the absorbance was measured with an Infinite F200 plate reader (TECAN) at 600 nm. Protein concentrations were determined using a calibration curve with known concentrations of BSA (range: 0 to 1.5 mg/ml, Sigma-Aldrich) on each plate.

### 6. Biofilm composition determined by 16 rDNA Sequencing

### 6.1. DNA extraction for sequencing

For DNA extraction, the biofilms were resuspended in 100 µl PBS and disaggregated 30 s in a sonicator bath (model Raypa VCI-50) at low ultrasound intensity. After this, DNA was isolated by MagNA Pure LC 2.0 Instrument (Roche Diagnostics), using the MagNA Pure LC DNA Isolation Kit III for Bacteria and Fungi (Roche Diagnostics) following the manufacturer's instructions with an additional enzymatic lysis step: to a salivary pellet in 100 µl PBS, 130 µl lysis buffer and 2.5 µl of enzyme mix, containing 20 mg/ml lysozyme (Thermomixer comfort), 5 mg/l lysostaphin (Sigma-Aldrich) and 0.625 mg/ml mutanolysin (Sigma-Aldrich), were added and incubated for 60 min at 37°C. DNA was resuspended in 100 µl elution buffer and frozen at -20°C until further analysis. To determine the amount of DNA for sequencing, the Quant-iT^{™} PicoGreen^{®} dsDNA Assay Kit (ThermoFisher) and a Qubit^{™} 3 Fluorometer (ThermoScientific) were used, according to manufacturer's instructions.

### 6.2. 16 rDNA Sequencing

A pre-amplification step of the V1-V5 regions of the 16S rRNA gene was performed, following Dzidic et al., 2019. An Illumina amplicon library was performed following the 16S rDNA gene Metagenomic Sequencing Library Preparation Illumina protocol (Part #15044223 Rev. A). The gene-specific primer sequences used in this protocol were 16S Amplicon F (SEQ ID NO: 8) and 16S Amplicon R (SEQ ID NO: 9), targeting the 16S rDNA gene V3 and V4 regions, resulting in a single amplicon of 460 bp. Overhang adapter sequences were used together for compatibility with Illumina index and sequencing adapters. After 16S rDNA gene amplification, the DNA was sequenced on a MiSeq Sequencer according to manufacturer's instructions (Illumina) using the 2x300 bp paired-ends protocol.

### 6.3. Taxonomic classification

The sequences were analyzed according to Boix-Amorós et al., , 2016. In short, the reads were quality-filtered and end-trimmed in 10 bp windows with Prinseq. The PCR chimeras were removed with UCHIME according to Edgar et al., 2011. Only filtered sequences >250 bp were used to be taxonomically assigned at the genus level with the classifier of the Ribosomal Database Project (Wang et al., 2007), using a confidence interval of 80%. Operational Taxonomic Unit (OTU) picking was performed using VSEARCH (Rognes, 2016) at a 97% of sequence identity. Each OTU was aligned centroid using BLAST at 97% of identity and 100% query coverage, and retrieved only those species that agreed with the previous classification of the centroid at genus level provided by RDP classifier.

6.4. Statistical analysis. Overall R programming language was used for statistical computing to perform downstream analyses. Genera with an abundance of <0.01% were removed from all groups. For multivariant analysis, an Adonis test (Permutational Multivariate Analysis of Variance Using Distance Matrices), provided by the Vegan library of R (Oksanen, 2017), was used to compare groups. To visualize groups and their differences in a two-dimensional map, constrained principal components were computed via constrained correspondence analysis (CCA) which is also part of Vegan library. For univariate analyses, paired non-parametric Wilcoxon tests were carried out to test the differences in genera, OTUs and all other parameters between groups (5h control vs 5h nitrate, and 9h control vs 9h nitrate). Unadjusted p-values were used for taxonomic comparisons.

### Results

### 7. Effect of nitrate on biofilm growth

The addition of 6.5 mM nitrate (i.e., 403 mg/L) did not show significant changes in real-time impedance measurements of biofilm growth compared to the control biofilms (FIG. 1A). In agreement with this, protein levels did not differ significantly between the different conditions (FIG. 1B).

### 8. Changes in nitrate, nitrite, ammonium, lactate and pH during biofilm growth

Mixtures of saliva and BHI medium with or without 6.5 mM nitrate before growth (0h) and the supernatants after 5h and 9h of growth were analyzed. At 0h, there were differences in the measured parameters between donors due to person-specific saliva properties (FIG. 2A-E).

In the condition with an additional 6.5 mM nitrate (i.e., 403 mg/I), most nitrate was used up after 5h (FIG. 2A): in 7 individuals there was no nitrate detectable after 5h, while for the other 5 donors, the nitrate had decreased 76-85%. Nitrite, in turn, increased from an average of 1.64 mg/l at 0h to 64.68 mg/l at 5h, while at 9h most of it was metabolized as well (FIG. 2B).

After 5h, the average ammonium had increased 1.72x in the control condition and 2.21x in the nitrate condition (both p < 0.005), and the difference between nitrate and control conditions was significant (p < 0.01, FIG. 2C). After 9h, the ammonium had further increased under both conditions (p < 0.005), remaining significantly higher in the nitrate group (p < 0.005). The average lactate increased notably after 5h in both conditions (4.74x in the control condition and 3.40x in the nitrate condition, both p < 0.005), but was significantly lower in the nitrate condition (p < 0.005, FIG. 2D). After 9h, the lactate seemed to have been partly metabolized, decreasing significantly in both conditions (p < 0.005), but stayed significantly lower in the nitrate condition (p < 0.005). There was a negative correlation between lactate and ammonium at 9h that was more evident in the nitrate condition (R= -0.71, p < 0.05 in control and R = -0.87, p < 0.0005 in nitrate condition).

In accordance with a higher amount of ammonium production and lower amounts of lactate, pH was significantly higher in the nitrate condition at 5h and 9h (both, p < 0.005, FIG. 2E). In respect to this, the pH dropped significantly after 5h in the control condition (p < 0.005), but not in the nitrate condition (p = 0.056). Interestingly, at 5h, there was a negative correlations between pH and nitrite in the nitrate condition (R= -0.82, p < 0.005): individuals with 0 mg/L nitrite, possibly all used up, had the highest pH. Likewise, in the nitrate condition, ammonium correlated negatively with nitrite at 5h (R= -0.64, p < 0.05).

To see if nitrate would have an effect on salivary acidification by sugar without the presence of cultivation medium, unstimulated saliva was incubated with 0.2% glucose and a concentration range of nitrate from 0.5-8.5 mM during 5h (FIG. 3). The salivary pH before growth was 7.17 (SD 0.41). After 5h of incubation with 0.2% glucose without nitrate, the pH dropped to pH 4.71 (SD 0.29, LQ 4.49, UQ 4.96). All nitrate concentrations from 0.5 mM to 8.5 mM resulted in a higher pH after 5h compared to 0 mM nitrate (p < 0.05 for 1 mM and 1.5 mM, p < 0.01 for higher concentrations up to 8.5 mM).

### 9. Nitrate strongly affects biofilm composition

The addition of nitrate had a significant effect on biofilm bacterial composition (Compound Cluster Analysis, CCA, Adonis p-value: 0.001), explaining a large proportion of data variability regardless of biofilm sampling time (FIG. 4).

In the control condition, the five most common genera after 5h of biofilm growth (Table 1) were *Streptococcus* (48.02% SD 9.93%), *Veillonella* (18.43% SD 8.01%), *Haemophilus* (7.33% SD 4.52%), *Neisseria* (6.56% SD 2.98%), and *Prevotella* (3.82% SD 5.19% / SE 1.50%). In the nitrate condition, the most abundant genera after 5h were *Streptococcus* (45.29% SD 9.53%), *Neisseria* (20.65% SD 8.36%), *Veillonella* (10.84% SD 8.11%), *Haemophilus* (7.30% SD 4.27%) and *Gemella* (2.03% SD 1.60%). At 9h the percentages changed slightly, but the order of the 5 most abundant genera remained identical with the exception of *Prevotella* being slightly more prevalent than *Gemella* in the nitrate condition. In the saliva used as inoculum, comparable dominant genera were found with a similar percentage of *Streptococcus* (43.38%, SD 2.97%) on the first place and then *Neisseria, Gemella* and *Veillonella* (data not shown).

**Table 1: Bacterial abundance in oral biofilms with and without 6.5 mM nitrate**

| **Genus** | **5 h (n = 12)** | | | | **9 h (n = 12)** | | | |
|---|---|---|---|---|---|---|---|---|
| | **Nitrate (%)** | | **Control (%)** | | **Nitrate (%)** | | **Control (%)** | |
| | **Average** | **SD** | **Average** | **SD** | **Average** | **SD** | **Average** | **SD** |
| ***Streptococcus*** | 45.29 | 9.53 | 48.02 | 9.93 | 31.75 | 4.41 | 38.48 | 6.16 |
| ***Veillonella*** | 10.84 | 8.11 | 18.43 | 8.01 | 20.46 | 3.38 | 28.04 | 3.76 |
| ***Neisseria*** | 20.65 | 8.36 | 6.56 | 2.98 | 20.99 | 5.42 | 5.78 | 3.24 |
| ***Haemophilus*** | 7.30 | 4.27 | 7.33 | 4.52 | 8.86 | 3.89 | 8.37 | 3.60 |
| ***Prevotella*** | 1.99 | 3.43 | 3.82 | 5.19 | 2.78 | 4.63 | 3.37 | 5.22 |
| ***Gemella*** | 2.78 | 2.54 | 2.03 | 1.60 | 1.83 | 1.51 | 1.30 | 1.10 |
| ***Granulicatella*** | 2.16 | 1.60 | 2.38 | 1.34 | 1.56 | 0.73 | 1.85 | 0.76 |
| **unclass. *Pasteurellaceae*** | 2.00 | 3.08 | 2.22 | 3.88 | 2.12 | 2.30 | 2.19 | 3.14 |
| ***Porphyromonas*** | 0.56 | 0.37 | 1.11 | 0.99 | 1.51 | 1.05 | 1.88 | 1.37 |
| ***Fusobacterium*** | 0.15 | 0.14 | 0.40 | 0.33 | 0.38 | 0.25 | 1.17 | 1.27 |
| **unclass. *Neisseriaceae*** | 0.95 | 0.38 | 0.38 | 0.18 | 1.07 | 0.49 | 0.35 | 0.18 |
| ***Aggregatibacter*** | 0.55 | 0.72 | 0.59 | 1.30 | 0.72 | 0.93 | 0.34 | 0.40 |
| ***Leptotrichia*** | 0.14 | 0.12 | 0.61 | 1.10 | 0.18 | 0.31 | 0.28 | 0.25 |
| **unclass. *Veillonellaceae*** | 0.26 | 0.17 | 0.49 | 0.38 | 0.38 | 0.13 | 0.57 | 0.16 |
| ***Rothia*** | 0.53 | 0.52 | 0.23 | 0.27 | 0.25 | 0.24 | 0.13 | 0.12 |
| ***Alloprevotella*** | 0.21 | 0.29 | 0.50 | 0.70 | 0.37 | 0.48 | 0.51 | 0.58 |
| **Other** | 3.64 | 1.59 | 4.89 | 3.27 | 4.80 | 3.92 | 5.39 | 4.02 |

The lower abundance of *Veillonella* in the nitrate condition compared to the control condition was significant at 5h and 9h (p < 0.01, FIG. 5) as well as the lower percentage of *Streptococcus* at 9h (p < 0.01). The genera *Streptococcus* and *Veillonella* are both caries associated (i.e., these genera increase in caries). Interestingly, a shift of OTUs was observed within these genera, including an increase of *S. parasanguinis* at 5h and an increase of V. *disparat* 9h (both p < 0.05, data not shown). The periodontitis- and halitosis-associated genus *Prevotella* at 5h was lower in the control condition (3.82%, SD 5.19% compared to 1.99%, SD 3.43% in the nitrate condition, p < 0.01). At 9h, no significant difference in *Prevotella* was observed on a genus level but at the OTU level, a relative higher percentage of the most abundant species, *P. pallens,* was observed in the nitrate condition (p < 0.05, data not shown). No significant differences were observed between *Haemophilus and Gemella* in the two conditions.

The 3.5x increase at 5h and 4.9x at 9h of health-associated *Neisseria* in the nitrate condition compared to the control condition were significant (both p < 0.01, FIG. 5). While *Neisseria* clearly increased in number, the identified OTUs, *N. flavescens, N. subflava, N. bacilliformis,* and *N*. *elongata,* did not change significantly in relative abundance. *Rothia,* another nitrate-reducing genera associated with health dominated by an OTU classified as *"R. aeria* or *R. dentocariosa",* was less abundant (i.e., average in all conditions 0.3% SD 0.34%, range 0.01-1.7%), but 2.7x higher in the nitrate condition at 5h and 9h (p < 0.01 and p < 0.05, respectively). Finally, the health-associated genus *Kingella* increased significantly after 5h (p < 0.05). Interestingly *Rothia, Neisseria* and *Kingella* are health-associated from a caries, periodontal diseases and halitosis point of view.

Other genera significantly lower (p < 0.05 at 5h and/or 9h) in the nitrate condition were periodontitis- and halitosis-associated *Porphyromonas* (including an OTU *"P. endodontalis* or *oral taxon 285*")*, Fusobacterium* (including *F. periodonticum* and *F. nucleatum*), *Leptotrichia* (including *an OUT "L. wadei* or oral taxon 417", and *L. hongkongensis*), *Avoprevotella* (including *A. rava* and *A. tannerae*), *Dialister* and *Parvimonas.* Additionally, caries-associated *Atopobium* and and *Oribacterium* (including *O. parvum* and *O. sinus*) decreased significantly (p < 0.05 at 5h or 5h and 9h, respectively). There was also a trend in the decrease of the periodontitis- and halitosis-associated bacteria *Peptostreptococcus, Eubacterium, Treponema, Tannerella, Solobacterium* and *Selenomonas,* but the difference was not significant (FIG. 5).

When grouping the sequenced biofilms from 12 donors (D1-D12) in both conditions (control and nitrate) together, *Leptotrichia* and *Oribacterium* correlated positively with lactate at 5h (R = 0.72 and R = 0.70, both p < 0.05). In the nitrate condition, *Veillonella* correlated negatively with pH at 9h (R = -0.77 and R = -0.76, respectively, both p < 0.005). In contrast, Neisseria correlated positively with pH at 9h in both conditions (control condition; R = 0.75, p < 0.005, nitrate condition; R = 0.84, p < 0.001).

### Conclusions

10. The effects of 6.5 mM nitrate during oral biofilm development *in vitro* were tested. The results show that biofilms grown with nitrate contained several times higher levels of the health-associated, nitrate-reducing genera *Neisseria* and *Rothia.* This included an increase of total abundance of *Rothia mucilaginosa* and *Neisseria flavescens.* It appears that species of *Rothia* and *Neisseria* have a selective advantage in the presence of nitrate. *Neisseria* correlates with anti-inflammatory mediators and is associated with a better recovery of the gingiva after experimental gingivitis. Also *Rothia* is often related to periodontal health and *Rothia dentocariosa* was recently associated with halitosis-free individuals. Additionally, both *Rothia* and *Neisseria* have been associated with caries-free individuals. The increase of *Neisseria* and *Rothia* may thus be considered a positive change related to general oral health.

11. Another important observation in the experiments was that periodontal-disease associated *Porphyromonas, Fusobacterium, Prevotella, Leptotrichia* and *Alloprevotella* were significantly lower in biofilms grown with nitrate after 5h. *Porphyromonas, Fusobacterium,* and *Prevotella* contain species of the classic 'red and orange complexes', which in that study were the bacteria with the strongest association with periodontitis, including *Fusobacterium periodonticum, Prevotella intermedia* and *Prevotella nigrescens* that were also identified in our study. Similarly, *Leptotrichia* has a strong association with periodontitis, while very recently it was confirmed that *Alloprevotella* is more abundant in disease too. It is interesting to note that the other two members of the 'red complex', namely *Tannerella* and *Treponema,* were also found at lower levels in the nitrate condition, but the difference was not significant.

It is interesting to note that *Porphyromonas, Fusobacterium, Leptotrichia* and *Prevotella* are also associated with halitosis - bad breath resulting from microbial production of volatile sulfur compounds (VSCs). These VSCs include hydrogen sulfide and methyl mercaptan. Hydrogen sulfide is known to be genotoxic (i.e., it damages the DNA of human cells) and causes inflammation in the colon and mouth. In respect to this, hydrogen sulfide production is thought to contribute to the development of periodontal diseases, linking halitosis with periodontitis.

12. Other genera that showed a trend in decreasing in the nitrate condition were *Peptostreptococcus, Eubacterium, Solobacterium* and *Selenomonas,* which all have been associated with periodontitis and halitosis. In summary, the addition of nitrate decreases the abundance of periodontal diseases- and halitosis-associated species.

13. The data also show that less lactate and more ammonium was produced at 5h and 9h in the nitrate condition, and, accordingly, the pH was higher than in the control condition (all p < 0.01). Additionally, there was a strong negative correlation between lactate and ammonium after 9h of biofilm growth, which was more evident in the nitrate condition. This supports the hypotheses that alkali production and lactate consumption by nitrate-reducing communities limit a drop in pH when carbohydrates are fermented. *In vivo* this could potentially reduce the time that the dental tissue is under demineralizing pH, a critical factor for tooth decay. In this study, it is found that nitrate concentrations from 0.5 mM prevented salivary acidification due to glucose fermentation after 5h, while no additional benefits were observed for concentrations above 3.5 mM.

14. Regarding bacterial composition, a decrease in *Veillonella, Streptococcus, Atopobium Oribacterium* was observed, which are genera associated with lactate, acidification and caries in the nitrate condition after 5h or 9h (p < 0.05).

15. The metabolism of nitrite and the production of ammonium in this study indicate Dissimilatory Nitrate Reduction to Ammonium (DNRA) activity by oral species. The observation that nitrite correlated negatively with ammonium and pH at 5h (biofilms that metabolized all nitrite produced most ammonium and had the highest pH) further supports this. In the nitrate condition, the amount of ammonium after 9h was 4.75 mM higher than in the control condition. Stoichiometrically, this could account for 73.1% of the 6.5 mM added nitrate, while (part of) the other 26.9% of nitrate must have been denitrified into nitric oxide and other nitrogenous products.

16. Nitrate administration is therefore used as a prebiotic that is converted by certain oral microorganisms into ammonium, increasing the local pH and thereby having an anti-caries effect *in vivo.* Apart from ammonium production, the conversion of nitrite into nitric oxide could further limit caries development. In the case of periodontitis, nitrate supplementation could lead to nitric oxide production, limiting the growth of periopathogenic species. This is an advantage compared to arginine supplementation that increases periopathogenic bacteria, like *Treponema, Prevotella* and *Eubacterium* (Koopman et al., 2016).

17. The results in this study showed that nitrate caused a structural and functional shift in oral communities that would be of benefit to the human host. Based on the results it can be concluded that nitrate is necessary for a healthy oral microbiota and could be used as a prebiotic, reducing levels of cariogenic, periopathogenic and halitosis-associated species, while increasing levels of health-associated, nitrate-reducing species. The strength of this shift in biofilm composition was reflected by the observation that nitrate could explain a large proportion of data variability in bacterial composition regardless of biofilm sampling time. Additionally, it is concluded that nitrate metabolism provide resilience to acidification resulting from sugar metabolism by increasing lactate consumption and ammonium production. In biofilms grown with nitrate, *Veillonella,* a genus that uses lactate as a carbon source, correlated negatively with pH and *Neisseria* positively. *Neisseria, Rothia* and *Kingella* have essential roles in maintaining a healthy symbiotic relationship between the oral microbiota and the host by the reduction of salivary nitrate.

### EXAMPLE 2: In vivo study - oral administration of nitrate to human persons

Overview: Clinical studies were performed in which it was obtained the first *in vivo* evidence that a nitrate-rich supplement affects bacterial activity directly after a single intake (i.e. an acute effect). This effect was shown to happen via topic application and via ingestion of the product.

### 18. Methods

### Study 1: Topic (direct) and ingested (indirect) effect on salivary nitrate

Nitrate was measured as in EXAMPLE 1 in different saliva samples, collected every 30 minutes for a period of 6 hours, from an individual under fasting conditions, after ingestion of 220 mg nitrate in 200 ml volume.

### Study 2: Effect of a topic nitrate composition on bacterial activity

Nitrate, nitrite and pH was measured as in EXAMPLE 1, in different saliva samples collected from 6 individuals according to the following protocol:
- Collect saliva before and 10 minutes after a 10% sucrose rinse
- Intake of a highly concentrated nitrate-rich supplement (300 mg nitrate in 70 ml volume)
- Wait for 1 hour without eating or drinking
- Collect saliva before and 10 minutes after a 10% sucrose rinse.

### Study 3: Effect of an ingested nitrate composition on bacterial activity

Nitrate, nitrite and pH was measured as in EXAMPLE 1, in different oral samples collected from 6 individuals according to the following protocol:
Day 1 (control day)
   - Collect saliva sample in the morning, before and 10 minutes after a 10% sucrose rinse.
   - Wait for 4 hours without eating
   - Collect saliva sample before and 10 minutes after a 10% sucrose rinse
Dav 2 (supplement day)
Morning:
   - Collect saliva sample in the morning, before and 10 minutes after a 10% sucrose rinse.
   - Take nitrate-rich supplement (220 mg per dose)
   - Wait for 4 hours without eating and collect saliva samples every hour (samples 1h, 2h, and 3h)
   - Collect saliva at 4 hours, before and 10 minutes after a 10% sucrose rinse.

### Study 4: Compared effect of an ingested nitrate composition and placebo (blinded cross-sectional study)

Nitrate, nitrite and pH was measured as in EXAMPLE 1, in different oral samples collected from 12 individuals according to the following protocol:
- Collect saliva sample in the morning, before and 10 minutes after a 10% sucrose rinse.
- Take nitrate-rich supplement (250 mg nitrate in 200 ml water) or placebo (0 mg nitrate in 200 ml water)
- Wait for 1h 45mins without eating or drinking
- Collect saliva sample before and 10 minutes after a 10% sucrose rinse.

### 19. Results

In Study 1, it was tested how a nitrate-rich supplement affects salivary nitrate levels (FIG. 6). Data show a direct increase of salivary nitrate after the consumption of the supplement as a consequence of the topical contact of the product with oral tissues, which is slowly reduced by saliva clearance and swallowing. A second peak is observed after 2.5 hours, as a consequence of the recycling activity of the salivary glands, which concentrate nitrate from plasma into saliva, resulting in elevated nitrate concentrations during the 6 tested hours.

Thus, Study 2 and Study 3 were performed to study the acute effect of a single use of a nitrate-containing product on bacterial activity during the direct (topical administration) and indirect (ingested administration) salivary nitrate increase, respectively. For this, the effect of nitrate-rich supplements on the bacterial acidogenic metabolism resulting from a 10% sugar rinse, which decreases the salivary pH, was tested. Pre- and post-sugar measurements of pH, nitrate and nitrite in basal and after-supplement saliva samples in these two studies are shown in Table 2.

**Table 2: Salivary nitrate, nitrite and pH levels in Studies 2 and 3.**

| **Study** | | **Study 2 (n = 6)** | | **Study 3 (n = 6)** | | | |
|---|---|---|---|---|---|---|---|
| **Nitrate dose** | | 300 mg in 70 ml | | 220 mg in ≥150 ml | | None (control day) | |
| **Timepoint** | | Baseline | 1h | Baseline | 4h | Baseline | 4h |
| **Nitrate (mg/l)** | **pre-sugar** | 120.0 | 546.7 | 13.3 | 45.8 | - | - |
| | **post-sugar** | 58.3 | 388.3 | 11.7 | 32.5 | - | - |
| **Nitrite (mg/l)** | **pre-sugar** | 13.2 | 53.3 | 9.8 | 17.3 | - | - |
| | **post-sugar** | 5.5 | 71.5 | 8.2 | 10.4 | - | - |
| **pH** | **pre-sugar** | 6.95 | 6.97 | 6.98 | 7.32 | 6.95 | 7.18 |
| | **post-sugar** | 6.58 | 6.73 | 6.52 | 6.87 | 6.57 | 6.57 |

Both studies showed that nitrate and nitrite always increased significantly after the supplement and that nitrate and nitrite always decreased significantly right after sugar rinse. This indicated that a single dose of nitrate had an acute effect in nitrate salivary levels and that it had an acute effect in microbial activity by inducing nitrate-reducing and nitrite-reducing activity.

Regarding pH values, in Study 2 it was observed that 1 hour after intake of a concentrated supplement, nitrate already prevented the second drop in pH by the sugar rinse (FIG. 7A). However, the basal salivary pH did not increase significantly. In Study 3, data showed that after 4h, the basal pH increased significantly (FIG. 7B. Additionally, the pH drop after taking the supplement was not significant anymore. The data therefore showed that the effect of a topically-administered nitrate composition on bacterial activity is significant, but less strong than the indirect effect of an ingested nitrate composition, and that the strong effect observed after 4 hours *in vivo* matches the one observed in EXAMPLE 1 *ex vivo.*

In Study 4, the pH drop after the nitrate supplement compared to the placebo supplement was limited (FIG. 8B). Additionally, there was a trend of basal pH increase (p = 0.098, FIG. 8A). These data indicated that a nitrate composition requires an indirect effect as a consequence of ingestion to influence basal changes in bacterial activity, whereas a topically administered nitrate composition is sufficient to influence post-sugar changes in bacterial activity. In addition, the significant effect of the supplement compared to the placebo confirmed that the observed effect *in vivo* is due to nitrate and not to water ingestion.

### 20. Conclusions

The data obtained from the different Studies *in vivo* demonstrated that after a single application of a nitrate supplement, nitrate and nitrite are detected in saliva, and that they instantly drop after a sugar rinse (only 10 minutes time). In addition, a single nitrate administration always mitigated the pH drop after a sugar rinse. This implies that a single application of a nitrate product is enough to modify the activity of the oral microbiota, providing resilience against acidification. This resilience can be increased by waiting more time (4h) so that nitrate-reducing bacteria have increased, like observed ex *vivo* after 5h (EXAMPLE 1). Possible mechanisms that prevent the post-sugar pH drop include production of the acid-neutralizer ammonium, production of the antimicrobial molecule nitric oxide and lactate consumption by nitrate-reducing bacteria, as shown in EXAMPLE 1. Thus, our *in vivo* results support the ex *vivo* results shown in EXAMPLE 1: an acute nitrate application limits pH drops and could thus be an effective prebiotic against caries, by reducing the bacterial dysbiosis associated to sugar, which is the main disease driver of tooth decay.

Without being limited to theory - it was a surprise for the present inventors that there was an improvement (alkalization) of the salivary pH both before and after a sugar rinse and also that this improvement was observed acutely after 1-4 hours. The sugar rinse simulates a meal, where it is known that the pH decreases - accordingly, this example surprisingly shows that by administration of nitrate may provide a protection from a pH drop e.g. after a meal. Further, the results provide *in vivo* evidence that the levels of nitrate in saliva remain at elevated concentrations (i.e., above the fasting levels of donors) for a period of at least 6 hours. This makes it plausible that e.g. a food supplement, a toothpaste or a tablet containing nitrate has a positive effect to reduce oral dysbiosis after a single dose and within 24 h, i.e. an acute effect, as opposed to the current state of the art where changes in oral microbiota composition *in vivo* are shown only after 1-4 weeks treatment, i.e. a chronic effect. Additionally, doses of 220 mg and 250 mg (below the ADI of an adult of 60 or 70 kg, respectively) of nitrate were enough to prevent a pH drop due to sugar metabolism, while in prior art, the daily doses used for 1-4 weeks were of 372-770 mg (1.7-3.5x the ADI for an adult of 60 kg).

### EXAMPLE 3: Isolation of nitrate-reducing species from healthy donors for use as probiotics

### Materials and methods

### 21. Donor selection and sample procedure

Subjects without caries and periodontitis were recruited as donors. All participants were required to have good oral health, which was assessed by a dentist, and a healthy blood pressure, which was measured with an Automatic Blood Pressure Monitor Model M6 Comfort IT (OMRON Healthcare Europe B.V). Plaque or tongue coating samples were collected by a dentist and resuspended in 1 mLof PBS.

### 22. Nitrate-reducing species isolation

Plaque or tongue samples in PBS were diluted 10² to 10⁷ times and plated on Brain Heart Infusion (BHI) 1.4% agar plates (Merck Millipore). Plates were incubated at 37°C during 2 days to obtain separated colonies in some of the dilutions. A protocol adapted from Doel et al., 2005 and Mashimo et al., 2015 was employed to detect nitrate-reducing activity directly on the plates. It consists of a double agar overlay method based on the Griess reaction that stains nitrite. The colonies with nitrate-reducing capability produced a red colour due to the presence of nitrite. These colonies were then transferred to new BHI agar plates and incubated during 2 more days at 37°C. The nitrate-reducing capability of the isolates was confirmed repeating the double overlaid agar method for each isolate. Subsequently, one colony was passed to 5 mL of liquid BHI and incubated aerobically for 2 days at 37°C. After 2 days, part of the medium was used to create a glycerol stock of each isolate for future experiments. The rest of the medium was centrifuged at 4000 rpm during 15 min and the pellet was suspended in 100 µL PBS and stored at -20°C until DNA extraction.

### 23. DNA extraction from nitrate-reducing isolates and taxonomic classification

DNA extraction was performed using MagNA Pure LC DNA Isolation Kit III (Bacteria, Fungi) (Roche Diagnostics, Mannheim, Germany), according to the manufacturer's protocol, and DNA concentrations were measured using a NanoDrop 1000 spectrophotometer (ThermoScientific). A PCR was performed to amplify the 16S rRNA gene of each isolate, using universal primers 8-F and 785-R for the 16S rRNA gene, comprising the hypervariable regions V1-V2-V3-V4 (SEQ ID NO: 9 and 10). The PCR products were then purified using flat 96 well filter plates (NucleoFast 96 PCR, Macherey-Nagel) and sequenced by Sanger technology. To taxonomically assign the isolates, the sequences were analyzed using BLASTn against 16S ribosomal RNA sequences at NCBI nr database.

### 24. Nitrate Reduction Test of bacterial isolates

The concentrations of nitrate, nitrite and ammonium (the ion of ammonia) were measured in spent medium to determine the capacity of each isolate to reduce or produce these compounds. For this, isolates were taken out of their stocks and incubated in 5 mL of BHI liquid medium overnight at 37°C. The next day, isolates were diluted in BHI to an OD of 0.01 and a final nitrate concentration of 6.5 mM. Then, the tubes were incubated for 7 hours and 1 mL was taken at 4 and 7 hours after vortexing. The OD was measured at 4 and 7 hours and the samples were frozen at -20°C before other measurements. The same experiment was performed with 5h of growth using three types of buffered medium (100 mM MES, pH 6.0; 100 mM HEPES, pH 7.0; 100 mM HEPES pH 7.5) to keep a stable pH and see the effect of different pH levels on the nitrate-reducing capacity of a selection of isolates.

### 25. Nitrate, nitrite, ammonium and pH measurements

Nitrate, nitrite, ammonium and pH were measured with a reflectometer as described in EXAMPLE 1.

### 25.1. Effect of isolates on in vitro biofilms

Six isolates selected as best potential probiotics based on the results from previous experiments were studied *in vitro* to define the effect of these isolates when they are added to an oral biofilm. These isolates were tested by growing them with saliva of two different donors (D2 and D25 in our database) in a 96-wells plate. For each experiment, there were 4 conditions: control, nitrate (i.e., 6.5 mM nitrate), control + isolate, and nitrate + isolate. For all samples prepared in duplicate, 100 µL of BHI (with 0.05 mg/L haemin, 0.005 mg/L menadione and 0.2 mM vitamin K) were added to each well. Then, 100 µL of saliva (or BHI for negative controls) was added and, for the nitrate conditions, 10 µL of nitrate solution 65 mM was added (or 10 µL of BHI for control conditions). Negative controls were added in each experiment: only BHI medium and BHI medium + nitrate. Before being added to the 96-well plate, the isolates were grown for 24h. Then, 40 µL isolate in BHI solution with OD 1.5 was added (or 40 µL of just BHI in conditions without isolates) to each well. The final concentration of nitrate was 6.5 mM and starting OD of the isolate was 0.24. The 96-well plate was sealed to prevent oxygen presence, and incubated during 5 h 30 min at 37°C. After that, the supernatant was collected, the pellet resuspended in 30 µL of PBS and both stored at -20°C until measurements were performed. The DNA was extracted and the biofilms were sequenced as described in EXAMPLE 1.

### Results

### 26. Identification of nitrate-reducing species

Tongue and plaque samples were plated from 5 different healthy donors. The colonies that reduce nitrate were detected by a red tone, resulting from a Griess reaction. In total, stocks of 67 nitrate reducing isolates were identified.

### 27. Best probiotic isolate selection based on nitrate reducing capacity

Different percentages of nitrate were reduced by each isolate when incubating them with 6.5 mM of nitrate during 4 and 7 hours. Only D3T4 reduced 100% of the nitrate after 4 hours of incubation. In contrast, another six isolates had not reduced any percentage of nitrate by this time and were discarded (FIG. 9A).

Thirty-three isolates that had reduced 100% of the nitrate at 7 hours and >19% of nitrate at 4 hours were selected (FIG. 9B). From these 33 isolates, 5 isolates were selected from tongue (T) and 5 isolates from plaque (P) that did not lower the medium pH below 6.8 (i.e. they were not acidogenic) and had a good growth rate (optical density higher than 0.7 after 7 hours of incubation). Moreover, between the isolates that fulfilled the requirements, different species from different donors were selected. The isolates selected to continue with our studies were D1P7 (CECT9999), D1P10, D1P15A, D1P17 (CECT30000), D3T4 (CECT30001), D4P7, D4T4 (CECT30002), D4T6 (CECT30003), D4T9 (CECT30004), D5T11A (CECT30005).

### 28. Nitrate-reducing capacity depending on pH

When the 10 selected isolates were incubated during 5 hours with 6.5 mM of nitrate at three different pH levels (pH 6.0, 7.0 and 7.5), it was shown that the nitrate-reducing capacity differed between pH values and this was isolate-dependent. For example, isolate D1P7 reduced 100% of nitrate after 5 hours of incubation at a pH of 7.5, but only reduced around 52% of nitrate when pH was 6.0. Opposite to D1P7, D4T6 reduced 77% of nitrate when pH was 6.0, but it reduced only 35% of nitrate at a pH of 7.5 (FIG. 10A, Table 3).

The amount of nitrite further reduced to nitric oxide and other compounds, expressed as a percentage of the nitrate reduced after 5 h of incubation, differed depending on pH (FIG. 10B). Some of the isolates converted most of the nitrite to nitric oxide or other compounds, but the optimal pH level differed between isolates. At pH 6, nitrite reduction was stimulated compared to pH 7 and 7.5 (p < 0.05 and p < 0.01, respectively) but the degree of nitrite reduction was also strain-dependent.

It was determined how much of the percentage of nitrate reduced after 5 h (FIG. 10A) was detected as nitrite, taking into account a 1:1 molar reaction (Table 3). The rest of the reduced nitrate, which was not detected as nitrite, had been further reduced to other compounds. Nitrite can be reduced to ammonia and nitric oxide. Isolates that produced most nitrite were considered to be suitable for systemic applications (e.g., to reduce hypertension), while isolates that produced most nitric oxide and other compounds were considered to be suitable to prevent oral diseases. The pH at which these compounds were detected further determined for what preferred oral disease each isolate would be suitable (caries are caused by an acidic pH, while periodontal diseases and halitosis happen at neutral to slightly alkaline pH levels).

**Table 3. Nitrite and nitric oxide produced by isolates grown at different pH for 5h with 6.5 mM nitrate.**

| **Isolate** | **Species (16S)** | **Nitrate reduced (NO3R) into:** | **pH 6.5** | **pH 7** | **pH 7.5** | **Suitable applications** |
|---|---|---|---|---|---|---|
| D1P7 (CECT9999*¹) | *Rothia aeria* | **Total NO3R (%)** | **52*** | - | **100*** | Periodontal diseases (perio.)*³ / general oral health |
| | | % nitrite | 18 | - | 78* | |
| | | % nitric oxide*² | 34 | - | 22* | |
| D1P10 | *Rothia dentocariosa* | **Total NO3R (%)** | **48** | **47** | **23** | Caries |
| | | % nitrite | 14 | 24 | 21 | |
| | | % nitric oxide*² | 34* | 23* | 2 | |
| D1P15A | *Rothia dentocariosa* | **Total NO3R (%)** | **50** | **53** | **43** | General oral health |
| | | % nitrite | 16 | 33.5 | 24 | |
| | | % nitric oxide*² | 34* | 19.5 | 19* | |
| D1P17 (CECT30000) | *Rothia dentocariosa* | **Total NO3R (%)** | **48** | **49** | **49** | Systemic applications |
| | | % nitrite | 19.5* | 33 | 34* | |
| | | % nitric oxide*² | 28.5 | 16 | 15 | |
| D3T4 (CECT30001) | *Rothia mucilaginosa* | **Total NO3R (%)** | **34** | **61*** | **69*** | Perio. / systemic applications |
| | | % nitrite | 12.5 | 39* | 50* | |
| | | % nitric oxide*² | 21.5 | 22* | 19* | |
| D4P7 | *Rothia dentocariosa* | **Total NO3R (%)** | **53*** | **46** | **48** | General oral health |
| | | % nitrite | 14 | 31 | 25.5 | |
| | | % nitric oxide*² | 39* | 15 | 22.5* | |
| D4T4 (CECT30002) | *Rothia mucilaginosa* | **Total NO3R (%)** | **52*** | 55 | **52*** | Systemic applications |
| | | % nitrite | 19* | 44* | 36* | |
| | | % nitric oxide*² | 33 | 11 | 16 | |
| D4T6 (CECT30003) | *Rothia mucilaginosa* | **Total NO3R (%)** | **77*** | **66*** | **35** | Caries |
| | | % nitrite | 38* | 37* | 32 | |
| | | % nitric oxide*² | 39* | 29* | 3 | |
| D4T9 (CECT30004) | *Rothia mucilaginosa* | **Total NO3R (%)** | **59*** | **67*** | **57*** | General oral health |
| | | % nitrite | 21.5* | 33 | 29 | |
| | | % nitric oxide*² | 37.5* | 34* | 28* | |
| D5T11A (CECT30005) | *Rothia mucilaginosa* | **Total NO3R (%)** | **48** | **55** | **78*** | Systemic applications |
| | | % nitrite | 18* | 38* | 59.5* | |
| | | % nitric oxide*² | 30 | 17 | 18.5 | |
| Medians | - | **Total NO3R (%)** | **51** | **55** | **51** | - |
| | - | % nitrite | 18 | 33 | 33 | - |
| | - | % nitric oxide*² | 34 | 19 | 18 | - |

| | | | | | | |
|---|---|---|---|---|---|---|
| * number above median *¹ registered in culture collection and included in this patent *² nitric oxide and other compounds that may result from nitrite reduction (e.g., ammonium) *³ due to protein degradation, halitosis also happens at a neutral to alkaline pH and the same probiotics proposed for periodontal diseases can be applied | | | | | | |

### 29. Effects of isolates on oral biofilm development

The effect of six out of 10 selected isolates (five *Rothia mucilaginosa* and one *Rothia aeria*) on oral biofilm development was tested *in vitro.* For this, biofilms were grown from saliva of two different donors (named with codes D2 and D25, FIG. 11) during 5h 30 min. The isolates, which were added from the beginning, appeared to colonize the biofilms successfully as indicated by the final percentages of their corresponding species detected by sequencing (Table 4). All isolates grew better in the presence of nitrate. Biofilms grown with and without nitrate in the presence or absence of the isolates were compared (FIG. 11). Additionally, the results were compared with controls of growth medium with and without nitrate (Cnt, FIG. 11).

**Table 4: Percentage of probiotics in biofilms at 5h of growth after probiotic application in samples from two donors with different NRCs**

| **Isolate** | **Condition** | **Donor 2** | | **Donor 25** | |
|---|---|---|---|---|---|
| | | ***R. mucilaginosa* (%)** | ***R. aeria* (%)** | ***R. mucilaginosa* (%)** | ***R. aeria* (%)** |
| **None** | Control | 4.48 | 0.09 | 5.11 | 0.03 |
| | Nitrate | 2.33 | 0.06 | 7.94 | 0.03 |
| D1P7 (CECT9999) | Control | 0.85 | 10.13 | 0.15 | 32.34 |
| | Nitrate | 2.64 | 17.49 | 0.36 | 41.09 |
| D3T4 (CECT30001) | Control | 22.08 | 0.02 | 37.15 | 0.00 |
| | Nitrate | 26.36 | 0.04 | 45.83 | 0.00 |
| D4T4 (CECT30002) | Control | 17.14 | 0.02 | 47.43 | 0.00 |
| | Nitrate | 27.95 | 0.04 | 58.63 | 0.00 |
| D4T6 (CECT30003) | Control | 15.23 | 0.03 | 33.74 | 0.00 |
| | Nitrate | 24.53 | 0.05 | 58.14 | 0.00 |
| D4T9 (CECT30004) | Control | 19.96 | 0.03 | 42.43 | 0.00 |
| | Nitrate | 34.12 | 0.04 | 54.53 | 0.00 |
| D5T11A (CECT30005) | Control | 5.14 | 0.04 | 35.15 | 0.00 |
| | Nitrate | 14.43 | 0.04 | 40.72 | 0.00 |

### 30. Adding isolates to donor without oral NRC (D25)

The *in vitro* oral biofilm grown from the saliva of D25, which had an alkaline pH (pH 7.8 before mixing it 1:1 with growth medium), the nitrate did not decrease compared to the control and little nitrite was produced, showing that the oral microbiota of this donor had a dramatically low nitrate-reducing capacity (NRC). However, a large percentage of nitrate was reduced in nitrate + isolate condition (FIG. 11E) and the concentration of nitrite also increased notably (FIG. 11F). This indicates that the addition of a probiotic was able to compensate the lack of nitrate-reducing capacity.

The concentration of nitrate was higher when nitrate was added to saliva in BHI (FIG. 11E, None, black bars) than when nitrate was added to just BHI (None, FIG. 11E, Cnt, striped black bars), indicating that the saliva contained some nitrate. The pH was maintained stable when nitrate was added to saliva (nitrate condition) but it decreased when isolates were added without nitrate. When adding isolates and nitrate together, the pH dropped significantly less (p<0.05), and for some isolates it even increased (e.g., D3T4, FIG. 11G).

### 31. Adding isolates to donor with normal oral NRC (D2)

When the same experiment was performed with the saliva of donor D2, who had a lower salivary pH (pH 6.8 before mixing 1:1 with BHI growth medium), it was shown that pH increased when adding nitrate in the presence or absence of isolates (FIG. 11C). In one case (D3T4), the addition of the isolate together with nitrate increased pH more than nitrate alone. Interestingly, the isolates without nitrate also appeared to prevent a pH drop compared to the control condition for this donor.

The biofilms of D2 grown with nitrate almost reduced all nitrate after 5 h 30 min, even when no isolates were added. This suggests that the donor has a good nitrate-reducing capacity and the addition of nitrate alone is enough to promote its reduction.

In the case of both donors, when grouping all isolates together, the pH was significantly higher in the presence of nitrate than in the absence of nitrate (p<0.05). Looking at ammonium, the concentrations of ammonium were similar in all conditions for both donors and no significant changes were observed (FIG. 11D & H). Other factors explaining the prevention of a pH drop when nitrate is reduced are lactate consumption and apparent nitric oxide production as observed in EXAMPLE 1.

### Conclusions

32. Isolates that further reduced most nitrite to other compounds were selected as potential probiotics against oral diseases, taking into account that nitric oxide could be produced, which is an antimicrobial compound (especially against strict anaerobes), and that ammonia could also be produced (which would buffer acidic pH). Depending on the pH level at which the isolate reduced nitrite to other compounds, a preferred application was selected, namely general oral health (all pH levels: D1P15A, D4P7, D4T9), caries (acidic pH: D1P10, D4T6) or periodontal diseases and halitosis (neutral to slightly alkaline pH: D1P7, D3T4). Isolates that produced most nitrite (D1P17, D3T4, D4T4, D5T11A) were considered to be suitable to increase systemic nitric oxide levels by nitrite ingestion. It has been shown that this has a broad range of benefits, including the lowering of blood pressure, improved endothelial function, increase in sport performance and reversal of metabolic syndrome, as well as antidiabetic effects (Lundberg et al., 2018). Importantly, the isolates at different pH levels were tested alone too. When adding isolates to an oral biofilm, other bacteria could further reduce the nitrite, which can lead to local benefits and the prevention of oral diseases. Apart from nitric oxide, ammonium can be produced that can prevent the development of caries as described in EXAMPLE 1.

33. All these results together show that different types of nitrate-reducing bacteria can be found in the oral cavity of different individuals. These nitrate-reducing isolates could be used as probiotics and symbiotics (i.e. the probiotics and a source of nitrate as a prebiotic) aimed at increasing the nitrate and nitrite reduction capacities of individuals with an impaired nitrate-reducing capability, as shown for individual D25 above. An increase in nitrate and nitrite reduction capacities could improve oral and cardiovascular health, but for some individuals, nitrate alone could be sufficient to achieve a health-associated state, as shown in the examples above for individual D2. Thus, a personalized treatment with nitrate as a prebiotic, a nitrate-reducing probiotic or a symbiotic (pre- + probiotic) could be directed depending on the NRC of a given individual.

### EXAMPLE 4: Products and administration forms

### 4.1. Nitrate-rich vegetable extract supplement application (direct and indirect effect)

Participants brushed their teeth in the morning like usual. Then, from a jar with 92 g of beetroot extract/vitamin C/molybdenum supplement (Table 5), a dose was taken of 11.5 g by using a plastic spoon provided with the jar and filling it until the 25 ml line. The dose was dissolved in 200 ml water, mixed and ingested. A dose of supplement contains 250 mg (i.e., below the 259 mg ADI for an adult of 70 kg) nitrate naturally present in the beetroot extract and the current daily-recommended doses of vitamin C (80 mg) and molybdenum (50 µg) for adults. Vitamin C is an anti-oxidant that stimulates nitrite reduction to nitric oxide, preventing the formation of toxic N-nitroso compounds, and molybdenum is a cofactor for bacterial nitrate reduction enzymes. The combination of these molecules stimulates denitrification, especially in individuals lacking these nutrients. In two individuals, the nitrate was measured every hour over 5 hours and the salivary nitrate concentration stayed elevated over the entire period. In EXAMPLE 2, twelve individuals took this supplement, which increased nitrate and nitrite levels after 1.5 h in all individuals, and prevented a pH drop by a sugar rinse compared to a placebo supplement. The placebo supplement had an identical composition except for the beetroot extract that was replaced with an identical weight of orange extract (i.e., a vegetable with an insignificant nitrate content, FIG. 8).

This product is supplied in a single dose per day, preferably in the morning, in the form of a food supplement as a vegetable extract powder, and provides an immediate (within an hour, due to the retention of nitrate in the oral cavity during swallowing) and an acute but indirect effect (between 1 and 6 hours) due to nitrate recycling, during which a drop pH after a meal is diminished and therefore protection against oral diseases (such as dental caries) is provided.

**Table 5: beetroot supplement composition**

| **Ingredient** | **mg/jar of 92 g** | **mg/dosis (11.5 g)** |
|---|---|---|
| Beetroot extract 3% nitrate (Beta vulgaris) | 66664.0 | 8333.00 |
| Apple Pectin E-440 | 23064.0 | 2883.00 |
| Natural Red Fruits Flavor (Doler) | 1600.0 | 200.00 |
| Vitamin C, L-Ascorbic Acid | 640.0 | 80.00 |
| Sucralose E-955 (richness 98-102%) | 32.0 | 4.00 |
| Ammonium molybdate (heptamolybdate: 54.32% Mo) | 0.73638 | 0.09205 (= 50 µg Mo) |
| **Total** | **92000.7** | **11500.1** |

### 4.2. Daily dose chewing tablet application (direct and indirect)

Chewing tablets (1 g) containing nitrate (222 mg if 1 dose, 111 mg if 2 doses, 74 mg if 3 doses) were consumed daily by chewing and swallowing after breakfast and oral hygiene in the morning and, if divided in two doses, also after lunch and, if divided in three doses, also after dinner and oral hygiene at the end of the day. Some variants of the tablets of 1, 2 or 3 doses also contained 80 mg, 40 mg or 26.67 mg vitamin C, respectively, and/or 50 µg, 25 µg or 16.67 µg molybdenum. Finally, some variants of the tablets contained daily acceptable amounts of commercially available anti-oxidants, which were divided over 1, 2 or 3 doses. To choose the optimal combinations and amounts of molecules, the different tablets were tested. This is ideal for an acute direct and indirect effect on oral diseases, during a period of 0-6 h after ingestion.

### 4.3. Anti-caries chewing tablet application (direct and indirect)

Tablets (1 g) containing 74 mg nitrate were consumed by swallowing before a meal. A maximum of three tablets could be consumed per day and it was recommended to consume them 1h before the three meals or snacks with most sugar, preferably each in a different part of the day (morning, afternoon and evening). Other molecules were added based on EXAMPLE 4.2. This is ideal for an acute indirect effect on oral diseases, during a period of 1-6 h after ingestion, as well as to improve all health conditions that are influenced by a deficit of nitric oxide.

### 4.4. Chewing gum application (direct and indirect)

Chewing gums (1 g) containing 37 mg nitrate were consumed by swallowing before a meal. A maximum of six chewing gums could be consumed per day and it was recommended to consume them right after meals or snacks, preferably at least one in a different part of the day (morning, afternoon and evening). Other molecules were added based on EXAMPLE 4.2. This is ideal for an acute indirect effect on oral diseases, during a period of 1-6 h after ingestion, as well as to improve all health conditions that are influenced by a deficit of nitric oxide.

### 4.5. Toothpaste application (direct)

A toothpaste dose of 0.3 g containing 74 mg of nitrate, 26.67 mg vitamin C and 16.67 µg molybdenum and other molecules based on EXAMPLE 4.2 was used by individuals like normally without exceeding three times of toothbrushing per day. This is administered when brushing, as with a standard toothpaste, by contact with the teeth and gum, which provides a topic application of nitrate directly to oral biofilms, being part of the nitrate also retained in the oral cavity until saliva clearance eliminates it. It is recommended that the mouth is not washed after application.

### 4.6. Mouthwash application (direct)

A oral rinse of 15 ml containing (111 mg nitrate in the total volume) for 10 s is made by which a topic application of the nitrate product is given to tongue, teeth, oral mucosa and gums, and nitrate is therefore directly provided to oral biofilms, being part of the nitrate also retained in the oral cavity until saliva clearance eliminates it. It is recommended that the mouth is not washed after application and the ADI of 222 mg for an adult of 60 kg (or 3.7 mg nitrate per kg body weight) is not exceeded by using the mouthwash more than twice per day.

### 4.7. Oral gel for periodontal pockets application (direct)

A buccoadhesive gel is applied with a syringe by a professional inside the periodontal pockets of patients with a periodontal disease, containing a concentration of 222 mg nitrate to divide over all periodontal pockets, with or without a nitrate-reducing probiotic. In a preferred preparation, the gel also contains molybdenum + vitamin C. It is applied inside the pockets at the basal, treatment and follow-up visits of the patient as an initial treatment. It is recommended not to eat or drink for an hour after application. A maintenance treatment can be combined, in which a daily nitrate supplement or tablet is provided for 1 to 4 weeks in the morning. When the composition comprises probiotic bacteria, the product is not recommended for immunosuppressed patients.

### 4.8. Daily dose capsule or pill application (indirect)

Capsules (1 g) containing nitrate (222 mg if 1 dose, 111 mg if 2 doses, 74 mg if 3 doses) were consumed daily by ingestion before breakfast and oral hygiene in the morning and, if divided in two doses, also before lunch and, if divided in three doses, also before dinner and oral hygiene at the end of the day. Some variants of the capsules of 1, 2 or 3 doses also contained 80 mg, 40 mg or 26.67 mg vitamin C, respectively, and/or 50 µg, 25 µg or 16.67 µg molybdenum. Finally, some variants of the capsules contained daily acceptable amounts of commercially available anti-oxidants, which were divided over 1, 2 or 3 doses. To choose the optimal combinations and amounts of molecules, the different capsules were tested. This is ideal for an acute direct and indirect effect on oral diseases, during a period of 0-6 h after ingestion.

### 4.9. Probiotics application

A nitrate-reducing probiotic is provided in a lyophilized form with vitamin C and molybdenum, as well as a thickening agent. This is mixed with water and applied in the teeth with a ferule for 5-30 minutes, to allow bacterial colonization of the dental biofilm. This is applied at night at least 30 minutes after standard oral hygiene, avoiding eating or drinking for at least an hour after application. This is especially suited to treat and prevent dental diseases (caries or gum diseases). In another preferred mode of application, the probiotic preparation is applied to the tongue for 1-5 minutes, which is especially suited to treat halitosis. This product is not recommended for immunosuppressed patients.

### 4.10. Parenteral nutrition for intravenous application at Intensive Care Units (indirect)

Parenteral nutrition (35 ml/kg body weight/day) for intravenous application was given to patients at the Intensive Care Units (ICU), containing the patient-dependent daily nutrients, 222 mg nitrate and 80 mg vitamin C. Patients at ICU suffer from inflammation, caries and halitosis and these conditions rapidly worsen when they arrive at the ICU. The addition of nitrate to parenteral nutrition limited the development of one or more of these conditions.

### 4.11. Sugar-containing candy for children and adults

Different types of candies, including gummi bears or other gummi animals (2-5 g per gummi animal) and other gelatin- or pectin-based candies (2-25 g), small lollipops (4-25 g per lollipop), large lollipops (25-150 g per lollipop), chocolate and candy bars (20-70 g), chewing gum or bubble gum (1-5 g), chocolate coins and other chocolates (2-50 g), licorice candies (2-25 g), peanut butter candies (2-25 g), caramel candies (2-25 g), fruit flavored hard and chewy candies (2-25 g), nougat bars (20-70 g), taffies (4-25 g), toffees (4-25 g), candy sticks (4-25 g), marshmallows (2-20 g), heart-shaped candies (2-20 g), and other types of candies were used. Different amounts of nitrate in the form of nitrate salts or vegetable extracts were added to the abovementioned candies to obtain final nitrate amounts of 100 micrograms to 222 milligrams per candy. Nitrate salts and vegetable extracts were sometimes combined with vitamin C and/or commercially available anti-oxidants (as described in EXAMPLES 4.1-4.10). To find the optimal balance between sugar and nitrate for a pH buffering effect that prevents acidification of saliva and oral biofilms when sugar is consumed (as observed in EXAMPLES 1 and 2), each candy was administered with different amounts of nitrate. In some cases, fractions of the recommended daily doses of molybdenum and copper were added as co-factors to each candy to improve the pH buffering effect and antimicrobial effects derived from nitrate reduction.

### 4.12. Starch- and sugar-containing products

Nitrate (100 microgram to 222 milligrams per serving) and, in some cases, nitrate and co-factors (as described in EXAMPLE 4.11) were added to other starch- and sugar-containing products to limit their cariogenic potential. These products included bread, cakes, crackers, dried fruits, fruit drinks, fruit juice, ice creams, noodles, rice, sweetened cereals, sweetened sport drinks, protein bars, premade soups, cereal bars, canned fruit, (low fat) yogurt, barbecue sauce, ketchup, pasta sauce and other sauces, sweetened soda and other sweetened beverages, as well as to sugar itself added to tea, coffee and other products.

### 4.13. Pet and livestock food and snacks

Other embodiments containing nitrate supplementation were used to improve oral health (e.g. to treat halitosis, gum diseases, tartar or dental caries), or as a prevention strategy for oral diseases and nitric oxide-related systemic diseases in mammals other than humans, including cats, dogs, horses, cows, pigs, goats, sheep, donkeys, buffalo, oxen, llamas and camels. Nitrate (with a recommended dose of 1,43 micrograms to 3.3 milligrams per kg of animal's weight) and, in some cases, nitrate and co-factors were added to different products suitable for animal consumption. For dogs and cats, these were dry and wet food, chews, biscuits, dental sticks, wet treats and other treats. For horses, cows, pigs, goats, sheep, donkeys, buffalo, oxen, llamas and camels, these were dry food, salt blocks, fruit nuggets, cookies and other treats. The nitrate was added in the form of nitrate salts or vegetable extracts to the abovementioned products to obtain final nitrate amounts of 100 micrograms to 222 milligrams per serving of food or treats.

### 4.14. Low daily doses of nitrate in products for oral health

All products with topical applications described of EXAMPLES 4.1-4.12 were produced with low doses of nitrate, so that the total amount of nitrate applied or ingested after using a product one or several times per day remained far below the ADI (e.g., 100 micrograms to 74 milligrams of nitrate per day), but were high enough to provide beneficial effects on oral health. The beneficial oral effects started from the first dose. However, a treatment plan consisting of a daily-dose over a week or several weeks or months was applied for an accumulative effect on nitrate reduction capacity improvement, an increase in health-associated nitrate-reducing bacteria (e.g., *Rothia* spp. and *Neisseria* spp.) and a decrease of disease-associated bacteria (E.g., *Streptococcus mutans* and *Porphyromonas gingivalis*) in oral biofilms.

### 4.15. Tongue paste application (direct)

Tongue paste doses of 0.3, 0.5 or 1 g, containing 111 mg of nitrate and, in some cases, 40 mg vitamin C, 25 µg molybdenum, 0.5 mg copper and/or other molecules based on EXAMPLE 4.2 were used by individuals that were instructed to brush their tongue twice per day with a tooth brush or tongue brush for 3 minutes. To increase the beneficial oral effects and systemic levels of nitric oxide, it is recommended that the mouth is not washed after application.

### 4.16. Dental floss with nitrate (direct)

Dental floss coated with 111 mg nitrate per 50 cm, and, in some cases, 40 mg vitamin C per 50 cm, 25 µg molybdenum per 50 cm, 0.5 mg copper per 50 cm and/or other molecules based on EXAMPLE 4.2 was used by individuals that were instructed to floss their teeth like normally without exceeding the recom-mended two times of flossing per day.

### EXAMPLE 5: Nitrate reduction capacity (NRC) determination

An experienced dentist assessed the oral health of 20 participants. Neither active caries nor any history of periodontitis was detected and all participants were considered orally healthy. Additionally, no systemic diseases were reported and no hypertension was detected among the participants. The donors were asked to restrain from oral hygiene and breakfast (only water consumption was allowed) in the morning and donated ~4 ml saliva around 9 am. Two Eppendorf tubes of saliva with 8 mM nitrate were prepared (450 µl saliva with 50 µl sterile 80 mM nitrate in water solution). One was directly frozen at -20°C and the other one incubated for 2 hours at 37°C. The nitrate was measured as described in EXAMPLE 1 and the two time-points were compared to determine how many mg/l had been reduced.

On average 112.20 mg/l (SD 87.43 mg/l) of nitrate were reduced (median: 91 mg/l). The nitrate reduced after 2h (NO3R2h) in different participants was split in percentiles of one third (33.33%) and two thirds (66.67%), which were 56 mg/l and 176 mg/l, respectively (Table 6). When represented as a percentage of the initial nitrate detected this was 15% and 35%, respectively (table 6). Then, subjects were divided into bad nitrate reducers (below 57 mg/l), intermediate nitrate reducers (between and including 57-175 mg/l) and good nitrate reducers (above 175 mg/l) with 8, 6 and 6 participants in each group, respectively.

**Table 6. NRC1: division based on thirds**

| **BASED ON THIRDS** | **NO3R2h (mg)** | **NO3R2h (%)** | **Classification** | **N (20)** |
|---|---|---|---|---|
| Below 1/3 | <57 | <15% | Bad | 8 |
| Between and including 1/3 and 2/3 | 57-175 | 15-35% | Intermediate | 6 |
| Above 1/3 | >175 | >35% | Good | 6 |

### EXAMPLE 6: Ex vivo study - effects of nitrate on oral biofilm growth of human patients with periodontitis

In addition to the oral biofilms from healthy individuals provided in EXAMPLE 1, *ex vivo* experiments with a dysbiotic community (subgingival plaque samples from patients with periodontitis) were performed. Eleven patients diagnosed with periodontitis were enrolled into the study. Subgingival plaque was collected by inserting eight to ten sterile paper points inside the deepest periodontal pockets. The paper points were then transferred to a 2 mL tube containing reduced transport media (RTF) and stored in a cooling box at around 4 °C for max. 18 hours. For the biofilm growth, BHI containing vitamin K and hemin (as described in EXAMPLE 1) was used. Transport medium was removed by centrifugation (1 min, 12000 rpm), followed by resuspension into 1 mL BHI. The xCELLigence system described in EXAMPLE 1 was used. Patient samples were incubated under four different conditions; control, nitrate, control+probiotic and nitrate+probiotic. First, 100 µl of periodontal plaque in BHI was added to the wells. Then, for the nitrate condition, 5 µl of 250 mM nitrate in BHI was added to the wells to obtain a 5 mM nitrate concentration. For the control+probiotic and nitrate+probiotic conditions, 45 µl of R. aeria D1P7 culture of OD (600 nm) = 0.417 was added to the wells, resulting in a final OD (600 nm) of 0.075 per well. BHI was added to reach a final volume of 250 µl. A medium control (without patient sample or probiotic) with nitrate (Cntr) was used to determine the initial amounts of nitrate and nitrite in the medium. After 7 hours of incubation, supernatant and biofilm was harvested for nitrate/nitrite measurements and 16S rRNA gene sequencing, respectively (both described in EXAMPLE 1).

In this experiment, adding 6.5 mM nitrate to the sample improved biofilm composition, reducing the levels of periodontal pathogens such as *Porphyromonas, Eikenella* and *Tannerella,* among others (FIG. 13A). However, when a symbiotic treatment was applied (i.e. nitrate as a prebiotic together with *Rothia aeria* D1P7 as a probiotic), a highly efficient improvement in bacterial composition was achieved, with a significant increase of *Rothia* (beneficial species) and a significant decrease in periodontal pathogens including *Fusobacterium, Fretibacterium* and *Treponema* (FIG. 13B), showing that dysbiosis was reversed. Thus, more periodontal pathogens were reduced with the symbiotic treatment, and that reduction was larger, compared to the outcome when only nitrate was added. This further improvement is due to the low levels of nitrate-reducing bacteria in highly dysbiotic bacterial communities like those in periodontal pockets, making the addition of a probiotic highly effective. The same results were obtained when analysing samples at the species taxonomic level, with larger reductions in periodontal pathogens in the symbiotic treatment compared to the nitrate-only treatment (FIG. 13C and 13D). From this experiment it is concluded that results from pure cultures cannot foresee the effect of nitrate or nitrate-reducing bacteria on complex oral biofilms. Instead, the effects need to be tested in real biological samples i.e. dysbiotic bacterial oral biofilms. The same superior benefit of nitrate+probiotic treatment compared to nitrate-alone was also seen when analysing Nitrate-Reducing Capacity. In the nitrate treatment, around 20% of the nitrate was reduced after the 7 hours of growth in these periodontal samples. When nitrate was applied together with the probiotic *Rothia aeria* D1P7, 90% of the nitrate was reduced, indicating that although both treatments allowed nitrate reduction, the symbiotic treatment was superior (FIG. 14A). When measuring the nitrite produced, the nitrate+probiotic treatment triplicated the values obtained when adding nitrate alone (FIG. 14B). Thus, adding nitrate improved dysbiosis and the ability to reduce nitrate, whereas adding nitrate plus one of the probiotics described in the present application further reversed dysbiosis and restored nitrate reduction capacity in severe patients.

In addition, the mean growth curve of the periodontitis biofilms in the nitrate conditions (N) was lower than in the control condition (C). This is shown in FIG. 14C-F, showing the average growth curves of all 11 patients (FIG. 14C) and different types of curves of three individual patients (FIG. 14D-F). This shows that nitrate has an anti-biofilm accumulation effect (or an or anti-plaque effect) on dysbiotic biofilms. In conclusion, in this Example, we show that nitrate reduces dysbiosis but also reduces the biofilm quantity (i.e. dental plaque).

### EXAMPLE 7: Identification of the bacteria from human samples collected in EXAMPLE 2 (In vivo study - oral administration of nitrate to healthy human individuals)

Oral samples collected in EXAMPLE 2 were used to determine bacterial composition by 16S rRNA gene Illumina sequencing (methods as described in EXAMPLE 1, points 6.1-6.3). Data show that four hours after ingesting a 220 mg nitrate supplement, human subjects show an increase in the nitrate reducing bacteria *Rothia* and *Neisseria* and a decrease in several caries pathogens including *Veillonella, Atopobium* or *Oribacterium,* and a decrease in periodontal and halitosis pathogens (FIG. 15A), including *Dialister, Fretibacterium, Saccharimonas, Treponema, Peptostreptococcus, Alloprevotella, Selenomonas, Prevotella, Porphyromonas* and *Fusobacterium.* Results at the species level confirm the results, with many caries-associated, periodontitis-associated and halitosis-associated species decreasing (FIG. 15B). This confirms that a single dose of ingested nitrate reduces oral periodontal and halitosis pathogens *in vivo.* In addition, it shows that results in the complex multi-species biofilm model used in EXAMPLE 1 and 6 are confirmed by *in vivo* data in EXAMPLE 2 and 7. This highlights the importance of using a robust and representative biofilm model to replicate real conditions in the oral cavity, as opposed to single-species pure cultures, whose results cannot be extrapolated to complex oral microbial communities.

### EXAMPLE 8: Characterization of nitrate-reducing species identified in EXAMPLE 3

Another simpler calculation of nitrite and nitric oxide produced in EXAMPLE 3 was performed, considering 100% of nitrite as the maximum nitrite level present in all samples. Results show estimated levels of nitrite and nitric oxide for different isolates, and their potential applications (Table 7). Importantly, all probiotics reduce nitrate and produce nitrite and reduction products of nitrite (e.g., nitric oxide and/or ammonia). Therefore, all probiotics are suitable to treat all different conditions (i.e., caries, periodontal diseases, halitosis, cardiovascular and other systemic conditions), but some strains may be more efficient to treat one condition than others.

**Table 7. Nitrite and nitric oxide produced by isolates grown at different pH for 5h with 6.5 mM nitrate. Calculations are based on the maximum nitrite concentration detected being 100%.**

| **Isolate** | **Species (16S)** | **Nitrate reduced (NO3R) into:** | **pH 6.5** | **pH 7** | **pH 7.5** | **Application examples*³** |
|---|---|---|---|---|---|---|
| D1P7 (CECT9999*¹) | *Rothia aeria* | **Total NO3R (%)** | **52*** | **100** | **100*** | Periodontal diseases (perio.)*⁴ / general oral health |
| | | % nitrite | 20 | 77* | 85* | |
| | | % nitric oxide*² | 32 | 23* | 15* | |
| D1P10 | *Rothia dentocariosa* | **Total NO3R (%)** | **48** | **47** | **23** | Caries |
| | | % nitrite | 15 | 27 | 23 | |
| | | % nitric oxide*² | 33* | 20* | 0.3 | |
| D1P15A | *Rothia dentocariosa* | **Total NO3R (%)** | **50** | **53** | **43** | General oral health |
| | | % nitrite | 17 | 36 | 27 | |
| | | % nitric oxide*² | 33* | 16 | 17* | |
| D1P17 (CECT30000) | *Rothia dentocariosa* | **Total NO3R (%)** | **48** | **49** | **49** | Systemic applications |
| | | % nitrite | 21* | 37 | 37* | |
| | | % nitric oxide*² | 27 | 13 | 12 | |
| D3T4 (CECT30001) | *Rothia mucilaginosa* | **Total NO3R (%)** | **34** | **61*** | **69*** | Perio. / systemic applications |
| | | % nitrite | 14 | 43* | 55* | |
| | | % nitric oxide*² | 20 | 18* | 14* | |
| D4P7 | *Rothia dentocariosa* | **Total NO3R (%)** | **53*** | **46** | **48** | General oral health |
| | | % nitrite | 16 | 34 | 28 | |
| | | % nitric oxide*² | 38* | 12 | 20* | |
| D4T4 (CECT30002) | *Rothia mucilaginosa* | **Total NO3R (%)** | **52*** | **55** | **52*** | Systemic applications |
| | | % nitrite | 21* | 48* | 40* | |
| | | % nitric oxide*² | 31 | 7 | 12 | |
| D4T6 (CECT30003) | *Rothia mucilaginosa* | **Total NO3R (%)** | **77*** | **66*** | **35** | Caries |
| | | % nitrite | 41* | 40* | 35 | |
| | | % nitric oxide*² | 35* | 26* | 0 | |
| D4T9 (CECT30004) | *Rothia mucilaginosa* | **Total NO3R (%)** | **59*** | **67*** | **57*** | General oral health |
| | | % nitrite | 24* | 36 | 32 | |
| | | % nitric oxide*² | 35* | 30* | 25* | |
| D5T11A (CECT30005) | *Rothia mucilaginosa* | **Total NO3R (%)** | **48** | **55** | **78*** | Systemic applications |
| | | % nitrite | 20* | 42* | 65* | |
| | | % nitric oxide*² | 28 | 13 | 13 | |
| Medians | - | **Total NO3R (%)** | **51** | **55** | **51** | - |
| | - | % nitrite | 20 | 38 | 36 | - |
| | - | % nitric oxide*² | 33 | 17 | 13 | - |

| | | | | | | |
|---|---|---|---|---|---|---|
| * number above median *¹ registered in culture collection and included in this patent *² nitric oxide and other compounds that may result from nitrite reduction (e.g., ammonium) ⁻³ note that all probiotics could be used to improve all mentioned conditions as nitrate reduction stimulates general oral and systematic health. *⁴ due to protein degradation, halitosis also happens at a neutral to alkaline pH and the same probiotics proposed for periodontal diseases can be applied. | | | | | | |

### REFERENCES LIST

Pérez-Chapparo et al., 2014 ("Newly Identified Pathogens Associated with Periodontitis"; J Dental Res 2014; 93(9): 846-858)
Koopman et al., 2016 ("Nitrate and the Origin of Saliva Influence Composition and Short Chain Fatty Acid Production of Oral Microcosms"; Microb Ecol 2016; 72:479-492)
Velmurugan et al., 2016 ("Dietary nitrate improves vascular function in patients with hypercholes-terolemia: a randomized, double-blind, placebo-controlled study"; Am J Clin Nutr 2016; 103:25-38)
Vanhatalo et al., 2018 ("Nitrate-responsive oral microbiome modulates nitric oxide homeostasis and blood pressure in humans"; Free Radic Biol Med. 2018 Aug 20; 124: 21-30)
Kuang et al., 2018 ("Novel Approaches to the Control of Oral Microbial Biofilms"; Biomed Res 2018; 2018:6498932)
Mira 2018 ("Oral Microbiome studies: Potential diagnostic and therapeutic implications"; Advances Dent Res 2018; 29(1):71-77)
Simón-Soro et al., 2013 ("A tissue-dependent hypothesis of dental caries"; Caries Research 2013; 47(6):591- 600)
Jockel-Schneider et al., 2016 ("Stimulation of the nitrate-nitrite-NO-metabolism by repeated lettuce juice consumption decreases gingival inflammation in periodontal recall patients: a randomized, double-blinded, placebo-controlled clinical trial"; J Clin Periodontol 2016; 43: 603-608)
Li et al., 2007 ("Salivary nitrate--an ecological factor in reducing oral acidity"; Oral Microbiol Immunol 2007; 22(1):67-71)
Rosier et al., 2018 ("Resilience of the Oral Microbiota in Health: Mechanisms That Prevent Dysbiosis"; Journal of Dental Research 2018; Apr;97(4):371-380)
Burgleigh et al., 2019 ("Dietary nitrate supplementation alters the oral microbiome but does not improve the vascular responses to an acute nitrate dose"; Nitric Oxide 89 2019; 54-63)
Takahashi et al., 2005 ("Microbial ecosystem in the oral cavity: Metabolic diversity in an ecological niche and its relationship with oral diseases"; International Congress Series 2005; 1284; 103-112)
DeGruttola et al., 2016 ("Current Understanding of Dysbiosis in Disease in Human and Animal Models"; Inflamm Bowel Dis 2016; 22(5); 1137-1150)
lebba et al., 2016 ("Eubiosis and dysbiosis: the two sides of the microbiota"; New Microbiol 2016; Jan; 39(1): 1-12)
Navazesh & Christensen, 1982 ("A comparison of whole mouth resting and stimulated salivary measurement procedures"; J Dent Res 1982; 61; 1158-1162)
Mira et al., 2019 ("Development of an in vitro system to study oral biofilms in real time through impedance technology: validation and potential applications"; J Oral Microbiol 2019; 11(1): 1609838)
Ferrer et al., 2017 ("Effect of antibiotics on biofilm inhibition and induction measured by real-time cell analysis"; J Appl Microbiol 2017 Mar; 122(3):640-650)
Dzidic et al., 2019 ("Oral microbiota maturation during the first 7 years of life in relation to allergy development"; Allergy 2018 Oct; 73(10); 2000-2011.
Boix-Amorós et al., 2016 ("Relationship between Milk Microbiota, Bacterial Load, Mac-ronutrients, and Human Cells during Lactation"; Front Microbiol 2016 Apr 20; 7(492); eCollection 2016)
Edgar et al., 2011 ("UCHIME improves sensitivity and speed of chimera detection"; Bioinformatics 2011; 27(16); 2194-200)
Wang et al., 2007 ("Naive Bayesian classifier for rapid assignment of rRNA sequences into the new bacterial taxonomy"; Appl Environ Microbiol 2007; 73(16); 5261-7)
Rognes et al., 2016 ("VSEARCH: a versatile open source tool for metagenomics"; PeerJ 2016; 4; e2584)
Oksanen et al., 2007 ("H. vegan: Community Ecology Package, R package version 2.4-2"; http://CRAN.R-project.org/package=vegan 2017)
Koopman et al., 2016 ("Changes in the oral ecosystem induced by the use of 8% arginine toothpaste"; Arch Oral Biol 2016; 73; 79-87)
Doel et al., 2005 ("Evaluation of bacterial nitrate reduction in the human oral cavity"; European journal of oral sciences 2005; 113(1); 14-19)
Mashimo et al., 2015 ("Variations of Nitrate-reducing Activity in Oral Rothia mucilaginosa"; Journal of Oral Tissue Engineering 2015; 13(1); 18-26)
Lundberg et al., 2018 ("Metabolic Effects of Dietary Nitrate in Health and Disease"; Cell Metab 2018 Jul 3; 28(1); 9-22)

## Claims

1. An oral composition comprising nitrate for use in an acute treatment or prevention of oral dysbiosis in a mammal,
wherein the acute treatment or prevention of oral dysbiosis comprises changing the bacterial composition and functions of oral biofilms in a mammal, and decreasing the amount of disease-associated bacteria and increasing the amount of health-associated bacteria;
wherein the composition is orally administered to the mammal and thereby increases the concentration of nitrate in the saliva of the mouth;
wherein the amount of nitrate per dose of composition is at least 3 micrograms when the oral composition is a topically applied composition, and at least 12 micrograms when the oral composition is an ingested composition; and
wherein the acute treatment or prevention has effects before 24 hours of a biofilm-mediated oral disease.

2. The composition for use according to claim 1, wherein the acute treatment or prevention of oral dysbiosis comprises:
increasing the amount of at least one health-associated bacteria of oral biofilms selected from the group consisting of *Neisseria, Rothia* and *Kingella* in the oral biofilms, and/or
decreasing the amount of at least one caries-associated bacteria of oral biofilms selected from the group consisting of *Streptococcus, Veillonella, Oribacterium* and *Atopobium* in the oral biofilms, and/or
decreasing the amount of at least one periodontal diseases/halitosis-associated bacteria of oral biofilms selected from the group consisting of *Porphyromonas, Fusobacterium, Leptotrichia, Prevotella, Treponema, Tannerella, Alloprevotella, Peptostreptococcus, Dialister, Eubacterium, Parvimonas, Selenomonas,* and *Solobacterium* in the oral biofilms.

3. The composition for use according to any of claims 1-2, wherein the biofilm-mediated oral disease is selected from the group consisting of a periodontal disease, halitosis and caries.

4. The composition for use according to any of the claims 1-3,
wherein the composition is a topically applied composition selected from the group consisting of:
- a toothpaste;
- a mouthwash;
- an oral gel;
- a dental floss;
- a tongue paste;
- a food extract;
- a chewing gum;
- a chewing tablet; and
- a supplement powder; or
wherein the composition is an ingested composition selected from the group consisting of:
- tablets, pills or capsules;
- a food extract;
- a chewing gum;
- a chewing tablet;
- a pet and livestock food or snack;
- starch- and sugar-containing products;
- a supplement powder; and
- parenteral nutrition for intravenous application.

5. The composition for use according to claim 4, wherein the composition is a food supplement comprising a nitrate-rich vegetable extract or nitrate in form of a salt, an antioxidant and/or a nitrate-reductase enzyme cofactor.

6. The composition for use according to claim 5, wherein the nitrate-reductase enzyme cofactor is molybdenum, or copper.

7. The composition for use according to any of claims 5-6, wherein the nitrate-rich vegetable extract is a beetroot extract.

8. The composition for use according to claim 4, wherein the composition is a food supplement comprising a nitrate-rich vegetable extract which is a beetroot extract, an antioxidant and/or a nitrate-reductase enzyme cofactor which is molybdenum, a salt thereof or a molybdenum-rich vegetable extract.

9. The composition for use according to any of claims 1-8, wherein the salivary concentration of nitrate in the mouth after administration of the composition on top of fasting salivary levels is:
at least 0.1 mM 30 s after administration when the composition is a topically applied composition and,
at least 0.1 mM at least 1.5 h after administration when the composition is an ingested composition.

10. The composition for use according to any of claims 1-9, administered in combination with at least a bacterial strain belonging to *Rothia* genus, wherein the *Rothia* bacterial strain:
a) reduces 100% of nitrate after 7 h of incubation at 37 °C starting with an optical density (OD) of 0.01 in BHI medium with 6.5 mM nitrate;
b) reduces more than 15% of nitrate after 4 h of incubation at 37 °C starting with an OD of 0.01 in BHI medium with 6.5 mM nitrate;
c) does not decrease the pH of BHI medium with 6.5 mM nitrate after 7 h of incubation at 37 °C starting with an OD of 0.01 below pH 6.8;
d) grows to an optical density over 0.7 after 7 h of growth in BHI medium with 6.5 mM nitrate at 37 °C starting with an OD of 0.01; and
e) is able to colonize an *in vitro* oral biofilm grown from human saliva during 5 h at 37 °C when adding 1:1 *Rothia* bacterial strain in BHI (OD 0.40):saliva inoculum, reaching a proportion of more than 10% of total bacteria in the formed biofilm.

11. The composition for use according to claim 10, wherein the bacterial strain is selected from the group consisting of the strains deposited in the Spanish Type Culture Collection (CECT) under the accession numbers CECT 9999, CECT 30000, CECT 30001, CECT 30002, CECT 30003, CECT 30004, and CECT 30005.

## Patentansprüche

1. Eine orale Zusammensetzung umfassend Nitrat zur Verwendung bei einer akuten Behandlung oder Prävention von oraler Dysbiose bei einem Säugetier,
wobei die akute Behandlung oder Prävention von oraler Dysbiose die Veränderung der bakteriellen Zusammensetzung und der Funktionen von oralen Biofilmen bei einem Säugetier und die Verringerung der Menge an krankheitsassoziierten Bakterien und die Erhöhung der Menge an gesundheitsassoziierten Bakterien umfasst;
wobei die Zusammensetzung dem Säugetier oral verabreicht wird und dadurch die Konzentration von Nitrat im Speichel des Mundes erhöht;
wobei die Menge an Nitrat pro Dosis der Zusammensetzung mindestens 3 Mikrogramm beträgt, wenn die orale Zusammensetzung eine topisch angewandte Zusammensetzung ist, und mindestens 12 Mikrogramm beträgt, wenn die orale Zusammensetzung eine eingenommene Zusammensetzung ist; und
wobei die akute Behandlung oder Prävention Auswirkungen 24 Stunden vor einer biofilmvermittelten oralen Erkrankung hat.

2. Die Zusammensetzung zur Verwendung nach Anspruch 1, wobei die akute Behandlung oder Prävention von oraler Dysbiose Folgendes umfasst:
die Erhöhung der Menge von mindestens einem gesundheitsassoziierten Bakterium oraler Biofilme, das aus der Gruppe ausgewählt ist, die aus *Neisseria, Rothia* und *Kingella* besteht, in den oralen Biofilmen und/oder
die Verringerung der Menge von mindestens einem kariesassoziierten Bakterium oraler Biofilme, das aus der Gruppe ausgewählt ist, die aus *Streptococcus, Veillonella, Oribacterium* und *Atopobium* besteht, in den oralen Biofilmen und/oder
die Verringerung der Menge von mindestens einem Parodontalerkrankungen/Halitose-assoziierten Bakterium von oralen Biofilmen, das aus der Gruppe ausgewählt ist, die aus *Porphyromonas, Fusobacterium, Leptotrichia, Prevotella, Treponema, Tannerella, Alloprevotella, Peptostreptococcus, Dialister, Eubacterium, Parvimonas, Selenomonas* und *Solobacterium* besteht, in den oralen Biofilmen.

3. Die Zusammensetzung zur Verwendung nach einem der Ansprüche 1 bis 2, wobei die biofilmvermittelte orale Erkrankung ausgewählt ist aus der Gruppe bestehend aus einer Parodontalerkrankung, Halitose und Karies.

4. Die Zusammensetzung zur Verwendung nach einem der Ansprüche 1 bis 3,
wobei die Zusammensetzung eine topisch angewandte Zusammensetzung ist, die ausgewählt ist aus der Gruppe bestehend aus:
- einer Zahnpasta;
- einer Mundspülung;
- einem oralen Gel;
- einer Zahnseide;
- einer Zungenpaste;
- einem Nahrungsmittelextrakt;
- einem Kaugummi;
- einer Kautablette; und
- einem Ergänzungspulver; oder
wobei die Zusammensetzung eine aufgenommene Zusammensetzung ist, die ausgewählt ist aus der Gruppe bestehend aus:
- Tabletten, Pillen oder Kapseln;
- einem Nahrungsmittelextrakt;
- einem Kaugummi;
- einer Kautablette;
- einem Haustier- und Viehfutter oder Snack;
- Stärke- und Zuckerhaltigen Produkten;
- einem Ergänzungspulver; und
- parenteraler Ernährung zur intravenösen Anwendung.

5. Die Zusammensetzung zur Verwendung nach Anspruch 4, wobei die Zusammensetzung ein Nahrungsergänzungsmittel ist, das einen nitratreichen Pflanzenextrakt oder Nitrat in Form eines Salzes, ein Antioxidans und/oder einen Nitrat-Reduktase-Enzym-Cofaktor umfasst.

6. Die Zusammensetzung zur Verwendung nach Anspruch 5, wobei der Nitrat-Reduktase-Enzym-Cofaktor Molybdän oder Kupfer ist.

7. Die Zusammensetzung zur Verwendung nach einem der Ansprüche 5 bis 6, wobei der nitratreiche Pflanzenextrakt ein Rote-Bete-Extrakt ist.

8. Die Zusammensetzung zur Verwendung nach Anspruch 4, wobei die Zusammensetzung ein Nahrungsergänzungsmittel ist, das Folgendes umfasst: einen nitratreichen pflanzlichen Extrakt, der ein Rote-Bete-Extrakt ist, ein Antioxidans und/oder einen Nitrat-Reduktase-Enzym-Cofaktor, der Molybdän, ein Salz davon oder ein molybdänreicher pflanzlicher Extrakt ist.

9. Die Zusammensetzung zur Verwendung nach einem der Ansprüche 1 bis 8, wobei die Speichelkonzentration von Nitrat im Mund nach der Verabreichung der Zusammensetzung zusätzlich zu den nüchternen Speichelspiegeln beträgt:
mindestens 0,1 mM 30 s nach der Verabreichung, wenn die Zusammensetzung eine topisch angewandte Zusammensetzung ist und,
mindestens 0,1 mM mindestens 1,5 h nach der Verabreichung, wenn die Zusammensetzung eine eingenommene Zusammensetzung ist.

10. Die Zusammensetzung zur Verwendung nach einem der Ansprüche 1 bis 9, verabreicht in Kombination mit mindestens einem Bakterienstamm, der zur Gattung *Rothia* gehört, wobei der Rothia-Bakterienstamm:
a) 100% Nitrat nach 7 h Inkubation bei 37 °C beginnend mit einer optischen Dichte (OD) von 0,01 in BHI-Medium mit 6,5 mM Nitrat reduziert;
b) mehr als 15% Nitrat nach 4 h Inkubation bei 37 °C beginnend mit einer OD von 0,01 in BHI-Medium mit 6,5 mM Nitrat reduziert;
c) den pH-Wert von BHI-Medium mit 6,5 mM Nitrat nach 7 h Inkubation bei 37 °C beginnend mit einer OD von 0,01 unter pH 6,8 nicht verringert;
d) nach 7 h Wachstum in BHI-Medium mit 6,5 mM Nitrat bei 37 °C beginnend mit einer OD von 0,01 auf eine optische Dichte von über 0,7 wächst; und
e) in der Lage ist, einen *in vitro* oralen Biofilm, der aus menschlichem Speichel gewachsen ist, während 5 h bei 37 °C zu besiedeln, wenn 1:1 *Rothia*-Bakterienstamm in BHI (OD 0,40):Speichelinokulum hinzugefügt wird, wodurch ein Anteil von mehr als 10 % der gesamten Bakterien im gebildeten Biofilm erreicht wird.

11. Die Zusammensetzung zur Verwendung nach Anspruch 10, wobei der bakterielle Stamm ausgewählt ist aus der Gruppe bestehend aus den Stämmen, die bei der spanischen Artenkultursammlung (CECT) unter der Hinterlegungsnummer CECT 9999, CECT 30000, CECT 30001, CECT 30002, CECT 30003, CECT 30004 und CECT 30005 hinterlegt sind.

## Revendications

1. Une composition orale comprenant du nitrate pour une utilisation dans un traitement aigu ou la prévention de la dysbiose orale chez un mammifère,
dans lequel le traitement aigu ou la prévention de la dysbiose orale comprend la modification de la composition bactérienne et des fonctions des biofilms oraux chez un mammifère, et la diminution de la quantité de bactéries associées à la maladie et l'augmentation de la quantité de bactéries associées à la santé ;
dans lequel la composition est administrée par voie orale au mammifère et augmente ainsi la concentration de nitrate dans la salive de la bouche ;
dans lequel la quantité de nitrate par dose de composition est d'au moins 3 microgrammes lorsque la composition orale est une composition appliquée localement, et d'au moins 12 microgrammes lorsque la composition orale est une composition ingérée ; et
dans lequel le traitement aigu ou la prévention a des effets avant 24 heures d'une maladie buccale médiée par un biofilm.

2. La composition pour l'utilisation selon la revendication 1, dans laquelle le traitement aigu ou la prévention de la dysbiose orale comprend :
l'augmentation de la quantité d'au moins une bactérie associée à la santé de biofilms oraux choisie dans le groupe constitué par *Neisseria, Rothia* et *Kingella* dans les biofilms oraux, et/ou la diminution de la quantité d'au moins une bactérie associée à la carie des biofilms oraux choisie dans le groupe constitué par *Streptococcus, Veillonella, Oribacterium* et *Atopobium* dans les biofilms oraux, et/ou
la diminution de la quantité d'au moins une bactérie associée aux maladies parodontales/à l'halitose de biofilms oraux choisie dans le groupe constitué par *Porphyromonas, Fusobacterium, Leptotrichia, Prevotella, Treponema, Tannerella, Alloprevotella, Peptostreptococcus, Dialister, Eubacterium, Parvimonas, Selenomonas* et *Solobacterium* dans les biofilms oraux.

3. La composition pour l'utilisation selon l'une quelconque des revendications 1 à 2, dans laquelle la maladie buccale médiée par un biofilm est choisie dans le groupe constitué par une maladie parodontale, l'halitose et la carie.

4. La composition pour l'utilisation selon l'une quelconque des revendications 1 à 3,
dans lequel la composition est une composition appliquée localement choisie dans le groupe constitué par :
- un dentifrice ;
- un bain de bouche ;
- un gel oral ;
- un fil dentaire ;
- une pâte de langue ;
- un extrait alimentaire ;
- un chewing-gum ;
- un comprimé à mâcher ; et
- une poudre de supplément ; ou
dans laquelle la composition est une composition ingérée choisie dans le groupe constitué par :
- des comprimés, des pilules ou des capsules ;
- un extrait alimentaire ;
- un chewing-gum ;
- un comprimé à mâcher ;
- une nourriture ou une collation pour animaux de compagnie et d'élevage ;
- des produits contenant de l'amidon et du sucre ;
- une poudre de supplément ; et
- de la nutrition parentérale pour application intraveineuse.

5. La composition pour l'utilisation selon la revendication 4, dans laquelle la composition est un complément alimentaire comprenant un extrait végétal riche en nitrate ou du nitrate sous la forme d'un sel, un antioxydant et/ou un cofacteur d'enzyme nitrate-réductase.

6. La composition pour l'utilisation selon la revendication 5, dans laquelle le cofacteur de l'enzyme nitrate-réductase est le molybdène ou le cuivre.

7. La composition pour l'utilisation selon l'une quelconque des revendications 5 à 6, dans laquelle l'extrait végétal riche en nitrate est un extrait de betterave.

8. La composition pour l'utilisation selon la revendication 4, dans laquelle la composition est un complément alimentaire comprenant un extrait végétal riche en nitrate qui est un extrait de betterave, un antioxydant et/ou un cofacteur d'enzyme nitrate-réductase qui est le molybdène, un sel de celui-ci ou un extrait végétal riche en molybdène.

9. La composition pour l'utilisation selon l'une quelconque des revendications 1 à 8, dans laquelle la concentration salivaire de nitrate dans la bouche après l'administration de la composition sur les niveaux salivaires à jeun est :
au moins 0,1 mM 30 s après l'administration lorsque la composition est une composition appliquée localement et,
au moins 0,1 mM au moins 1,5 h après l'administration lorsque la composition est une composition ingérée.

10. La composition pour l'utilisation selon l'une quelconque des revendications 1 à 9, administrée en combinaison avec au moins une souche bactérienne appartenant au genre *Rothia,* dans laquelle la souche bactérienne *Rothia* :
a) réduit 100 % de nitrate après 7 h d'incubation à 37 °C à partir d'une densité optique (DO) de 0,01 dans un milieu BHI avec 6,5 mM de nitrate ;
b) réduit plus de 15 % de nitrate après 4 h d'incubation à 37 °C en commençant par une DO de 0,01 dans un milieu BHI avec 6,5 mM de nitrate ;
c) ne diminue pas le pH du milieu BHI avec 6,5 mM de nitrate après 7 h d'incubation à 37 °C à partir d'une DO de 0,01 inférieure à pH 6,8 ;
d) atteint une densité optique supérieure à 0,7 après 7 h de croissance en milieu BHI avec 6,5 mM de nitrate à 37 °C à partir d'une DO de 0,01 ; et
e) est capable de coloniser un biofilm oral *in vitro* cultivé à partir de salive humaine pendant 5 h à 37 °C en ajoutant une souche bactérienne *Rothia* 1:1 dans BHI (DO 0,40) : inoculum salivaire, atteignant une proportion de plus de 10 % des bactéries totales dans le biofilm formé.

11. La composition pour l'utilisation selon la revendication 10, dans laquelle la souche bactérienne est choisie dans le groupe constitué par les souches déposées dans la collection espagnole de cultures type (CECT) sous les numéros de dépôt CECT 9999, CECT 30000, CECT 30001, CECT 30002, CECT 30003, CECT 30004 et CECT 30005.
